# EUROPEAN PATENT APPLICATION

(11) **EP 4 644 991 A1**
(43) Date of publication of application: **05.11.2025**
(21) Application number: 23911911.8
(22) Date of filing: 20.12.2023
(51) Int. Cl.: G03F 7/004, C07C 25/18, C07C 309/05, C07C 309/17, C07C 381/12, C07D 307/00, C07D 309/12, C07D 327/06, C07D 333/46, C07D 333/48, C07D 333/76, C09K 3/00, G03F 7/20, G03F 7/038, G03F 7/039

(54) **ACTINIC RAY-SENSITIVE OR RADIATION-SENSITIVE RESIN COMPOSITION, RESIST FILM, PATTERN FORMATION METHOD, ELECTRONIC DEVICE MANUFACTURING METHOD, AND ONIUM SALT**

(30) Priority: 27.12.2022 JP 2022210176
(71) Applicant: FUJIFILM Corporation, Tokyo 106-8620 (JP)
(72) Inventor: KANEKO, Akihiro, Haibara-gun, Shizuoka 421-0396 (JP); ITO, Junichi, Haibara-gun, Shizuoka 421-0396 (JP); GOTO, Akiyoshi, Haibara-gun, Shizuoka 421-0396 (JP)
(74) Representative: HGF
(86) International application number: PCT/JP2023/045816
(87) International publication number: WO 2024/143131

(57) **Abstract**

To provide an actinic ray-sensitive or radiation-sensitive resin composition that has high LWR performance and can achieve reduction in the amount of post-development residue, a resist film using the actinic ray-sensitive or radiation-sensitive resin composition, a pattern forming method, and a method for producing an electronic device, and onium salts that can be used for the actinic ray-sensitive or radiation-sensitive resin composition.

An actinic ray-sensitive or radiation-sensitive resin composition containing a resin that is subjected to an action of an acid to undergo an increase in polarity and an onium salt having a specified structure, a resist film using the actinic ray-sensitive or radiation-sensitive resin composition, a pattern forming method, a method for producing an electronic device, and onium salts having specified structures.

## Description

### BACKGROUND OF THE INVENTION

### 1. Field of the Invention

The present invention relates to an actinic ray-sensitive or radiation-sensitive resin composition, a resist film, a pattern forming method, a method for producing an electronic device, and onium salts. More specifically, the present invention relates to an actinic ray-sensitive or radiation-sensitive resin composition that can be suitably used in ultramicrolithography processes applicable to, for example, processes for producing ultra-LSIs (Large Scale Integrations) and high-capacity microchips, processes for producing nanoimprint molds, and processes for producing high-density information recording media, and other photofabrication processes, an actinic ray-sensitive or radiation-sensitive film, a pattern forming method, a method for producing an electronic device, and onium salts.

### 2. Description of the Related Art

In fabrication processes for semiconductor devices such as ICs (Integrated Circuits) or LSIs (Large Scale Integrations), microprocessing by lithography using resist compositions has been performed. In recent years, with an increase in the degree of integration of integrated circuits, formation of ultrafine patterns in the submicron range or the quarter micron range has come to be in demand. With this, there is a trend for exposure wavelengths toward shorter wavelengths from the g-line to the i-line further to the KrF excimer laser beam; currently, exposure apparatuses using, as light sources, the ArF excimer laser having a wavelength of 193 nm have been developed. In addition, as a technique of further increasing the resolving power, a technique in which the space between a projection lens and a sample is filled with a liquid having a high refractive index (hereafter, also referred to as "immersion liquid"), what is called, the immersion method is being developed.

In addition, currently, lithography using, instead of excimer laser beams, an electron beam (EB), X-rays, extreme ultraviolet rays (EUV), or the like is also being developed. With this, resist compositions effectively sensitive to various actinic rays or radiations have been developed.

Various compositions are known as actinic ray-sensitive or radiation-sensitive resin compositions. WO2018/101339A describes a radiation-sensitive resin composition including a resin including a structural unit having an acid-dissociable group, an onium salt having a specified structure, and a solvent.

### SUMMARY OF THE INVENTION

There has recently been an increasing demand for higher performance in resist compositions. In particular, there has been a demand for further improvements in the line width roughness (Line Width Roughness: LWR) performance and reduction in the amount of post-development residue. The LWR performance refers to the performance of providing a pattern having a lower LWR.

An object of the present invention is to provide an actinic ray-sensitive or radiation-sensitive resin composition that has high LWR performance and can achieve reduction in the amount of post-development residue.

Another object of the present invention is to provide a resist film using the actinic ray-sensitive or radiation-sensitive resin composition, a pattern forming method, a method for producing an electronic device, and onium salts that can be used for the actinic ray-sensitive or radiation-sensitive resin composition.

The inventors of the present invention have found that the following features can address the above-described objects.

[1] An actinic ray-sensitive or radiation-sensitive resin composition containing a resin that is subjected to action of an acid to undergo an increase in polarity, and an onium salt represented by a formula (N1) below: 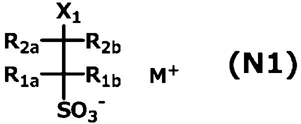
   wherein, in the formula (N1), R₁ₐ represents a cyano group, a nitro group, or a substituent represented by a formula (1A) below;
   R_{1b} and R_{2b} each independently represent a substituent having no fluorine atoms or a hydrogen atom;
   R₂ₐ represents a cyano group, a nitro group, a substituent represented by the formula (1A) below, or a substituent represented by a formula (1B) below;
   X₁ represents a hydrogen atom or an organic group;
   M⁺ represents an organic cation; and
   at least two of R₁ₐ, R₁₁, R₂ₐ, R_{2b}, and X₁ may be bonded together to form a ring, 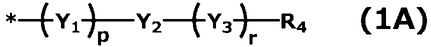
   in the formula (1A), Y₁ and Y₃ each independently represent -O- or -NR₃-;
   R₃ represents a hydrogen atom or an alkyl group;
   Y₂ represents -C(=O)- or -SO₂-;
   R₄ represents an alkyl group, a cycloalkyl group, or an aryl group;
   p and r each independently represent 0 or 1, and
   * represents a bonding site,

      ***-CH₂-R_{Y}** **(1B**)
   in the formula (1B), R_{Y} represents a cyano group, a nitro group, or a substituent represented by the formula (1A); and
   * represents a bonding site.
[2] The actinic ray-sensitive or radiation-sensitive resin composition according to [1], wherein, when R₁ₐ, R₁₁, R₂ₐ, and R_{2b} in the formula (N1) correspond to (i) below, R₂₆ represents a nitro group, a substituent represented by the formula (1A), or a substituent represented by the formula (1B),
   when R₁ₐ, R₁₁, R₂ₐ, and R_{2b} in the formula (N1) correspond to (ii) below, X₁ represents an organic group, or
   when R₁ₐ, R₁₁, and R₂ₐ in the formula (N1) correspond to (iii) below, X₁ represents an organic group,
      (i) a case where both of R₁ₐ and R_{1b} are not substituents represented by the formula (1A) and only one of R₁ₐ and R_{1b} is a cyano group, R₂ₐ is a cyano group, and R₂₆ is not a cyano group,
      (ii) a case where only one of R₁ₐ and R_{1b} is a cyano group, and both of R₂ₐ and R_{2b} are cyano groups, or
      (iii) a case where R₁ₐ is a substituent represented by the formula (1A), and Y₂ in the formula (1A) is -C(=O)-, R_{1b} is a hydrogen atom, and R₂ₐ is a substituent represented by the formula (1A).
[3] The actinic ray-sensitive or radiation-sensitive resin composition according to [1] or [2], wherein, in the formula (N1), R₁ₐ represents a cyano group, a nitro group, -COOR₄, -OCOOR₄, or -SO₂R₄, and R₄ has the same meaning as R₄ in the formula (1A).
[4] The actinic ray-sensitive or radiation-sensitive resin composition according to any one of [1] to [3], wherein, in the formula (N1), R₂ₐ represents a cyano group, a nitro group, -COOR₄, -OCOOR₄, -SO₂R₄, or a substituent represented by a formula (1C) below; and R₄ has the same meaning as R₄ in the formula (1A),

   ***-CH₂-R_{Y2}** **(1C)**

   in the formula (1C), R_{Y2} represents a cyano group, a nitro group, -COOR₄, -OCOOR₄, or -SO₂R₄;
   R₄ has the same meaning as R₄ in the formula (1A); and
   * represents a bonding site.
[5] The actinic ray-sensitive or radiation-sensitive resin composition according to any one of [1] to [4], wherein the onium salt represented by the formula (N1) is represented by a formula (N2) below: 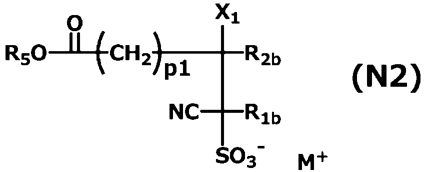
   in the formula (N2), R₅ represents an alkyl group, a cycloalkyl group, or an aryl group; p1 represents 0 or 1, and
   R_{1b}, R_{2b}, X₁, and M⁺ respectively have the same meanings as R_{1b}, R_{2b}, X₁, and M⁺ in the formula (N1).
[6] The actinic ray-sensitive or radiation-sensitive resin composition according to any one of [1] to [4], wherein the onium salt represented by the formula (N1) is represented by a formula (N3) below: 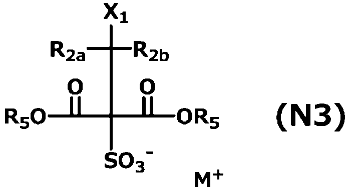
   in the formula (N3), R₅ represent an alkyl group, a cycloalkyl group, or an aryl group, and the plurality of R₅ may be the same or different; and
   R₂ₐ, R_{2b}, X₁, and M⁺ respectively have the same meanings as R₂ₐ, R_{2b}, X₁, and M⁺ in the formula (N1).
[7] The actinic ray-sensitive or radiation-sensitive resin composition according to any one of [1] to [4], wherein the onium salt represented by the formula (N1) is represented by a formula (N4) below: 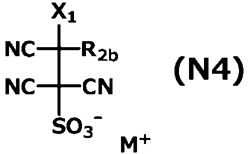 in the formula (N4), R_{2b}, X₁, and M⁺ respectively have the same meanings as R_{2b}, X₁, and M⁺ in the formula (N1).
[8] The actinic ray-sensitive or radiation-sensitive resin composition according to any one of [1] to [4], wherein the onium salt represented by the formula (N1) is represented by a formula (N5) below: 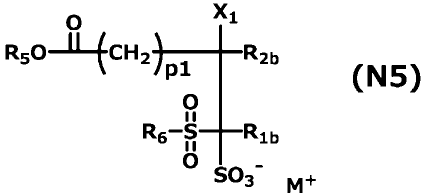
   in the formula (N5), R₅ and R₆ each independently represent an alkyl group, a cycloalkyl group, or an aryl group;
   p1 represents 0 or 1; and
   R₁₁, R_{2b}, X₁, and M⁺ respectively have the same meanings as R_{1b}, R_{2b}, X₁, and M⁺ in the formula (N1), and at least two of R₁₁, R_{2b}, R₆, and X₁ may be bonded together to form a ring.
[9] The actinic ray-sensitive or radiation-sensitive resin composition according to any one of [1] to [8], wherein M⁺ above is a sulfonium cation or an iodonium cation.
[10] A resist film formed using the actinic ray-sensitive or radiation-sensitive resin composition according to any one of [1] to [9].
[11] A pattern forming method including:
   a step of using the actinic ray-sensitive or radiation-sensitive resin composition according to any one of [1] to [9] to form a resist film on a substrate;
   a step of exposing the resist film; and
   a step of developing the exposed resist film using a developer.
[12] A method for producing an electronic device, the method including the pattern forming method according to [11].
[13] An onium salt represented by a formula (N2-1) below: 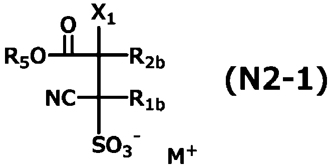
   wherein, in the formula (N2-1), R₅ represents an alkyl group, a cycloalkyl group, or an aryl group;
   R_{1b} and R_{2b} each independently represent a substituent having no fluorine atoms or a hydrogen atom;
   X₁ represents a hydrogen atom or an organic group;
   M⁺ represents an organic cation; and
   at least two of R₁₁, R_{2b}, and X₁ may be bonded together to form a ring.
[14] An onium salt represented by a formula (N5-1) below: 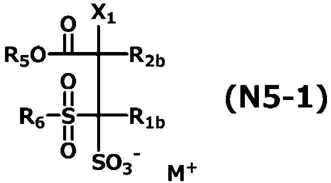
   wherein, in the formula (N5-1), R₅ and R₆ each independently represent an alkyl group, a cycloalkyl group, or an aryl group;
   R_{1b} and R_{2b} each independently represent a substituent having no fluorine atoms or a hydrogen atom;
   X₁ represents a hydrogen atom or an organic group;
   M⁺ represents an organic cation; and
   at least two of R₁₁, R_{2b}, R₆, and X₁ may be bonded together to form a ring.

The present invention can provide an actinic ray-sensitive or radiation-sensitive resin composition that has high LWR performance and can achieve reduction in the amount of post-development residue.

The present invention can also provide a resist film using the actinic ray-sensitive or radiation-sensitive resin composition, a pattern forming method, a method for producing an electronic device, and onium salts that can be used in the actinic ray-sensitive or radiation-sensitive resin composition.

### DESCRIPTION OF THE PREFERRED EMBODIMENTS

Hereinafter, the present invention will be described in detail.

In the following descriptions, features may be described on the basis of representative embodiments of the present invention; however, the present invention is not limited to such embodiments.

In this Specification, "actinic ray" or "radiation" means, for example, the emission line spectrum of a mercury lamp, far-ultraviolet rays represented by excimer lasers, extreme ultraviolet rays (EUV: Extreme Ultraviolet), X-rays, soft X-rays, or an electron beam (EB: Electron Beam).

In this Specification, "light" means an actinic ray or a radiation.

In this Specification, "exposure" includes, unless otherwise specified, not only exposure using, for example, the emission line spectrum of a mercury lamp, far-ultraviolet rays represented by excimer lasers, extreme ultraviolet rays, X-rays, or EUV, but also patterning using a corpuscular beam such as an electron beam or an ion beam.

In this Specification, "a value 'to' another value" is used to mean that it includes the value and the other value as the lower limit value and the upper limit value.

In this Specification, (meth)acrylate represents at least one of acrylate or methacrylate. (Meth)acrylic acid represents at least one of acrylic acid or methacrylic acid.

In this Specification, for resins, the weight-average molecular weight (Mw), the number-average molecular weight (Mn), and the dispersity (also referred to as molecular weight distribution) (Mw/Mn) are defined as polystyrene-equivalent values measured, using a GPC (Gel Permeation Chromatography) apparatus (HLC-8120GPC, manufactured by Tosoh Corporation), by GPC measurement (solvent: tetrahydrofuran, flow rate (sample injection amount): 10 µL, column: TSK gel Multipore HXL-M, manufactured by Tosoh Corporation, column temperature: 40°C, flow rate: 1.0 mL/min, detector: differential refractive index detector (Refractive Index Detector)).

In this Specification, for written forms of groups (atomic groups), written forms without referring to substituted or unsubstituted encompass, in addition to groups not having a substituent, groups including a substituent without departing from the spirit and scope of the present invention. For example, "alkyl group" encompasses not only alkyl groups not having a substituent (unsubstituted alkyl groups), but also alkyl groups having a substituent (substituted alkyl groups). In this Specification, "organic group" refers to a group including at least one carbon atom.

The substituent is preferably a monovalent substituent unless otherwise specified. Examples of the substituent include monovalent non-metallic atomic groups except for the hydrogen atom and, for example, can be selected from the group consisting of the following Substituents T.

### Substituents T

Examples of the substituents T include halogen atoms such as a fluorine atom, a chlorine atom, a bromine atom, and an iodine atom; alkoxy groups such as a methoxy group, an ethoxy group, and a tert-butoxy group; cycloalkyloxy groups; aryloxy groups such as a phenoxy group and a p-tolyloxy group; alkoxycarbonyl groups such as a methoxycarbonyl group and a butoxycarbonyl group; cycloalkyloxycarbonyl groups; aryloxycarbonyl groups such as a phenoxycarbonyl group; acyloxy groups such as an acetoxy group, a propionyloxy group, and a benzoyloxy group; acyl groups such as an acetyl group, a benzoyl group, an isobutyryl group, an acryloyl group, a methacryloyl group, and a methoxalyl group; a sulfanyl group; alkylsulfanyl groups such as a methylsulfanyl group and a tert-butylsulfanyl group; arylsulfanyl groups such as a phenylsulfanyl group and a p-tolylsulfanyl group; alkyl groups; alkenyl groups; cycloalkyl groups; aryl groups; aromatic heterocyclic groups; a hydroxy group; a carboxyl group; a formyl group; a sulfo group; a cyano group; alkylaminocarbonyl groups; arylaminocarbonyl groups; a sulfonamide group; a silyl group; an amino group; and a carbamoyl group. When such a substituent can additionally have one or more substituents, a group having, as the additional substituents, one or more substituents selected from the group consisting of the substituents described above (such as a monoalkylamino group, a dialkylamino group, an arylamino group, or a trifluoromethyl group) is also included in examples of the substituents T.

Note that, in the cases of descriptions of not having fluorine atoms such as the "substituent having no fluorine atoms" in R_{1b} and R_{2b} in the formula (N1), the substituents T do not include fluorine atoms.

In this Specification, the bonding directions of divalent groups described are not limited unless otherwise specified. For example, in a compound represented by a formula "X-Y-Z" where Y is -COO-, Y may be -CO-O- or may be -O-CO-. The compound may be "X-CO-O-Z" or may be "X-O-CO-Z".

In this Specification, the acid dissociation constant (pKa) represents pKa in an aqueous solution, specifically, a value determined using the following Software package 1, on the basis of the Hammett's substituent constant and the database of values in publicly known documents, by calculation. All the values of pKa described in this Specification are values determined by calculation using this software package.

### Software package 1: Advanced Chemistry Development (ACD/Labs) Software V8.14 for Solaris (1994-2007 ACD/Labs)

Alternatively, pKa can be determined by a molecular orbital calculation method. Specifically, this method may be a calculation method of calculating H⁺ dissociation free energy in an aqueous solution based on a thermodynamic cycle. The H⁺ dissociation free energy can be calculated by a method such as DFT (density functional theory); however, the calculation method is not limited thereto and various other methods have been reported in documents and the like. Note that there are a plurality of pieces of software for performing DFT, such as Gaussian 16.

In this Specification, as described above, pKa refers to a value determined using Software package 1, on the basis of the Hammett's substituent constant and the database of values in publicly known documents, by calculation; however, when use of this method cannot determine pKa, a value determined on the basis of DFT (density function theory) using Gaussian 16 is employed.

In this Specification, as described above, pKa refers to "pKa in an aqueous solution"; however, when pKa in an aqueous solution cannot be determined, "pKa in a dimethyl sulfoxide (DMSO) solution" is employed.

In this Specification, "solid content" means components forming the actinic ray-sensitive or radiation-sensitive film and does not include solvents. As long as a component forms the actinic ray-sensitive or radiation-sensitive film, even when the component has the form of liquid, it is regarded as the solid content.

### Actinic ray-sensitive or radiation-sensitive resin composition

An actinic ray-sensitive or radiation-sensitive resin composition of the present invention (also referred to as "the composition of the present invention") is an actinic ray-sensitive or radiation-sensitive resin composition containing a resin that is subjected to action of an acid to undergo an increase in polarity, and an onium salt represented by a formula (N1) below.

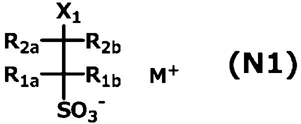

In the formula (N1), R₁ₐ represents a cyano group, a nitro group, or a substituent represented by a formula (1A) below. R_{1b} and R_{2b} each independently represent a substituent having no fluorine atoms or a hydrogen atom. R₂ₐ represents a cyano group, a nitro group, a substituent represented by the formula (1A) below, or a substituent represented by a formula (1B) below. X₁ represents a hydrogen atom or an organic group. M⁺ represents an organic cation. At least two of R₁ₐ, R₁₁, R₂ₐ, R_{2b}, and X₁ may be bonded together to form a ring.

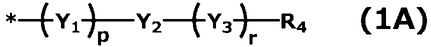

In the formula (1A), Y₁ and Y₃ each independently represent -O- or -NR₃-, and R₃ represents a hydrogen atom or an alkyl group. Y₂ represents -C(=O)- or -SO₂-. R₄ represents an alkyl group, a cycloalkyl group, or an aryl group. p and r each independently represent 0 or 1. * represents a bonding site.

***-CH₂-R_{Y}** **(1B)**

In the formula (1B), R_{Y} represents a cyano group, a nitro group, or a substituent represented by the formula (1A). * represents a bonding site.

The mechanism by which the composition of the present invention has high LWR performance and can achieve reduction in the amount of post-development residue is not clear, but is inferred by the inventors of the present invention as follows. Note that the present invention is not limited at all by the following inferred mechanism.

The onium salt represented by the formula (N1) contained in the composition of the present invention has high compatibility with the resin that is subjected to action of an acid to undergo an increase in polarity, so that the onium salt is homogeneously dispersed, and the variation in the acid generated from the onium salt is suppressed, so that high LWR performance is inferentially exerted. In addition, the onium salt represented by the formula (N1) is homogeneously dispersed, so that ionic aggregate that causes residue is less likely to be generated and the amount of post-development residue can be inferentially reduced. Furthermore, in the onium salt represented by the formula (N1), a cyano group, a nitro group, a substituent represented by the formula (1A), or a substituent represented by the formula (1B), which have electron-withdrawing properties, is bonded to the carbon atom to which -SO₃⁻ is bonded and the carbon atom bonded to the above-described carbon atom, and the acid strength of the acid generated from the onium salt can also be ensured.

The composition of the present invention is typically a resist composition, and may be a positive resist composition or may be a negative resist composition. The composition of the present invention may be a resist composition for alkali development or may be a resist composition for organic-solvent development.

The composition of the present invention may be a chemical amplification resist composition or may be a non-chemical amplification resist composition. The composition of the present invention is typically a chemical amplification resist composition.

The composition of the present invention can be used to form an actinic ray-sensitive or radiation-sensitive film. The actinic ray-sensitive or radiation-sensitive film formed using the composition of the present invention is typically a resist film.

Hereinafter, first, various components of the composition of the present invention will be described in detail.

### Onium salt represented by formula (N1)

The onium salt represented by the formula (N1) included in the composition of the present invention (also referred to as "onium salt (N1)") will be described.

The onium salt (N1) is preferably a compound that generates an acid upon irradiation with an actinic ray or a radiation (photoacid generator).

R₁ₐ in the formula (N1) represents a cyano group, a nitro group, or a substituent represented by the formula (1A).

In the formula (1A), Y₁ and Y₃ each independently represent -O- or -NR₃-, and R₃ represents a hydrogen atom or an alkyl group. The alkyl group represented by R₃ may be linear or branched, and is preferably an alkyl group having 1 to 12 carbon atoms, more preferably an alkyl group having 1 to 6 carbon atoms, and still more preferably an alkyl group having 1 to 3 carbon atoms.

Y₁ and Y₃ each preferably represent -O-.

p preferably represents 0.

r preferably represents 1.

In the formula (1A), Y₂ represents -C(=O)- or -SO₂-, and preferably represents -C(=O)-.

R₄ in the formula (1A) represents an alkyl group, a cycloalkyl group, or an aryl group. R₄ preferably represents an alkyl group or a cycloalkyl group.

The alkyl group represented by R₄ may be linear or branched, and is preferably an alkyl group having 1 to 12 carbon atoms, more preferably an alkyl group having 1 to 6 carbon atoms, and still more preferably an alkyl group having 1 to 3 carbon atoms. Examples of the alkyl group represented by R₄ include a methyl group, an ethyl group, an n-propyl group, an isopropyl group, an n-butyl group, an isobutyl group, a sec-butyl group, and a t-butyl group. The alkyl group represented by R₄ may have a substituent. The substituent that the alkyl group represented by R₄ may have is not particularly limited, but may be, for example, a cycloalkyl group or an aryl group. The descriptions and preferred ranges of the cycloalkyl group and the aryl group serving as the substituent that the alkyl group represented by R₄ may have are respectively the same as the following descriptions and preferred ranges of the cycloalkyl group and the aryl group represented by R₄.

The cycloalkyl group represented by R₄ may be monocyclic or polycyclic, and is preferably a cycloalkyl group having 3 to 20 carbon atoms, more preferably a cycloalkyl group having 4 to 15 carbon atoms, and still more preferably a cycloalkyl group having 5 to 10 carbon atoms. Examples of the cycloalkyl group represented by R₄ include a cyclopentyl group, a 1-methylcyclopentyl group, a cyclohexyl group, an adamantyl group, and a 1-ethyladamantyl group. The cycloalkyl group represented by R₄ may have a substituent. The substituent that the cycloalkyl group represented by R₄ may have is not particularly limited, but may be, for example, an alkyl group or an aryl group. The descriptions and preferred ranges of the alkyl group and the aryl group serving as the substituent that the cycloalkyl group represented by R₄ may have are respectively the same as the above descriptions and preferred ranges of the alkyl group represented by R₄ and the following descriptions and preferred ranges of the aryl group represented by R₄.

The aryl group represented by R₄ is preferably an aryl group having 6 to 20 carbon atoms, more preferably an aryl group having 6 to 15 carbon atoms, still more preferably an aryl group having 6 to 10 carbon atoms, particularly preferably a phenyl group or a naphthyl group, and most preferably a phenyl group. The aryl group represented by R₄ may have a substituent. The substituent that the aryl group represented by R₄ may have is not particularly limited, but may be, for example, an alkyl group or a cycloalkyl group. The descriptions and preferred ranges of the alkyl group and the cycloalkyl group serving as the substituent that the aryl group represented by R₄ may have are respectively the same as the above descriptions and preferred ranges of the alkyl group and the cycloalkyl group represented by R₄.

In the formula (N1), R₁ₐ preferably represents a cyano group, a nitro group, -COOR₄,-OCOOR₄, -OCOR₄, or -SO₂R₄, more preferably represents a cyano group, a nitro group,-COOR₄, -OCOOR₄, or -SO₂R₄, and still more preferably represents a cyano group, -COOR₄,-OCOOR₄, or -SO₂R₄. The definition, descriptions, and preferred ranges of R₄ are as described above.

R₂ₐ in the formula (N1) represents a cyano group, a nitro group, a substituent represented by the formula (1A), or a substituent represented by the formula (1B). When R₂ₐ is a substituent represented by the formula (1A), the descriptions and preferred ranges of the substituent represented by the formula (1A) are the same as the above descriptions and preferred ranges of the substituent represented by the formula (1A) in R₁ₐ.

In the formula (1B), R_{Y} represents a cyano group, a nitro group, or a substituent represented by the formula (1A). When R_{Y} is a substituent represented by the formula (1A), the descriptions and preferred ranges of the substituent represented by the formula (1A) are the same as the above descriptions and preferred ranges of the substituent represented by the formula (1A) in R₁ₐ.

R_{Y} preferably represents a cyano group, a nitro group, -COOR₄, -OCOOR₄, -OCOR₄, or -SO₂R₄, more preferably represents a cyano group, a nitro group, -COOR₄, -OCOOR₄, or-SO₂R₄, and still more preferably represents a cyano group, -COOR₄, -OCOOR₄, or -SO₂R₄. The definition, descriptions, and preferred ranges of R₄ are as described above.

In the formula (N1), R₂ₐ preferably represents a cyano group, a nitro group, -COOR₄, -OCOOR₄, -OCOR₄, -SO₂R₄, or a substituent represented by the following formula (1C), more preferably represents a cyano group, a nitro group, -COOR₄, -OCOOR₄, -SO₂R₄, or a substituent represented by the following formula (1C), and still more preferably represents a cyano group, -COOR₄, -OCOOR₄, -SO₂R₄, or a substituent represented by the following formula (1C). The definition, descriptions, and preferred ranges of R₄ are as described above.

***-CH₂-R_{Y2}** **(1C)**

In the formula (1C), R_{Y2} represents a cyano group, a nitro group, -COOR₄, -OCOOR₄, or -SO₂R₄. R₄ has the same meaning as R₄ in the formula (1A). * represents a bonding site. The descriptions and preferred ranges of R₄ are as described above.

In the formula (N1), R_{1b} represents a substituent not having fluorine atoms or a hydrogen atom.

The substituent not having fluorine atoms represented by R_{1b} is not particularly limited as long as it does not have fluorine atoms, but may be, for example, an organic group or a nitro group.

The organic group is not particularly limited, but is, for example, preferably a cyano group, an alkyl group, a cycloalkyl group, an aryl group, a substituent represented by the formula (1A), or a substituent represented by the formula (1B).

The alkyl group may be linear or branched, and is preferably an alkyl group having 1 to 12 carbon atoms, more preferably an alkyl group having 1 to 6 carbon atoms, and still more preferably an alkyl group having 1 to 3 carbon atoms. Examples of the alkyl group include a methyl group, an ethyl group, an n-propyl group, an isopropyl group, an n-butyl group, an isobutyl group, a sec-butyl group, and a t-butyl group.

The cycloalkyl group may be monocyclic or polycyclic, and is preferably a cycloalkyl group having 3 to 20 carbon atoms, more preferably a cycloalkyl group having 4 to 15 carbon atoms, and still more preferably a cycloalkyl group having 5 to 10 carbon atoms. Examples of the cycloalkyl group include a cyclopentyl group, a 1-methylcyclopentyl group, a cyclohexyl group, an adamantyl group, and a 1-ethyladamantyl group.

The aryl group is preferably an aryl group having 6 to 20 carbon atoms, more preferably an aryl group having 6 to 15 carbon atoms, still more preferably an aryl group having 6 to 10 carbon atoms, particularly preferably a phenyl group or a naphthyl group, and most preferably a phenyl group.

The alkyl group, the cycloalkyl group, and the aryl group may have a substituent other than a fluorine atom.

When R_{1b} is the substituent represented by the formula (1A) and the substituent represented by the formula (1B), the descriptions and preferred ranges of the substituent represented by the formula (1A) and the substituent represented by the formula (1B) are respectively the same as the descriptions and preferred ranges of the substituent represented by the formula (1A) and the substituent represented by the formula (1B) in R₂ₐ.

The definition, descriptions, and preferred ranges of R_{2b} in the formula (N1) are the same as the definition, descriptions, and preferred ranges of R_{1b}.

In the formula (N1), X₁ represents a hydrogen atom or an organic group.

The organic group is not particularly limited, but is, for example, preferably a cyano group, an alkyl group, a cycloalkyl group, an aryl group, a substituent represented by the formula (1A), or a substituent represented by the formula (1B).

The alkyl group may be linear or branched, and is preferably an alkyl group having 1 to 12 carbon atoms, more preferably an alkyl group having 1 to 6 carbon atoms, and still more preferably an alkyl group having 1 to 3 carbon atoms. Examples of the alkyl group include a methyl group, an ethyl group, an n-propyl group, an isopropyl group, an n-butyl group, an isobutyl group, a sec-butyl group, and a t-butyl group.

The cycloalkyl group may be monocyclic or polycyclic, and is preferably a cycloalkyl group having 3 to 20 carbon atoms, more preferably a cycloalkyl group having 4 to 15 carbon atoms, and still more preferably a cycloalkyl group having 5 to 10 carbon atoms. Examples of the cycloalkyl group include a cyclopentyl group, a 1-methylcyclopentyl group, a cyclohexyl group, an adamantyl group, and a 1-ethyladamantyl group.

The aryl group is preferably an aryl group having 6 to 20 carbon atoms, more preferably an aryl group having 6 to 15 carbon atoms, still more preferably an aryl group having 6 to 10 carbon atoms, particularly preferably a phenyl group or a naphthyl group, and most preferably a phenyl group.

The alkyl group, the cycloalkyl group, and the aryl group may have a substituent. The substituent preferably does not include fluorine atoms.

When X₁ is a substituent represented by the formula (1A) and a substituent represented by the formula (1B), the descriptions and preferred ranges of the substituent represented by the formula (1A) and the substituent represented by the formula (1B) are respectively the same as the descriptions and preferred ranges of the substituent represented by the formula (1A) and the substituent represented by the formula (1B) in R₂ₐ.

In the formula (N1), M⁺ represents an organic cation.

M⁺ is preferably a sulfonium cation or an iodonium cation.

The cation represented by M⁺ is not particularly limited. The cation may be monovalent, or di- or higher valent. The cation is preferably a cation represented by a formula (ZaI) (hereafter, also referred to as "cation (ZaI)") or a cation represented by a formula (ZaII) (hereafter, also referred to as "cation (ZaII)").

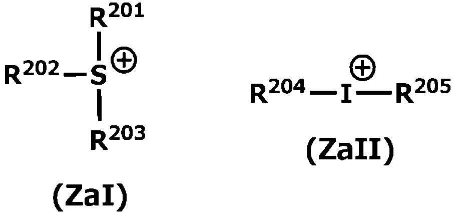

In the formula (ZaI), R²⁰¹, R²⁰², and R²⁰³ each independently represent an organic group.

For R²⁰¹, R²⁰², and R²⁰³, the organic group preferably has 1 to 30 carbon atoms, and more preferably 1 to 20 carbon atoms. Of R²⁰¹ to R²⁰³, two may be bonded together to form a ring structure and the ring may include an oxygen atom, a sulfur atom, an ester group, an amide group, or a carbonyl group. Examples of the group formed by bonding together two of R²⁰¹ to R²⁰³ include alkylene groups (such as a butylene group and a pentylene group), and -CH₂-CH₂-O-CH₂-CH₂-.

Preferred examples of the organic cation in the formula (ZaI) include a cation (ZaI-1), a cation (ZaI-2), a cation (ZaI-3b), and a cation (ZaI-4b) described later.

First, the cation (ZaI-1) will be described.

The cation (ZaI-1) is an aryl sulfonium cation represented by the above-described formula (ZaI) where at least one of R²⁰¹ to R²⁰³ is an aryl group.

In the aryl sulfonium cation, all of R²⁰¹ to R²⁰³ may be aryl groups, or a part of R²⁰¹ to R²⁰³ may be an aryl group and the other may be an alkyl group or a cycloalkyl group.

Alternatively, one of R²⁰¹ to R²⁰³ may be an aryl group and the other two of R²⁰¹ to R²⁰³ may be bonded together to form a ring structure in which the ring may include an oxygen atom, a sulfur atom, an ester group, an amide group, or a carbonyl group. Examples of the group formed by bonding together two of R²⁰¹ to R²⁰³ include alkylene groups in which one or more methylene groups may be substituted with an oxygen atom, a sulfur atom, an ester group, an amide group, and/or a carbonyl group (such as a butylene group, a pentylene group, and -CH₂-CH₂-O-CH₂-CH₂-).

Examples of the aryl sulfonium cation include triaryl sulfonium cations, diaryl alkyl sulfonium cations, aryl dialkyl sulfonium cations, diaryl cycloalkyl sulfonium cations, and aryl dicycloalkyl sulfonium cations.

The aryl group included in the aryl sulfonium cation is preferably a phenyl group or a naphthyl group, and more preferably a phenyl group. The aryl group may be an aryl group having a heterocyclic structure having an oxygen atom, a nitrogen atom, a sulfur atom, or the like. Examples of the heterocyclic structure include a pyrrole residue, a furan residue, a thiophene residue, an indole residue, a benzofuran residue, and a benzothiophene residue. When the aryl sulfonium cation has two or more aryl groups, the two or more aryl groups may be the same or different.

The alkyl group or cycloalkyl group that the aryl sulfonium cation has as needed is preferably a linear alkyl group having 1 to 15 carbon atoms, a branched alkyl group having 3 to 15 carbon atoms, or a cycloalkyl group having 3 to 15 carbon atoms, and more preferably a methyl group, an ethyl group, a propyl group, an n-butyl group, a sec-butyl group, a t-butyl group, a cyclopropyl group, a cyclobutyl group, or a cyclohexyl group.

For R²⁰¹ to R²⁰³, a substituent that the aryl group, the alkyl group, and the cycloalkyl group may have is preferably an alkyl group (having, for example, 1 to 15 carbon atoms), a cycloalkyl group (having, for example, 3 to 15 carbon atoms), an aryl group (having, for example, 6 to 14 carbon atoms), an alkoxy group (having, for example, 1 to 15 carbon atoms), a cycloalkylalkoxy group (having, for example, 1 to 15 carbon atoms), a halogen atom (for example, fluorine or iodine), a hydroxyl group, a carboxyl group, an ester group, a sulfinyl group, a sulfonyl group, an alkylthio group, or a phenylthio group.

The substituent may further have, when possible, a substituent; the alkyl group also preferably has, as a substituent, a halogen atom to serve as an alkyl halide group such as a trifluoromethyl group.

Such substituents are also preferably provided as appropriate combinations to form acid-decomposable groups.

Hereinafter, the cation (ZaI-2) will be described.

The cation (ZaI-2) is a cation represented by the formula (ZaI) where R²⁰¹ to R²⁰³ each independently represent an organic group not having an aromatic ring. The aromatic ring also encompasses aromatic rings including a heteroatom.

For R²⁰¹ to R²⁰³, the organic group not having an aromatic ring preferably has 1 to 30 carbon atoms and more preferably 1 to 20 carbon atoms.

R²⁰¹ to R²⁰³ are each independently preferably an alkyl group, a cycloalkyl group, an allyl group, or a vinyl group, more preferably a linear or branched 2-oxoalkyl group, a 2-oxocycloalkyl group, or an alkoxycarbonylmethyl group, and still more preferably a linear or branched 2-oxoalkyl group.

For R²⁰¹ to R²⁰³, the alkyl group and the cycloalkyl group may be, for example, a linear alkyl group having 1 to 10 carbon atoms or a branched alkyl group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

R²⁰¹ to R²⁰³ may be further substituted with a halogen atom, an alkoxy group (having, for example, 1 to 5 carbon atoms), a hydroxy group, a cyano group, or a nitro group.

For R²⁰¹ to R²⁰³, substituents are also preferably provided independently as appropriate combinations of substituents to form acid-decomposable groups.

Hereinafter, the cation (ZaI-3b) will be described.

The cation (ZaI-3b) is a cation represented by the following formula (ZaI-3b).

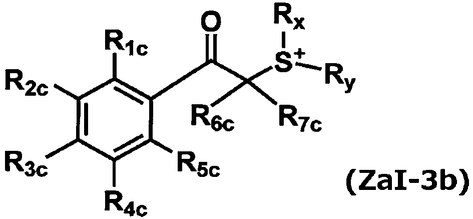

In the formula (ZaI-3b), R_{1c} to R₅, each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a halogen atom, a hydroxy group, a nitro group, an alkylthio group, or an arylthio group.

R_{6c} and R_{7c} each independently represent a hydrogen atom, an alkyl group (for example, a t-butyl group), a cycloalkyl group, a halogen atom, a cyano group, or an aryl group.

Rₓ and R_{y} each independently represent an alkyl group, a cycloalkyl group, a 2-oxoalkyl group, a 2-oxocycloalkyl group, an alkoxycarbonylalkyl group, an allyl group, or a vinyl group.

For R_{1c} to R_{7c} and Rₓ and R_{y}, such substituents are also preferably provided independently as appropriate combinations of substituents to form acid-decomposable groups.

Any two or more of R_{1c} to R_{5c}, R_{5c} and R_{6c}, R_{6c} and R_{7c}, R_{5c} and Rₓ, and Rₓ and R_{y} may be individually bonded together to form rings; these rings may each independently include an oxygen atom, a sulfur atom, a ketone group, an ester bond, or an amide bond.

Such a ring may be an aromatic or non-aromatic hydrocarbon ring, an aromatic or non-aromatic heterocycle, or a polycyclic fused ring formed as a combination of two or more of these rings. The ring may be a 3- to 10-membered ring, and is preferably a 4- to 8-membered ring, and more preferably a 5- or 6-membered ring.

Examples of the groups formed by bonding together any two or more of R_{1c} to R_{5c}, R_{6c} and R_{7c}, and Rₓ and R_{y} include alkylene groups such as a butylene group and a pentylene group. In such an alkylene group, a methylene group may be substituted with a heteroatom such as an oxygen atom.

The groups formed by bonding together R_{5c} and R_{6c}, and R_{5c} and Rₓ are preferably single bonds or alkylene groups. Examples of the alkylene groups include a methylene group and an ethylene group.

R_{1c} to R_{5c}, R_{6c}, R_{7c}, Rₓ, R_{y}, and the rings formed by individually bonding together any two or more of R_{1c} to R_{5c}, R_{5c} and R_{6c}, R_{6c} and R_{7c}, R_{5c} and Rₓ, and Rₓ and R_{y} may have a substituent.

Hereinafter, the cation (ZaI-4b) will be described.

The cation (ZaI-4b) is a cation represented by the following formula (ZaI-4b).

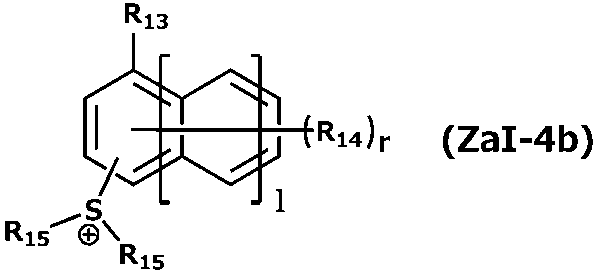

In the formula (ZaI-4b), 1 represents an integer of 0 to 2, and r represents an integer of 0 to 8.

R₁₃ represents a hydrogen atom, a halogen atom (for example, a fluorine atom or an iodine atom), a hydroxyl group, an alkyl group, an alkyl halide group, an alkoxy group, a carboxyl group, an alkoxycarbonyl group, or a group including a cycloalkyl group (may be the cycloalkyl group itself or may be a group including, as a part thereof, the cycloalkyl group). These groups may have a substituent.

R₁₄ represents a hydroxyl group, a halogen atom (for example, a fluorine atom or an iodine atom), an alkyl group, an alkyl halide group, an alkoxy group, an alkoxycarbonyl group, an alkylcarbonyl group, an alkylsulfonyl group, a cycloalkylsulfonyl group, or a group including a cycloalkyl group (may be the cycloalkyl group itself or may be a group including, as a part thereof, the cycloalkyl group). These groups may have a substituent. When a plurality of R₁₄'s are present, R₁₄'s each independently represent such a group, for example, a hydroxyl group.

R₁₅'s each independently represent an alkyl group, a cycloalkyl group, or a naphthyl group. Two R₁₅'s may be bonded together to form a ring. When two R₁₅'s are bonded together to form a ring, the ring skeleton may include a heteroatom such as an oxygen atom or a nitrogen atom.

In an example, two R₁₅'s are preferably alkylene groups and bonded together to form a ring structure. Note that the alkyl group, the cycloalkyl group, the naphthyl group, and the ring formed by bonding together two R₁₅'s may have a substituent.

In the formula (ZaI-4b), for R₁₃, R₁₄, and R₁₅, the alkyl groups may be linear or branched. Such an alkyl group preferably has 1 to 10 carbon atoms. Preferred examples of the alkyl group include a methyl group, an ethyl group, an n-butyl group, and a t-butyl group.

For R₁₃ to R₁₅, and Rₓ and R_{y}, such substituents are also preferably provided independently as appropriate combinations of substituents to form acid-decomposable groups.

Hereinafter, the formula (ZaII) will be described.

In the formula (ZaII), R²⁰⁴ and R²⁰⁵ each independently represent an aryl group, an alkyl group, or a cycloalkyl group.

For R²⁰⁴ and R²⁰⁵, the aryl group is preferably a phenyl group or a naphthyl group, and more preferably a phenyl group. For R²⁰⁴ and R²⁰⁵, the aryl group may be an aryl group having a heterocycle having an oxygen atom, a nitrogen atom, a sulfur atom, or the like. Examples of the skeleton of the aryl group having a heterocycle include pyrrole, furan, thiophene, indole, benzofuran, and benzothiophene.

For R²⁰⁴ and R²⁰⁵, the alkyl group and the cycloalkyl group are preferably a linear alkyl group having 1 to 10 carbon atoms, a branched alkyl group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

For R²⁰⁴ and R²⁰⁵, the aryl group, the alkyl group, and the cycloalkyl group may each independently have a substituent. For R²⁰⁴ and R²⁰⁵, examples of the substituent that the aryl group, the alkyl group, and the cycloalkyl group may have include alkyl groups (having, for example, 1 to 15 carbon atoms), cycloalkyl groups (having, for example, 3 to 15 carbon atoms), aryl groups (having, for example, 6 to 15 carbon atoms), alkoxy groups (having, for example, 1 to 15 carbon atoms), halogen atoms, a hydroxy group, and a phenylthio group. For R²⁰⁴ and R²⁰⁵ substituents are also preferably provided independently as appropriate combinations of substituents to form acid-decomposable groups.

The following are non-limiting specific examples of the cation represented by M⁺.

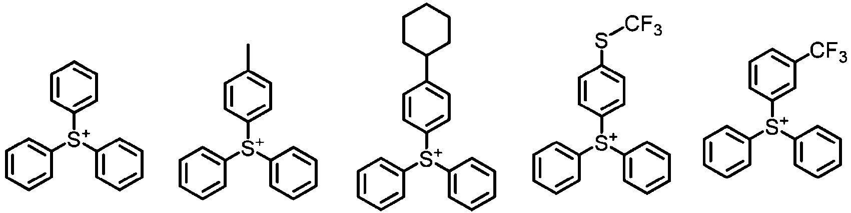

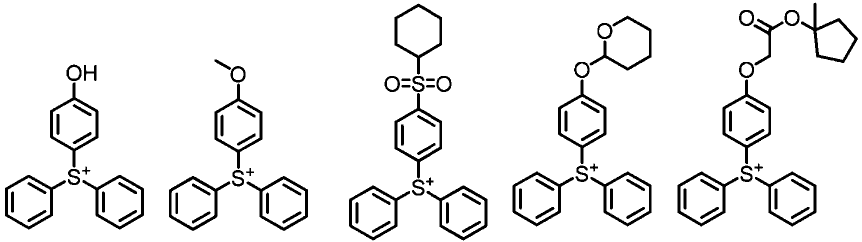

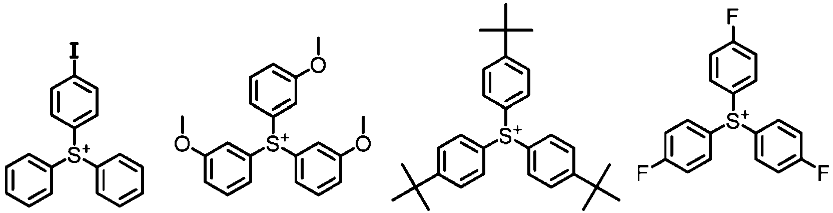

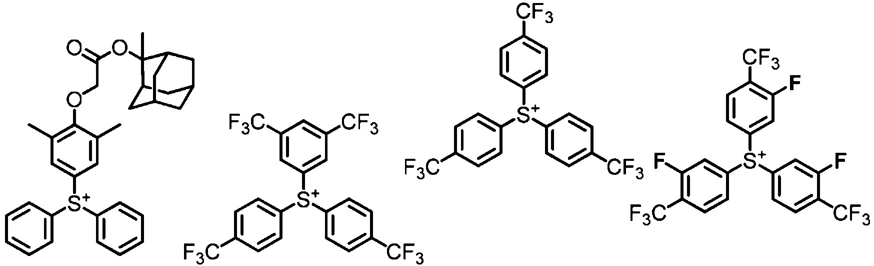

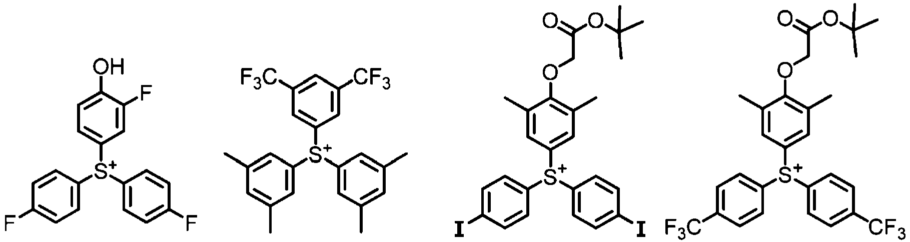

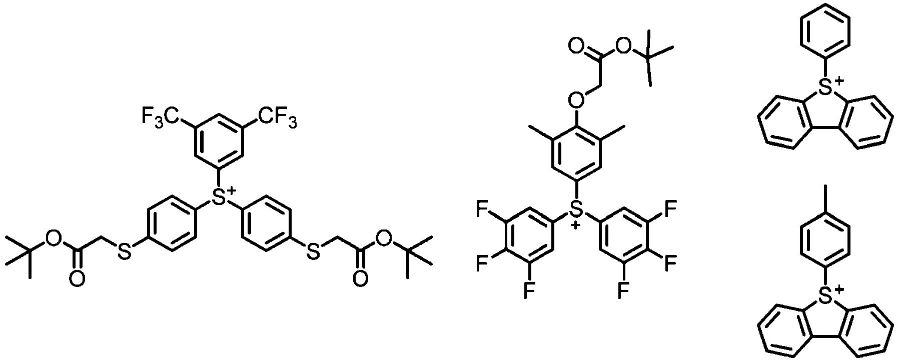

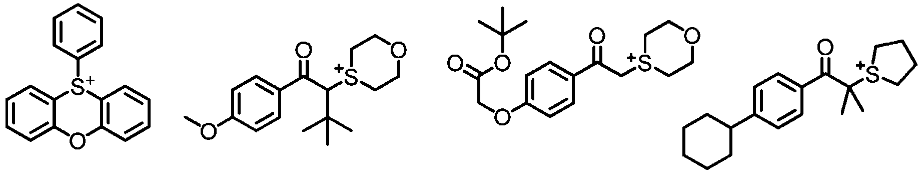

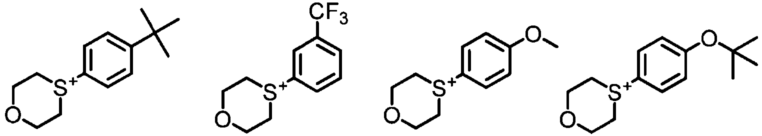

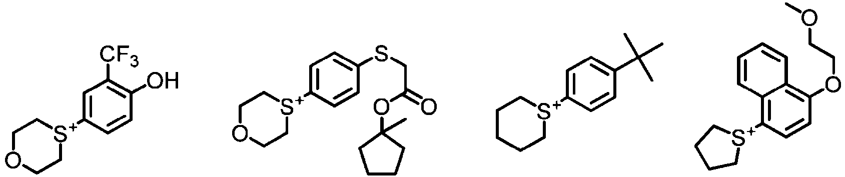

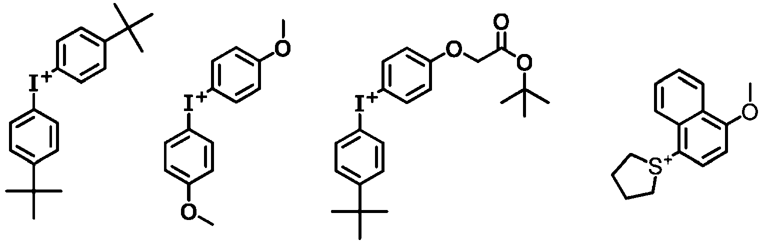

### Preferred embodiment of onium salt (N1)

For the reason that the composition of the present invention has further improved LWR performance and can achieve a further reduction in the amount of post-development residue, preferably, when R₁ₐ, R₁₁, R₂ₐ, and R_{2b} in the formula (N1) correspond to (i) below, R_{2b} represents a nitro group, a substituent represented by the formula (1A), or a substituent represented by the formula (1B),
when R₁ₐ, R₁₁, R₂ₐ, and R_{2b} in the formula (N1) correspond to (ii) below, X₁ represents an organic group, or
when R₁ₐ, R₁₁, and R₂ₐ in the formula (N1) correspond to (iii) below, X₁ represents an organic group:
   (i) a case where both of R₁ₐ and R_{1b} are not substituents represented by the formula (1A) and only one of R₁ₐ and R_{1b} is a cyano group, R₂ₐ is a cyano group, and R₂₆ is not a cyano group,
   (ii) a case where only one of R₁ₐ and R_{1b} is a cyano group, and both of R₂ₐ and R_{2b} are cyano groups, or
   (iii) a case where R₁ₐ is a substituent represented by the formula (1A), and Y₂ in the formula (1A) is -C(=O)-, R_{1b} is a hydrogen atom, and R₂ₐ is a substituent represented by the formula (1A).

Note that cases not corresponding to any of (i) to (iii) above are also included in preferred embodiments of the onium salt (N1).

The onium salt represented by the formula (N1) is preferably represented by the following formula (N2).

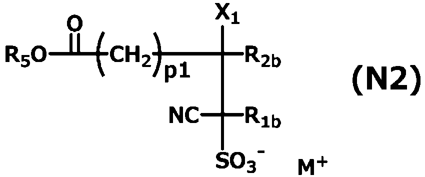

In the formula (N2), R₅ represents an alkyl group, a cycloalkyl group, or an aryl group. p1 represents 0 or 1. R₁₁, R_{2b}, X₁, and M⁺ respectively have the same meanings as R_{1b}, R_{2b}, X₁, and M⁺ in the formula (N1).

For R₅ in the formula (N2), the descriptions and preferred ranges of the alkyl group, the cycloalkyl group, and the aryl group are respectively the same as the descriptions and preferred ranges of the alkyl group, the cycloalkyl group, and the aryl group represented by R₄.

The descriptions and preferred ranges of R₁₁, R_{2b}, X₁, and M⁺ in the formula (N2) are respectively the same as the descriptions and preferred ranges of R_{1b}, R_{2b}, X₁, and M⁺ in the formula (N1).

The onium salt represented by the formula (N1) is also preferably an onium salt represented by the following formula (N2-1).

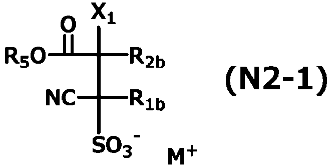

In the formula (N2-1), R₅ represents an alkyl group, a cycloalkyl group, or an aryl group. R_{1b} and R_{2b} each independently represent a substituent having no fluorine atoms or a hydrogen atom. X₁ represents a hydrogen atom or an organic group. M⁺ represents an organic cation. At least two of R₁₁, R_{2b}, and X₁ may be bonded together to form a ring.

For R₅ in the formula (N2-1), the descriptions and preferred ranges of the alkyl group, the cycloalkyl group, and the aryl group are respectively the same as the above descriptions and preferred ranges of the alkyl group, the cycloalkyl group, and the aryl group represented by R₄.

In the formula (N2), the descriptions and preferred ranges of R₁₁, R_{2b}, X₁, and M⁺ are respectively the same as the descriptions and preferred ranges of R_{1b}, R_{2b}, X₁, and M⁺ in the formula (N1).

The onium salt represented by the formula (N1) is also preferably represented by the following formula (N3).

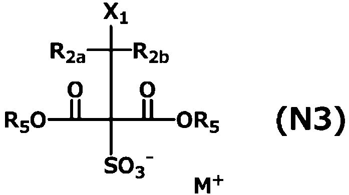

In the formula (N3), R₅ represent an alkyl group, a cycloalkyl group, or an aryl group, and the plurality of R₅ may be the same or different. R₂ₐ, R_{2b}, X₁, and M⁺ respectively have the same meanings as R₂ₐ, R_{2b}, X₁, and M⁺ in the formula (N1).

For R₅ in the formula (N3), the descriptions and preferred ranges of the alkyl group, the cycloalkyl group, and the aryl group are the same as the above descriptions and preferred ranges of the alkyl group, the cycloalkyl group, and the aryl group represented by R₄.

In the formula (N3), the descriptions and preferred ranges of R₂ₐ, R_{2b}, X₁, and M⁺ are respectively the same as the descriptions and preferred ranges of R₂ₐ, R_{2b}, X₁, and M⁺ in the formula (N1).

The onium salt represented by the formula (N1) is also preferably represented by the following formula (N4).

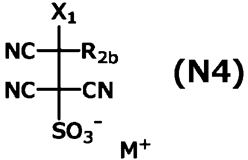

In the formula (N4), R_{2b}, X₁, and M⁺ respectively have the same meanings as R_{2b}, X₁, and M⁺ in the formula (N1).

In the formula (N4), the descriptions and preferred ranges of R_{2b}, X₁, and M⁺ are respectively the same as the descriptions and preferred ranges of R₂₆, X₁, and M⁺ in the formula (N1).

The onium salt represented by the formula (N1) is also preferably represented by the following formula (N5).

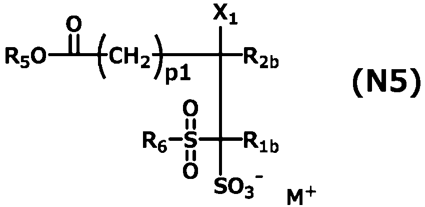

In the formula (N5), R₅ and R₆ each independently represent an alkyl group, a cycloalkyl group, or an aryl group. p1 represents 0 or 1. R_{1b}, R_{2b}, X₁, and M⁺ respectively have the same meanings as R₁₁, R_{2b}, X₁, and M⁺ in the formula (N1). At least two of R_{1b}, R_{2b}, R₆, and X₁ may be bonded together to form a ring.

For R₅ and R₆ in the formula (N5), the descriptions and preferred ranges of the alkyl group, the cycloalkyl group, and the aryl group are respectively the same as the above descriptions and preferred ranges of the alkyl group, the cycloalkyl group, and the aryl group represented by R₄.

In the formula (N5), the descriptions and preferred ranges of R₁₁, R_{2b}, X₁, and M⁺ are respectively the same as the descriptions and preferred ranges of R_{1b}, R_{2b}, X₁, and M⁺ in the formula (N1).

The onium salt represented by the formula (N1) is also preferably an onium salt represented by the following formula (N5-1).

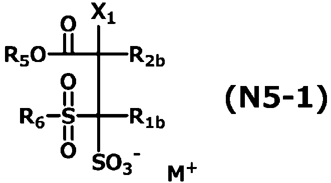

In the formula (N5-1), R₅ and R₆ each independently represent an alkyl group, a cycloalkyl group, or an aryl group. R_{1b} and R_{2b} each independently represent a substituent having no fluorine atoms or a hydrogen atom. X₁ represents a hydrogen atom or an organic group. M⁺ represents an organic cation. At least two of R₁₁, R_{2b}, R₆, and X₁ may be bonded together to form a ring.

For R₅ and R₆ in the formula (N5-1), the descriptions and preferred ranges of the alkyl group, the cycloalkyl group, and the aryl group are respectively the same as the above descriptions and preferred ranges of the alkyl group, the cycloalkyl group, and the aryl group represented by R₄.

In the formula (N5-1), the descriptions and preferred ranges of R₁₁, R_{2b}, X₁, and M⁺ are respectively the same as the descriptions and preferred ranges of R_{1b}, R_{2b}, X₁, and M⁺ in the formula (N1).

The onium salt (N1) may be in the form of a low-molecular-weight compound, or may be in the form of being incorporated into a portion of a polymer. Alternatively, the form of a low-molecular-weight compound and the form of being incorporated into a portion of a polymer may be used in combination.

When the onium salt (N1) is in the form of a low-molecular-weight compound, the molecular weight of the onium salt (N1) is not particularly limited, but is, for example, preferably 5000 or less, more preferably 3000 or less, and particularly preferably 2000 or less. The onium salt (N1) has a molecular weight of preferably 100 or more, and more preferably 200 or more.

When the onium salt (N1) is in the form of being incorporated into a portion of a polymer, it may be incorporated into a portion of the resin (A) or may be incorporated into a resin different from the resin (A).

The onium salt (N1) is preferably in the form of a low-molecular-weight compound.

The content of the onium salt (N1) in the composition of the present invention is not particularly limited, but is, relative to the total solid content of the composition of the present invention, preferably 1 mass% or more and 50 mass% or less, more preferably 5 mass% or more and 45 mass% or less, and still more preferably 10 mass% or more and 40 mass% or less.

Such onium salts (N1) may be used alone or in combination of two or more thereof. When two or more thereof are used, the total content thereof is preferably within such a preferred content range.

The method for producing the onium salt (N1) is not particularly limited, but, for example, the onium salt (N1) can be synthesized by the following method.

(1) 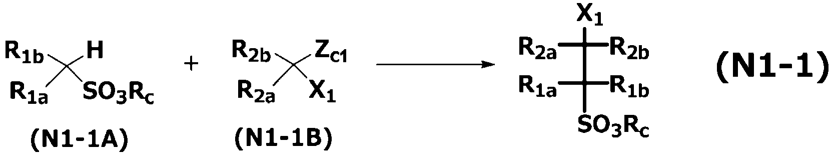
(2) 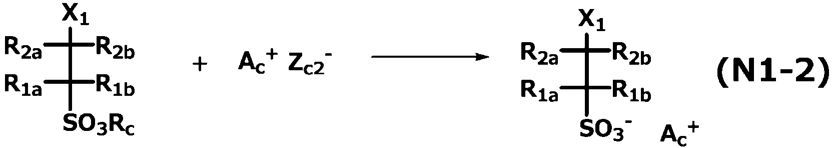
(3)

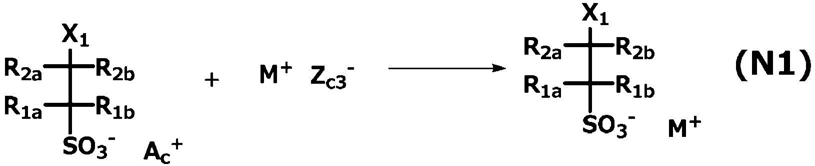

In the step (1), the intermediate (N1-1) is synthesized under strongly basic conditions. In the formula, R_{c} represents an alkyl group, and is preferably an ethyl group, a propyl group, a butyl group, or an isobutyl group. Z_{c1} represents a halogen atom, and is preferably a chlorine atom, a bromine atom, or an iodine atom. The base used is preferably strongly basic and has low nucleophilicity, and is preferably sodium hydride or potassium tert-butoxide. The reaction solvent is not particularly limited as long as it is a solvent that does not react with the base used, but is preferably an ether-based solvent such as tetrahydrofuran, an amide-based solvent such as N,N-dimethylformamide, or a mixed solvent of the foregoing. The reaction temperature is preferably 0°C to 80°C. The reaction is preferably performed in a nitrogen atmosphere.

In the step (1), the raw material (N1-1A) can be produced using commercially available reagents by an ordinary esterification or sulfonation method. Alternatively, the raw material (N1-1A) can also be produced by methods other than the above-described methods. For example, a sulfonic ester in which R₁ₐ is CH₃SO₂- and R_{1b} is H can be synthesized with reference to Non Patent Literature "Journal of Medicinal Chemistry, vol. 27 (1984), pp. 664 to 670".

In the step (1), the raw material (N1-1B) can be produced using commercially available reagents by an ordinary esterification or halogenation method.

In the step (2), the intermediate (N1-1) obtained in the step (1) is used to synthesize the intermediate (N1-2). In the formula, A_{c} represents an alkali metal atom, and is preferably sodium or potassium. Z_{c2} represents a halogen atom, and is preferably an iodine atom. A_{c}⁺Z_{c2}⁻is preferably sodium iodide. The reaction solvent is preferably a solvent that dissolves A_{c}⁺Z_{c2}⁻, and is preferably a nitrile-based solvent such as acetonitrile or a ketone-based solvent such as acetone. The reaction temperature is preferably 20°C to 100°C.

In the step (3), the intermediate (N1-2) obtained in the step (2) is used to synthesize the onium salt (N1). Z_{c3} represents a halogen atom, and is preferably a chlorine atom or a bromine atom. For the reaction solvent, a halogen-based solvent such as methylene chloride and water are used, and the reaction is preferably performed in a two layer system. The reaction temperature is preferably 0°C to 50°C.

The onium salt (N1) can also be produced by methods other than the above-described methods. For example, a compound in which R₁ₐ is CN, R_{1b} is H, R₂ₐ is -COOR₄, R_{2b} is H, and X₁ is H can be synthesized with reference to Non Patent Literature "Journal of Medicinal Chemistry, vol. 54 (2011), pp. 3606 to 3623".

### Resin that is subjected to action of acid to undergo increase in polarity

The composition of the present invention contains a resin that is subjected to the action of an acid to undergo an increase in polarity (also referred to as "resin (A)").

The resin (A) ordinarily includes a group that is decomposed by the action of an acid to undergo an increase in polarity (also referred to as an "acid-decomposable group"), and preferably includes a repeating unit having an acid-decomposable group. When the resin (A) has an acid-decomposable group, in a pattern forming method in this Specification, typically, in the case of employing a developer that is an alkali developer, a positive-type pattern is suitably formed or, in the case of employing a developer that is an organic-based developer, a negative-type pattern is suitably formed.

The repeating unit having an acid-decomposable group is, in addition to the repeating unit having an acid-decomposable group, preferably a repeating unit having an acid-decomposable group including an unsaturated bond.

### Repeating unit having acid-decomposable group

The acid-decomposable group refers to a group that is decomposed by the action of an acid to generate a polar group. The acid-decomposable group preferably has a structure in which the polar group is protected with a group (leaving group) that leaves by the action of an acid. Thus, the resin (A) has a repeating unit having a group that is decomposed by the action of an acid to generate a polar group. The resin having the repeating unit is subjected to the action of an acid to undergo an increase in polarity to undergo an increase in the degree of solubility in the alkali developer, but undergo a decrease in the degree of solubility in organic solvents.

The polar group is preferably an alkali-soluble group; examples include acidic groups such as a carboxyl group, a phenolic hydroxyl group, fluorinated alcohol groups, a sulfonic acid group, a phosphoric acid group, a sulfonamide group, a sulfonylimide group, (alkylsulfonyl)(alkylcarbonyl)methylene groups, (alkylsulfonyl)(alkylcarbonyl)imide groups, bis(alkylcarbonyl)methylene groups, bis(alkylcarbonyl)imide groups, bis(alkylsulfonyl)methylene groups, bis(alkylsulfonyl)imide groups, tris(alkylcarbonyl)methylene groups, and tris(alkylsulfonyl)methylene groups, and an alcoholic hydroxyl group.

In particular, the polar group is preferably a carboxyl group, a phenolic hydroxy group, a fluorinated alcohol group (preferably a hexafluoroisopropanol group), or a sulfonic acid group.

Examples of the group that leaves by the action of an acid include groups represented by formulas (Y1) to (Y4).

formula (Y1): -C(Rx₁)(Rx₂)(Rx₃)

formula (Y2): -C(=O)OC(Rx₁)(Rx₂)(Rx₃)

formula (Y3): -C(R₃₆)(R₃₇)(OR₃₈)

formula (Y4): -C(Rn)(H)(Ar)

In the formula (Y1) and the formula (Y2), Rx₁ to Rx₃ each independently represent an alkyl group (linear or branched), a cycloalkyl group (monocyclic or polycyclic), an alkenyl group (linear or branched), or an aryl group (monocyclic or polycyclic). Note that, when Rx₁ to Rx₃ are all alkyl groups (linear or branched), at least two of Rx₁ to Rx₃ are preferably methyl groups.

In particular, Rx₁ to Rx₃ preferably each independently represent a linear or branched alkyl group, and Rx₁ to Rx₃ more preferably each independently represent a linear alkyl group.

Two of Rx₁ to Rx₃ may be bonded together to form a monocycle or a polycycle.

For Rx₁ to Rx₃, the alkyl group is preferably an alkyl group having 1 to 5 carbon atoms such as a methyl group, an ethyl group, an n-propyl group, an isopropyl group, an n-butyl group, an isobutyl group, or a t-butyl group.

For Rx₁ to Rx₃, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For Rx₁ to Rx₃, the aryl group is preferably an aryl group having 6 to 10 carbon atoms, and may be, for example, a phenyl group, a naphthyl group, or an anthryl group.

For Rx₁ to Rx₃, the alkenyl group is preferably a vinyl group.

The ring formed by bonding together two of Rx₁ to Rx₃ is preferably a cycloalkyl group. The cycloalkyl group formed by bonding together two of Rx₁ to Rx₃ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group, and more preferably a monocyclic cycloalkyl group having 5 to 6 carbon atoms.

In the cycloalkyl group formed by bonding together two of Rx₁ to Rx₃, one of methylene groups constituting the ring may be replaced by a heteroatom such as an oxygen atom, a group including a heteroatom such as a carbonyl group, or a vinylidene group. In the cycloalkyl group, one or more ethylene groups constituting the cycloalkane ring may be replaced by vinylene groups.

The group represented by the formula (Y1) or the formula (Y2) preferably has a form in which, for example, Rx₁ is a methyl group or an ethyl group, and Rx₂ and Rx₃ are bonded together to form the above-described cycloalkyl group.

When the actinic ray-sensitive or radiation-sensitive resin composition is, for example, a resist composition used for EUV exposure, the alkyl groups, the cycloalkyl groups, the alkenyl groups, and the aryl groups represented by Rx₁ to Rx₃ and the ring formed by bonding together two of Rx₁ to Rx₃ also preferably further have, as a substituent, a fluorine atom or an iodine atom.

In the formula (Y3), R₃₆ to R₃₈ each independently represent a hydrogen atom or a monovalent organic group. R₃₇ and R₃₈ may be bonded together to form a ring. The monovalent organic group may be an alkyl group, a cycloalkyl group, an aryl group, an aralkyl group, or an alkenyl group. R₃₆ is also preferably a hydrogen atom.

Note that the alkyl group, the cycloalkyl group, the aryl group, and the aralkyl group may include a heteroatom such as an oxygen atom and/or a group including a heteroatom such as a carbonyl group. For example, in the alkyl group, the cycloalkyl group, the aryl group, and the aralkyl group, one or more methylene groups may be replaced by a heteroatom such as an oxygen atom and/or a group including a heteroatom such as a carbonyl group.

R₃₈ and another substituent of the main chain of the repeating unit may be bonded together to form a ring. The group formed by bonding together R₃₈ and another substituent of the main chain of the repeating unit is preferably an alkylene group such as a methylene group.

When the actinic ray-sensitive or radiation-sensitive resin composition is, for example, a resist composition used for EUV exposure, the monovalent organic groups represented by R₃₆ to R₃₈ and the ring formed by bonding together R₃₇ and R₃₈ also preferably further have, as a substituent, a fluorine atom or an iodine atom.

The formula (Y3) is preferably a group represented by the following formula (Y3-1).

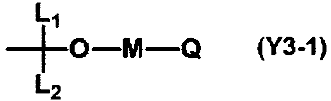

L₁ and L₂ above each independently represent a hydrogen atom, an alkyl group, a cycloalkyl group, an aryl group, or a group that is a combination of the foregoing (for example, a group that is a combination of an alkyl group and an aryl group).

M represents a single bond or a divalent linking group.

Q represents an alkyl group that may include a heteroatom, a cycloalkyl group that may include a heteroatom, an aryl group that may include a heteroatom, an amino group, an ammonium group, a mercapto group, a cyano group, an aldehyde group, or a group that is a combination of the foregoing (for example, a group that is a combination of an alkyl group and a cycloalkyl group).

In the alkyl group and the cycloalkyl group, for example, one of methylene groups may be replaced by a heteroatom such as an oxygen atom or a group including a heteroatom such as a carbonyl group.

Note that one of L₁ and L₂ is preferably a hydrogen atom and the other is preferably an alkyl group, a cycloalkyl group, an aryl group, or a group that is a combination of an alkylene group and an aryl group.

At least two of Q, M, and L₁ may be bonded together to form a ring (preferably a 5-membered or 6-membered ring).

From the viewpoint of forming finer patterns, L₂ is preferably a secondary or tertiary alkyl group, and more preferably a tertiary alkyl group. Examples of the secondary alkyl group include an isopropyl group, a cyclohexyl group, and a norbornyl group; examples of the tertiary alkyl group include a tert-butyl group and an adamantane group. In such examples, Tg (glass transition temperature) and activation energy are increased, so that film hardness is ensured and fogging can also be suppressed.

When the actinic ray-sensitive or radiation-sensitive resin composition is, for example, a resist composition used for EUV exposure, the alkyl groups, cycloalkyl groups, aryl groups, and groups that are combinations of the foregoing represented by L₁ and L₂ also preferably further have, as a substituent, a fluorine atom or an iodine atom. The alkyl groups, the cycloalkyl groups, the aryl groups, and the aralkyl groups also preferably include, in addition to a fluorine atom and an iodine atom, a heteroatom such as an oxygen atom. Specifically, in the alkyl groups, the cycloalkyl groups, the aryl groups, and the aralkyl groups, for example, one of methylene groups may be replaced by a heteroatom such as an oxygen atom or a group including a heteroatom such as a carbonyl group.

When the actinic ray-sensitive or radiation-sensitive resin composition is, for example, a resist composition used for EUV exposure, in the alkyl group that may include a heteroatom, cycloalkyl group that may include a heteroatom, aryl group that may include a heteroatom, amino group, ammonium group, mercapto group, cyano group, aldehyde group, and group that is a combination of the foregoing represented by Q, such a heteroatom is also preferably a heteroatom selected from the group consisting of a fluorine atom, an iodine atom, and an oxygen atom.

In the formula (Y4), Ar represents an aromatic ring group. Rn represents an alkyl group, a cycloalkyl group, or an aryl group. Rn and Ar may be bonded together to form a non-aromatic ring. Ar is preferably an aryl group.

When the actinic ray-sensitive or radiation-sensitive resin composition is, for example, a resist composition used for EUV exposure, the aromatic ring group represented by Ar and the alkyl group, cycloalkyl group, and aryl group represented by Rn also preferably have, as a substituent, a fluorine atom or an iodine atom.

From the viewpoint of providing a repeating unit having high acid-decomposability, in the leaving group protecting the polar group, when a non-aromatic ring is directly bonded to the polar group (or its residue), in the non-aromatic ring, a ring-member atom adjacent to a ring-member atom directly bonded to the polar group (or its residue) also preferably does not have, as a substituent, a halogen atom such as a fluorine atom.

Alternatively, the group that leaves by the action of an acid may be a 2-cyclopentenyl group having a substituent (such as an alkyl group) such as a 3-methyl-2-cyclopentenyl group, or a cyclohexyl group having a substituent (such as an alkyl group) such as a 1,1,4,4-tetramethylcyclohexyl group.

The repeating unit having an acid-decomposable group is also preferably a repeating unit represented by a formula (A).

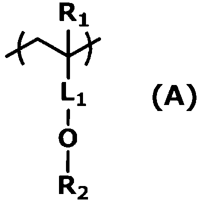

L₁ represents a divalent linking group that may have a fluorine atom or an iodine atom; R₁ represents a hydrogen atom, a fluorine atom, an iodine atom, or an alkyl group that may have a fluorine atom or an iodine atom, or an aryl group that may have a fluorine atom or an iodine atom; R₂ represents a leaving group that leaves by the action of an acid and that may have a fluorine atom or an iodine atom. Note that at least one of L₁, R₁, or R₂ has a fluorine atom or an iodine atom.

Examples of the divalent linking group that is represented by L₁ and may have a fluorine atom or an iodine atom include -CO-, -O-, -S-, -SO-, -SO₂-, hydrocarbon groups that may have a fluorine atom or an iodine atom (for example, alkylene groups, cycloalkylene groups, alkenylene groups, and arylene groups), and linking groups in which a plurality of the foregoing are linked together. In particular, L₁ is preferably -CO-, an arylene group, or an -arylene group-alkylene group having a fluorine atom or an iodine atom-, and more preferably -CO- or an - arylene group-alkylene group having a fluorine atom or an iodine atom-.

The arylene group is preferably a phenylene group.

The alkylene group may be linear or may be branched. The number of carbon atoms of the alkylene group is not particularly limited, but is preferably 1 to 10, and more preferably 1 to 3.

In the alkylene group having a fluorine atom or an iodine atom, the total number of fluorine atoms and iodine atoms is not particularly limited, but is preferably 2 or more, more preferably 2 to 10, and still more preferably 3 to 6.

The alkyl group represented by R₁ may be linear or may be branched. The number of carbon atoms of the alkyl group is not particularly limited, but is preferably 1 to 10, and more preferably 1 to 3.

In the alkyl group represented by R₁ and having a fluorine atom or an iodine atom, the total number of fluorine atoms and iodine atoms is not particularly limited, but is preferably 1 or more, more preferably 1 to 5, and still more preferably 1 to 3.

The alkyl group represented by R₁ may include a heteroatom other than halogen atoms, such as an oxygen atom.

Examples of the leaving group that is represented by R₂ and may have a fluorine atom or an iodine atom include leaving groups that are represented by the above-described formulas (Y1) to (Y4) and that have a fluorine atom or an iodine atom.

The repeating unit having an acid-decomposable group is also preferably a repeating unit represented by a formula (AI).

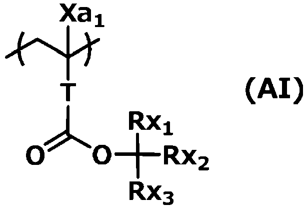

In the formula (AI), Xa₁ represents a hydrogen atom or an alkyl group that may have a substituent. T represents a single bond or a divalent linking group. Rx₁ to Rx₃ each independently represent an alkyl group (linear or branched), a cycloalkyl group (monocyclic or polycyclic), an alkenyl group (linear or branched), or an aryl group (monocyclic or polycyclic). Note that, when Rx₁ to Rx₃ are all alkyl groups (linear or branched), at least two of Rx₁ to Rx₃ are preferably methyl groups.

Two of Rx₁ to Rx₃ may be bonded together to form a monocycle or a polycycle (such as a monocyclic or polycyclic cycloalkyl group).

The alkyl group that is represented by Xa₁ and may have a substituent may be, for example, a methyl group or a group represented by -CH₂₋R₁₁. R₁₁ represents a halogen atom (such as a fluorine atom), a hydroxy group, or a monovalent organic group. The monovalent organic group represented by R₁₁ is, for example, an alkyl group that has 5 or less carbon atoms and that may be substituted with a halogen atom, an acyl group that has 5 or less carbon atoms and that may be substituted with a halogen atom, or an alkoxy group that has 5 or less carbon atoms and that may be substituted with a halogen atom, and is preferably an alkyl group having 3 or less carbon atoms, and more preferably a methyl group. Xa₁ is preferably a hydrogen atom, a methyl group, a trifluoromethyl group, or a hydroxymethyl group.

For T, the divalent linking group may be an alkylene group, an aromatic ring group, a -COO-Rt- group, or an -O-Rt- group. In the formulas, Rt represent an alkylene group or a cycloalkylene group.

T is preferably a single bond or a -COO-Rt- group. When T represents a -COO-Rt- group, Rt is preferably an alkylene group having 1 to 5 carbon atoms, and more preferably a -CH₂- group, a -(CH₂)₂- group, or a -(CH₂)₃- group.

For Rx₁ to Rx₃, the alkyl group is preferably an alkyl group having 1 to 4 carbon atoms such as a methyl group, an ethyl group, an n-propyl group, an isopropyl group, an n-butyl group, an isobutyl group, or a t-butyl group.

For Rx₁ to Rx₃, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For Rx₁ to Rx₃, the aryl group is preferably an aryl group having 6 to 10 carbon atoms and may be, for example, a phenyl group, a naphthyl group, or an anthryl group.

For Rx₁ to Rx₃, the alkenyl group is preferably a vinyl group.

The cycloalkyl group formed by bonding together two of Rx₁ to Rx₃ is preferably a monocyclic cycloalkyl group such as a cyclopentyl group or a cyclohexyl group. Also preferred are polycyclic cycloalkyl groups such as a norbornyl group, a tetracyclodecanyl group, a tetracyclododecanyl group, and an adamantyl group. In particular, preferred is a monocyclic cycloalkyl group having 5 to 6 carbon atoms.

In the cycloalkyl group formed by bonding together two of Rx₁ to Rx₃, for example, one of methylene groups constituting the ring may be replaced by a heteroatom such as an oxygen atom, a group including a heteroatom such as a carbonyl group, or a vinylidene group. In the cycloalkyl group, one or more of the ethylene groups constituting the cycloalkane ring may be replaced by vinylene groups.

The repeating unit represented by the formula (AI) preferably has a form in which, for example, Rx₁ is a methyl group or an ethyl group, and Rx₂ and Rx₃ are bonded together to form the above-described cycloalkyl group.

When the above-described groups each have a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms). The substituent preferably has 8 or less carbon atoms.

The repeating unit represented by the formula (AI) is preferably an acid-decomposable (meth)acrylic acid tertiary alkyl ester-based repeating unit (the repeating unit where Xa₁ represents a hydrogen atom or a methyl group and T represents a single bond).

The following are non-limiting specific examples of the repeating unit having an acid-decomposable group. Note that, in the formulas, Xa₁ represent H, CH₃, CF₃, or CH₂OH, and Rxa and Rxb each independently represent a linear or branched alkyl group having 1 to 5 carbon atoms.

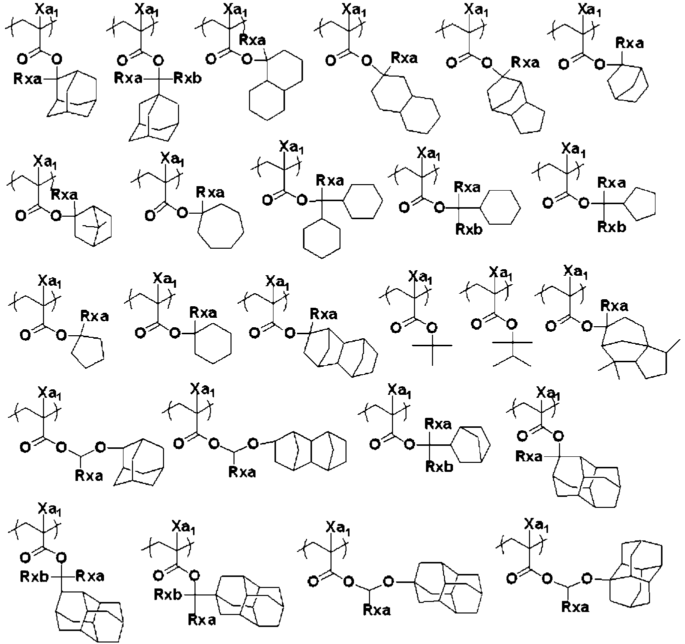

The resin (A) may have, as a repeating unit having an acid-decomposable group, a repeating unit having an acid-decomposable group including an unsaturated bond.

The repeating unit having an acid-decomposable group including an unsaturated bond is preferably a repeating unit represented by a formula (B).

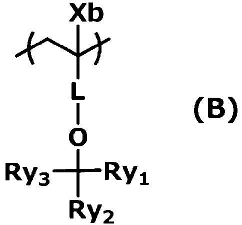

In the formula (B), Xb represents a hydrogen atom, a halogen atom, or an alkyl group that may have a substituent. L represents a single bond or a divalent linking group that may have a substituent. Ry₁ to Ry₃ each independently represent a linear or branched alkyl group, a monocyclic or polycyclic cycloalkyl group, an alkenyl group, an alkynyl group, or a monocyclic or polycyclic aryl group. Note that at least one of Ry₁ to Ry₃ represents an alkenyl group, an alkynyl group, a monocyclic or polycyclic cycloalkenyl group, or a monocyclic or polycyclic aryl group.

Two of Ry₁ to Ry₃ may be bonded together to form a monocycle or a polycycle (such as a monocyclic or polycyclic cycloalkyl group or cycloalkenyl group).

For Xb, the alkyl group that may have a substituent may be, for example, a methyl group or a group represented by -CH₂-R₁₁. R₁₁ represents a halogen atom (such as a fluorine atom), a hydroxy group, or a monovalent organic group such as an alkyl group that has 5 or less carbon atoms and that may be substituted with a halogen atom, an acyl group that has 5 or less carbon atoms and that may be substituted with a halogen atom, or an alkoxy group that has 5 or less carbon atoms and that may be substituted with a halogen atom, is preferably an alkyl group having 3 or less carbon atoms, and more preferably a methyl group. Xb is preferably a hydrogen atom, a fluorine atom, a methyl group, a trifluoromethyl group, or a hydroxymethyl group.

For L, the divalent linking group may be an -Rt- group, a -CO- group, a -COO-Rt-group, a -COO-Rt-CO- group, an -Rt-CO- group, or an -O-Rt- group. In the formulas, Rt represent an alkylene group, a cycloalkylene group, or an aromatic ring group, and is preferably an aromatic ring group.

L is preferably an -Rt- group, a -CO- group, a -COO-Rt-CO- group, or an -Rt-CO- group. Rt may have a substituent such as a halogen atom, a hydroxy group, or an alkoxy group.

For Ry₁ to Ry₃, the alkyl group is preferably an alkyl group having 1 to 4 carbon atoms such as a methyl group, an ethyl group, an n-propyl group, an isopropyl group, an n-butyl group, an isobutyl group, or a t-butyl group.

For Ry₁ to Ry₃, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For Ry₁ to Ry₃, the aryl group is preferably an aryl group having 6 to 10 carbon atoms, and may be, for example, a phenyl group, a naphthyl group, or an anthryl group.

For Ry₁ to Ry₃, the alkenyl group is preferably a vinyl group.

For Ry₁ to Ry₃, the alkynyl group is preferably an ethynyl group.

For Ry₁ to Ry₃, the cycloalkenyl group is preferably a structure in which a monocyclic cycloalkyl group such as a cyclopentyl group or a cyclohexyl group includes partially a double bond.

The cycloalkyl group formed by bonding together two of Ry₁ to Ry₃ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group. In particular, more preferred is a monocyclic cycloalkyl group having 5 to 6 carbon atoms.

In the cycloalkyl group or the cycloalkenyl group formed by bonding together two of Ry₁ to Ry₃, for example, one of methylene groups constituting the ring may be replaced by a heteroatom such as an oxygen atom, a group including a heteroatom such as a carbonyl group, a -SO₂- group, or a -SO₃- group, a vinylidene group, or a combination of the foregoing. In the cycloalkyl group or the cycloalkenyl group, one or more ethylene groups constituting the cycloalkane ring or the cycloalkene ring may be replaced by vinylene groups.

The repeating unit represented by the formula (B) preferably has a form in which, for example, Ry₁ is a methyl group, an ethyl group, a vinyl group, an allyl group, or an aryl group, and Ry₂ and Ry₃ are bonded together to form the above-described cycloalkyl group or cycloalkenyl group.

When the above-described groups each have a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms). The substituent preferably has 8 or less carbon atoms.

The repeating unit represented by the formula (B) is preferably an acid-decomposable (meth)acrylic acid tertiary ester-based repeating unit (the repeating unit where Xb represents a hydrogen atom or a methyl group, and L represents a -CO- group), an acid-decomposable hydroxystyrene tertiary alkyl ether-based repeating unit (the repeating unit where Xb represents a hydrogen atom or a methyl group, and L represents a phenyl group), or an acid-decomposable styrenecarboxylic acid tertiary ester-based repeating unit (the repeating unit where Xb represents a hydrogen atom or a methyl group, and L represents an -Rt-CO- group (where Rt is an aromatic group)).

The content of the repeating unit having an acid-decomposable group including an unsaturated bond relative to all the repeating units in the resin (A) is preferably 15 mol% or more, more preferably 20 mol% or more, and still more preferably 30 mol% or more. The upper limit value relative to all the repeating units in the resin (A) is preferably 80 mol% or less, more preferably 70 mol% or less, and still more preferably 60 mol% or less.

Specific examples of the repeating unit having an acid-decomposable group including an unsaturated bond include, for example, the repeating units described in [0067] to [0071] of WO2022/024928A. The above description is incorporated herein.

The content of the repeating unit having an acid-decomposable group relative to all the repeating units in the resin (A) is preferably 15 mol% or more, more preferably 20 mol% or more, and still more preferably 30 mol% or more. The upper limit value relative to all the repeating units in the resin (A) is preferably 90 mol% or less, more preferably 80 mol% or less, still more preferably 70 mol% or less, and particularly preferably 60 mol% or less.

The resin (A) may include at least one repeating unit species selected from the group consisting of the following Group A and/or at least one repeating unit species selected from the group consisting of the following Group B.
Group A: the group consisting of the following repeating units (20) to (25)
   (20) a repeating unit (described later) having an acid group;
   (21) a repeating unit (described later) not having an acid-decomposable group or an acid group, but having a fluorine atom, a bromine atom, or an iodine atom;
   (22) a repeating unit (described later) having a lactone group, a sultone group, or a carbonate group;
   (23) a repeating unit (described later) having a photoacid generation group;
   (24) a repeating unit (described later) represented by a formula (V-1) or a formula (V-2) below; and
   (25) a repeating unit for lowering the mobility of the main chain.
   Note that the repeating units described later and represented by a formula (A) to a formula (E) correspond to the repeating unit (25) for lowering the mobility of the main chain.
Group B: the group consisting of the following repeating units (30) to (32)
   (30) a repeating unit (described later) having at least one group species selected from the group consisting of a lactone group, a sultone group, a carbonate group, a hydroxy group, a cyano group, and an alkali-soluble group;
   (31) a repeating unit (described later) having an alicyclic hydrocarbon structure and not exhibiting acid-decomposability; and
   (32) a repeating unit (described later) not having a hydroxy group or a cyano group and represented by a formula (III).

The resin (A) preferably has an acid group and preferably includes a repeating unit having an acid group as described later. Note that the definition of the acid group will be described in a later part together with preferred examples of the repeating unit having an acid group. When the resin (A) has an acid group, a better interaction between the resin (A) and the acid generated from the photoacid generator is provided. This results in further suppression of diffusion of the acid, so that a pattern having a more square profile can be formed.

The resin (A) may have at least one repeating unit species selected from the group consisting of Group A above. When the actinic ray-sensitive or radiation-sensitive resin composition is used as an actinic ray-sensitive or radiation-sensitive resin composition for EUV exposure, the resin (A) preferably has at least one repeating unit species selected from the group consisting of Group A above.

The resin (A) may include at least one of a fluorine atom or an iodine atom. When the actinic ray-sensitive or radiation-sensitive resin composition is used as an actinic ray-sensitive or radiation-sensitive resin composition for EUV exposure, the resin (A) preferably includes at least one of a fluorine atom or an iodine atom. When the resin (A) includes both of a fluorine atom and an iodine atom, the resin (A) may have a repeating unit including both of a fluorine atom and an iodine atom, or the resin (A) may include two species that are a repeating unit having a fluorine atom and a repeating unit including an iodine atom.

The resin (A) may have a repeating unit having an aromatic group. When the actinic ray-sensitive or radiation-sensitive resin composition is used as an actinic ray-sensitive or radiation-sensitive resin composition for EUV exposure, the resin (A) also preferably has a repeating unit having an aromatic group.

The resin (A) may have at least one repeating unit species selected from the group consisting of Group B above. When the actinic ray-sensitive or radiation-sensitive resin composition is used as an actinic ray-sensitive or radiation-sensitive resin composition for ArF, the resin (A) preferably has at least one repeating unit species selected from the group consisting of Group B above.

Note that, when the actinic ray-sensitive or radiation-sensitive resin composition is used as an actinic ray-sensitive or radiation-sensitive resin composition for ArF, the resin (A) preferably does not include a fluorine atom or a silicon atom.

When the actinic ray-sensitive or radiation-sensitive resin composition is used as an actinic ray-sensitive or radiation-sensitive resin composition for ArF, the resin (A) preferably does not have an aromatic group.

### Repeating unit having acid group

The resin (A) may have a repeating unit having an acid group.

The acid group is preferably an acid group having a pKa of 13 or less. The acid group preferably has an acid dissociation constant of 13 or less, more preferably 3 to 13, and still more preferably 5 to 10.

When the resin (A) has an acid group having a pKa of 13 or less, the content of the acid group in the resin (A) is not particularly limited, but is often 0.2 to 6.0 mmol/g. In particular, preferred is 0.8 to 6.0 mmol/g, more preferred is 1.2 to 5.0 mmol/g, and still more preferred is 1.6 to 4.0 mmol/g. When the content of the acid group is within such a range, development suitably proceeds to form a pattern having a good profile at high resolution.

The acid group is preferably, for example, a carboxyl group, a phenolic hydroxyl group, a fluoroalcohol group (preferably a hexafluoroisopropanol group), a sulfonic acid group, a sulfonamide group, or an isopropanol group.

In the hexafluoroisopropanol group, one or more (preferably one to two) of the fluorine atoms may be substituted with groups other than fluorine atoms (such as alkoxycarbonyl groups). The acid group is also preferably -C(CF₃)(OH)-CF₂- formed in this manner. Alternatively, one or more of the fluorine atoms may be substituted with groups other than fluorine atoms, to form a ring including -C(CF₃)(OH)-CF₂-.

The repeating unit having an acid group is preferably a repeating unit different from the above-described repeating unit having a structure in which a polar group is protected with a group that leaves by the action of an acid and repeating units described later and having a lactone group, a sultone group, or a carbonate group.

The repeating unit having an acid group may have a fluorine atom or an iodine atom.

Specific examples of the repeating unit having an acid group include, for example, the repeating units described in [0088] to [0089] and [0103] to [0110] of WO2022/024928A. The above description is incorporated herein.

The repeating unit having an acid group is preferably a repeating unit represented by a formula (b1-1) below.

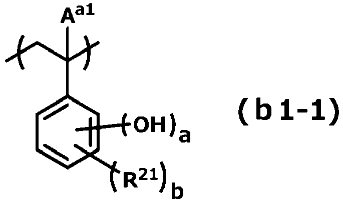

In the formula (b1-1), A^{a1} represents a hydrogen atom, an alkyl group, a cycloalkyl group, a halogen atom, or a cyano group. R²¹ represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group; when there are a plurality of R²¹'s, they may be the same or different. When the formula has a plurality of R²¹'s, they may together form a ring. R²¹ is preferably a hydrogen atom. a represents an integer of 1 to 3. b represents an integer of 0 to (5 - a).

When the resin (A) includes a repeating unit having an acid group, the content of the repeating unit having an acid group is, relative to all the repeating units in the resin (A), preferably 10 mol% or more, and more preferably 15 mol% or more. The upper limit value relative to all the repeating units in the resin (A) is preferably 70 mol% or less, more preferably 65 mol% or less, and still more preferably 60 mol% or less.

### Repeating unit not having acid-decomposable group or acid group, but having fluorine atom, bromine atom, or iodine atom

The resin (A) may have, aside from the above-described <repeating unit having an acid-decomposable group> and <repeating unit having an acid group>, a repeating unit not having an acid-decomposable group or an acid group, but having a fluorine atom, a bromine atom, or an iodine atom (hereinafter, also referred to as unit X). This <repeating unit not having an acid-decomposable group or an acid group, but having a fluorine atom, a bromine atom, or an iodine atom> is preferably different from other repeating unit species belonging to Group A such as the <repeating unit having a lactone group, a sultone group, or a carbonate group> and the <repeating unit having a photoacid generation group> described later.

The unit X is preferably a repeating unit represented by a formula (C).

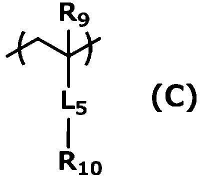

L₅ represents a single bond or an ester group. R₉ represents a hydrogen atom or an alkyl group that may have a fluorine atom or an iodine atom. R₁₀ represents a hydrogen atom, an alkyl group that may have a fluorine atom or an iodine atom, a cycloalkyl group that may have a fluorine atom or an iodine atom, an aryl group that may have a fluorine atom or an iodine atom, or a group that is a combination of the foregoing.

Specific examples of the repeating unit having a fluorine atom or an iodine atom include, for example, the repeating units described in [0116] to [0117] of WO2022/024928A. The above description is incorporated herein.

The unit X content relative to all the repeating units in the resin (A) is preferably 0 mol% or more, more preferably 5 mol% or more, and still more preferably 10 mol% or more. The upper limit value relative to all the repeating units in the resin (A) is preferably 50 mol% or less, more preferably 45 mol% or less, and still more preferably 40 mol% or less.

Of the repeating units of the resin (A), the total content of the repeating unit including at least one of a fluorine atom, a bromine atom, or an iodine atom relative to all the repeating units of the resin (A) is preferably 10 mol% or more, more preferably 20 mol% or more, still more preferably 30 mol% or more, and particularly preferably 40 mol% or more. The upper limit value is not particularly limited, but is, for example, relative to all the repeating units of the resin (A), 100 mol% or less.

Note that examples of the repeating unit including at least one of a fluorine atom, a bromine atom, or an iodine atom include a repeating unit having a fluorine atom, a bromine atom, or an iodine atom and having an acid-decomposable group, a repeating unit having a fluorine atom, a bromine atom, or an iodine atom and having an acid group, and a repeating unit having a fluorine atom, a bromine atom, or an iodine atom.

### Repeating unit having lactone group, sultone group, or carbonate group

The resin (A) may have a repeating unit having at least one selected from the group consisting of a lactone group, a sultone group, and a carbonate group (hereinafter, also referred to as "unit Y").

The unit Y also preferably does not have acid groups such as a hydroxy group and a hexafluoropropanol group.

The lactone group or the sultone group has a lactone structure or a sultone structure. The lactone structure or the sultone structure is preferably a 5- to 7-membered lactone structure or a 5- to 7-membered sultone structure. In particular, more preferred is a 5- to 7-membered lactone structure to which another ring structure is fused so as to form a bicyclo structure or a spiro structure, or a 5- to 7-membered sultone structure to which another ring structure is fused so as to form a bicyclo structure or a spiro structure.

The resin (A) preferably has a repeating unit having a lactone group or a sultone group provided by withdrawing, from ring-member atoms of the lactone structure represented by any one of formulas (LC1-1) to (LC1-21) below or the sultone structure represented by any one of formulas (SL1-1) to (SL1-3) below, one or more hydrogen atoms, and a lactone group or a sultone group may be directly bonded to the main chain. For example, ring-member atoms of a lactone group or a sultone group may constitute the main chain of the resin (A).

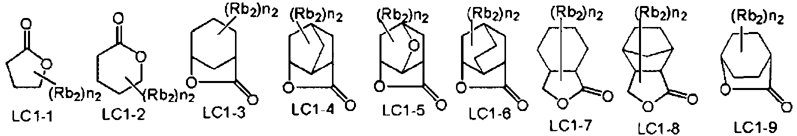

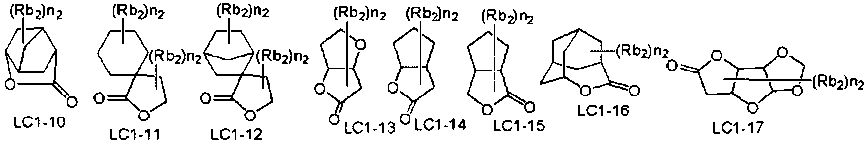

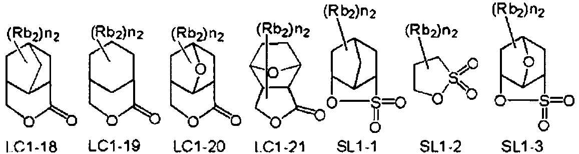

The lactone structure or the sultone structure may have a substituent (Rb₂). Preferred examples of the substituent (Rb₂) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxyl group, a halogen atom, a cyano group, and an acid-decomposable group. n₂ represent an integer of 0 to 4. When n₂ is 2 or more, the plurality of Rb₂'s present may be different, and the plurality of Rb₂'s present may be bonded together to form a ring.

The repeating unit having a group including the lactone structure represented by any one of the formulas (LC1-1) to (LC1-21) or the sultone structure represented by any one of the formulas (SL1-1) to (SL1-3) may be, for example, a repeating unit represented by the following formula (AI-2).

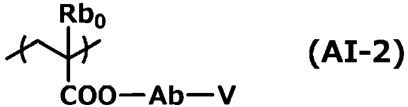

In the formula (AI-2), Rb₀ represents a hydrogen atom, a halogen atom, or an alkyl group having 1 to 4 carbon atoms. Preferred examples of the substituent that the alkyl group of Rb₀ may have include a hydroxy group and a halogen atom.

For Rb₀, the halogen atom may be a fluorine atom, a chlorine atom, a bromine atom, or an iodine atom. Rb₀ is preferably a hydrogen atom or a methyl group.

Ab represents a single bond, an alkylene group, a divalent linking group having a monocyclic or polycyclic alicyclic hydrocarbon structure, an ether group, an ester group, a carbonyl group, a carboxyl group, or a divalent linking group that is a combination of the foregoing. In particular, Ab is preferably a single bond or a linking group represented by -Ab₁-CO₂-. Ab₁ is a linear or branched alkylene group or a monocyclic or polycyclic cycloalkylene group, and preferably a methylene group, an ethylene group, a cyclohexylene group, an adamantylene group, or a norbornylene group.

V represents a group formed by withdrawing, from a ring-member atom of the lactone structure represented by any one of the formulas (LC1-1) to (LC1-21), a single hydrogen atom, or a group formed by withdrawing, from a ring-member atom of the sultone structure represented by any one of the formulas (SL1-1) to (SL1-3), a single hydrogen atom.

When the repeating unit having a lactone group or a sultone group has an optical isomer, any optical isomer may be used. A single optical isomer may be used alone, or a plurality of optical isomers may be used in combination. In the case of mainly using one of the optical isomers, its optical purity (ee) is preferably 90 or more, and more preferably 95 or more.

The carbonate group is preferably a cyclic carbonic acid ester group.

For the repeating unit having a cyclic carbonic acid ester group, for example, the description in [0127] to [0133] of WO2022/024928A can be referred to. The above description is incorporated herein.

When the resin (A) includes the unit Y, the content of the unit Y relative to all the repeating units in the resin (A) is preferably 1 mol% or more, and more preferably 10 mol% or more. The upper limit value relative to all the repeating units in the resin (A) is preferably 85 mol% or less, more preferably 80 mol% or less, still more preferably 70 mol% or less, and particularly preferably 60 mol% or less.

### Repeating unit having photoacid generation group

The resin (A) may have, as another repeating unit, a repeating unit having a group that generates an acid upon irradiation with an actinic ray or a radiation (also referred to as "photoacid generation group").

The repeating unit having a photoacid generation group may be a repeating unit represented by a formula (4).

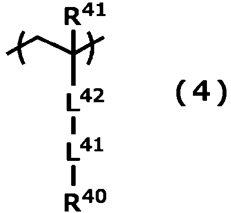

R⁴¹ represents a hydrogen atom or a methyl group. L⁴¹ represents a single bond or a divalent linking group. L⁴² represents a divalent linking group. R⁴⁰ represents a structural moiety that is decomposed upon irradiation with an actinic ray or a radiation to generate an acid in the side chain.

Specific examples of the repeating unit having a photoacid generation group include, for example, the repeating units described in [0094] to [0105] of JP2014-041327A, the repeating units described in [0094] of WO2018/193954A, and the repeating units described in [0138] of WO2022/024928A. The above description is incorporated herein.

Examples of the repeating unit represented by the formula (4) include the repeating units described in Paragraphs [0094] to [0105] of JP2014-041327A, and the repeating units described in Paragraph [0094] of WO2018/193954A.

The content of the repeating unit having a photoacid generation group relative to all the repeating units in the resin (A) is preferably 1 mol% or more, and more preferably 5 mol% or more. The upper limit value relative to all the repeating units in the resin (A) is preferably 40 mol% or less, more preferably 35 mol% or less, and still more preferably 30 mol% or less.

The above-described onium salt (N1) can also be used as the photoacid generation moiety of the repeating unit having a photoacid generation group.

### Repeating unit represented by formula (V-1) or formula (V-2) below

The resin (A) may have a repeating unit represented by a formula (V-1) below or a formula (V-2) below.

The repeating unit represented by the formula (V-1) below or the formula (V-2) below is preferably a repeating unit different from the above-described repeating units.

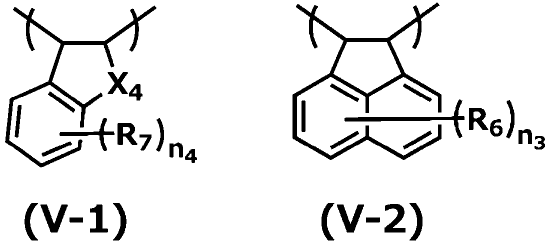

In the formulas,
R₆ and R₇ each independently represent a hydrogen atom, a hydroxyl group, an alkyl group, an alkoxy group, an acyloxy group, a cyano group, a nitro group, an amino group, a halogen atom, an ester group (-OCOR or -COOR: R is an alkyl group or a fluorinated alkyl group having 1 to 6 carbon atoms), or a carboxyl group. The alkyl group is preferably a linear, branched, or cyclic alkyl group having 1 to 10 carbon atoms.
n₃ represents an integer of 0 to 6.
n₄ represents an integer of 0 to 4.
X₄ is a methylene group, an oxygen atom, or a sulfur atom.

Examples of the repeating unit represented by the formula (V-1) or (V-2) are as follows.

Examples of the repeating unit represented by the formula (V-1) or (V-2) include the repeating units described in Paragraph [0100] of WO2018/193954A.

### Repeating unit for lowering mobility of main chain

The resin (A) preferably has, from the viewpoint of suppressing excessive diffusion of the generated acid or pattern collapse during development, a relatively high glass transition temperature (Tg). Tg is preferably more than 90°C, more preferably more than 100°C, still more preferably more than 110°C, and particularly preferably more than 125°C. Note that, from the viewpoint of providing a high dissolution rate in developers, Tg is preferably 400°C or less, and more preferably 350°C or less.

Note that, in this Specification, the glass transition temperatures (Tg) of polymers such as the resin (A) (hereafter, "Tg's of repeating units") are calculated in the following manner. First, for the repeating units included in a polymer, the Tg's of homopolymers composed only of the repeating units are individually calculated by the Bicerano method. Subsequently, the mass ratios (%) of the repeating units relative to all the repeating units in the polymer are calculated. Subsequently, the Fox equation (described in Materials Letters 62 (2008) 3152, for example) is used to calculate Tg's for the mass ratios and the Tg's are summed up to determine the Tg(°C) of the polymer.

The Bicerano method is described in Prediction of polymer properties, Marcel Dekker Inc, New York (1993). The calculation of Tg by the Bicerano method can be performed using a software for estimating properties of polymers, MDL Polymer (MDL Information Systems, Inc.).

In order to increase the Tg of the resin (A) (preferably, making Tg be more than 90°C), the mobility of the main chain of the resin (A) is preferably lowered. Examples of the method for lowering the mobility of the main chain of the resin (A) include the following methods (a) to (e):
(a) introduction of a bulky substituent to the main chain;
(b) introduction of a plurality of substituents to the main chain;
(c) introduction of a substituent that induces interaction between the resins (A), to the vicinity of the main chain;
(d) formation of the main chain with a ring structure; and
(e) linkage of a ring structure to the main chain.

Note that the resin (A) preferably has a repeating unit whose homopolymer has a Tg of 130°C or more.

Note that the repeating unit species whose homopolymer has a Tg of 130°C or more is not particularly limited and is a repeating unit whose homopolymer has a Tg of 130°C or more calculated by the Bicerano method. Note that the repeating units represented by a formula (A) to a formula (E) described later may, depending on the functional group species, belong to the repeating unit whose homopolymer has a Tg of 130°C or more.

An example of specific means for achieving (a) above is a method of introducing, into the resin (A), a repeating unit represented by a formula (A).

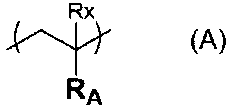

For the formula (A), R_{A} represents a group including a polycyclic structure. Rₓ represents a hydrogen atom, a methyl group, or an ethyl group. The group including a polycyclic structure is a group including a plurality of cyclic structures; the plurality of cyclic structures may be fused together or may not be fused together.

Specific examples of the repeating unit represented by the formula (A) include those described in Paragraphs [0107] to [0119] of WO2018/193954A.

An example of specific means for achieving (b) above is a method of introducing, into the resin (A), a repeating unit represented by a formula (B).

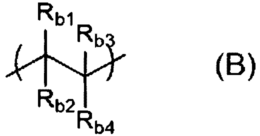

In the formula (B), R_{b1} to R_{b4} each independently represent a hydrogen atom or an organic group; at least two or more of R_{b1} to R_{b4} represent organic groups.

When at least one of the organic groups is a group whose cyclic structure is directly linked to the main chain in the repeating unit, the other organic group species is not particularly limited.

When none of the organic groups is a group whose cyclic structure is directly linked to the main chain in the repeating unit, at least two or more of the organic groups are substituents having three or more constituent atoms (except for hydrogen atoms).

Specific examples of the repeating unit represented by the formula (B) include those described in Paragraphs [0113] to [0115] of WO2018/193954A.

An example of specific means for achieving (c) above is a method of introducing, into the resin (A), a repeating unit represented by a formula (C).

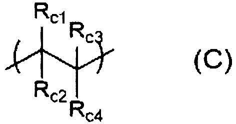

In the formula (C), R_{c1} to R_{c4} each independently represent a hydrogen atom or an organic group; at least one of R_{c1} to R_{c4} is a group including a hydrogen-bond-forming hydrogen atom positioned within three atoms from the carbon atom in the main chain. In particular, from the viewpoint of inducing the interaction between the main chains of the resin (A), it preferably has a hydrogen-bond-forming hydrogen atom positioned within two atoms (closer to the main chain side).

Specific examples of the repeating unit represented by the formula (C) include those described in Paragraphs [0119] to [0121] of WO2018/193954A.

An example of specific means for achieving (d) above is a method of introducing, into the resin (A), a repeating unit represented by a formula (D).

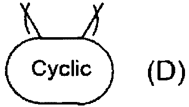

In the formula (D), "Cyclic" represents a group in which the ring structure forms the main chain. The number of atoms constituting the ring is not particularly limited.

Specific examples of the repeating unit represented by the formula (D) include those described in Paragraphs [0126] to [0127] of WO2018/193954A.

An example of specific means for achieving (e) above is a method of introducing, into the resin (A), a repeating unit represented by a formula (E).

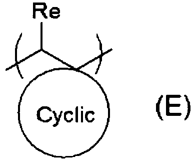

In the formula (E), Re each independently represent a hydrogen atom or an organic group. Examples of the organic group include alkyl groups, cycloalkyl groups, aryl groups, aralkyl groups, and alkenyl groups that may have substituents.

"Cyclic" is a cyclic group including a carbon atom of the main chain. The number of atoms included in the cyclic group is not particularly limited.

Specific examples of the repeating unit represented by the formula (E) include those described in Paragraphs [0131] to [0133] of WO2018/193954A.

### Repeating unit having at least one group species selected from the group consisting of lactone group, sultone group, carbonate group, hydroxy group, cyano group, and alkali-soluble group

The resin (A) may have a repeating unit having at least one group species selected from the group consisting of a lactone group, a sultone group, a carbonate group, a hydroxy group, a cyano group, and an alkali-soluble group.

In the resin (A), the repeating unit having a lactone group, a sultone group, or a carbonate group may be the repeating unit having been described above in <Repeating unit having lactone group, sultone group, or carbonate group>. Preferred contents are also the same as those having been described in <Repeating unit having lactone group, sultone group, or carbonate group>.

The resin (A) may have a repeating unit having a hydroxy group or a cyano group. This results in improvement in adhesiveness to the substrate and affinity for the developer.

The repeating unit having a hydroxy group or a cyano group is preferably a repeating unit having an alicyclic hydrocarbon structure substituted with a hydroxy group or a cyano group.

The repeating unit having a hydroxy group or a cyano group preferably does not have an acid-decomposable group. Examples of the repeating unit having a hydroxy group or a cyano group include those described in Paragraphs [0081] to [0084] of JP2014-098921A.

The resin (A) may have a repeating unit having an alkali-soluble group.

The alkali-soluble group may be a carboxyl group, a sulfonamide group, a sulfonylimide group, a bissulfonylimide group, or an aliphatic alcohol group substituted, at the α position, with an electron-withdrawing group (for example, a hexafluoroisopropanol group), and is preferably a carboxyl group. When the resin (A) includes the repeating unit having an alkali-soluble group, increased resolution is provided in the contact hole application. Examples of the repeating unit having an alkali-soluble group include those described in Paragraphs [0085] and [0086] of JP2014-098921A.

### Repeating unit having alicyclic hydrocarbon structure and not exhibiting acid-decomposability

The resin (A) may have a repeating unit having an alicyclic hydrocarbon structure and not exhibiting acid-decomposability. This results in, during liquid immersion exposure, a reduction in leaching of, from the resist film to the immersion liquid, low-molecular-weight components. Examples of the repeating unit having an alicyclic hydrocarbon structure and not exhibiting acid-decomposability include a repeating unit derived from 1-adamantyl (meth)acrylate, diamantyl (meth)acrylate, tricyclodecanyl (meth)acrylate, or cyclohexyl (meth)acrylate.

### Repeating unit not having hydroxy group or cyano group and represented by formula (III)

The resin (A) may have a repeating unit not having a hydroxy group or a cyano group and represented by a formula (III).

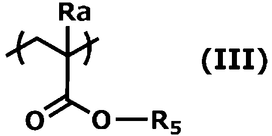

In the formula (III), R₅ represents a hydrocarbon group having at least one ring structure and not having a hydroxy group or a cyano group.

Ra represents a hydrogen atom, an alkyl group, or a -CH₂-O-Ra₂ group. In the formula, Ra₂ represents a hydrogen atom, an alkyl group, or an acyl group.

Examples of the repeating unit not having a hydroxy group or a cyano group and represented by the formula (III) include those described in Paragraphs [0087] to [0094] of JP2014-098921A.

### Other repeating unit

Furthermore, the resin (A) may have another repeating unit other than the above-described repeating units.

For example, the resin (A) may have a repeating unit selected from the group consisting of a repeating unit having an oxathiane ring group, a repeating unit having an oxazolone ring group, a repeating unit having a dioxane ring group, and a repeating unit having a hydantoin ring group.

The resin (A) may have, in addition to such repeating structural units, for the purpose of adjusting, for example, dry etching resistance, standard developer suitability, substrate adhesiveness, resist profile, resolution, heat resistance, and sensitivity, various repeating structure units.

For the resin (A), particularly when a composition of the present invention is used as an actinic ray-sensitive or radiation-sensitive resin composition for ArF, all the repeating units are preferably constituted by a repeating unit derived from a compound having an ethylenically unsaturated bond. In particular, all the repeating units are also preferably constituted by a (meth)acrylate-based repeating unit. When all the repeating units are constituted by a (meth)acrylate-based repeating unit, all the repeating units can be a methacrylate-based repeating unit, all the repeating units can be an acrylate-based repeating unit, or all the repeating units can be a methacrylate-based repeating unit and an acrylate-based repeating unit; the acrylate-based repeating unit content relative to all the repeating units is preferably 50 mol% or less.

The resin (A) can be synthesized by standard procedures (for example, radical polymerization).

The resin (A) has a weight-average molecular weight (Mw) of, as a polystyrene-equivalent value determined by the GPC method, preferably 30000 or less, more preferably 1000 to 30000, still more preferably 3000 to 30000, and particularly preferably 5000 to 15000.

The resin (A) has a dispersity (molecular weight distribution, Mw/Mn) of preferably 1 to 5, more preferably 1 to 3, still more preferably 1.2 to 3.0, and particularly preferably 1.2 to 2.0. As the dispersity lowers, the resolution becomes higher, the resist profile becomes better, the sidewalls of the resist pattern become smoother, and the roughness performance becomes higher.

In the actinic ray-sensitive or radiation-sensitive resin composition, the content of the resin (A) is, relative to the total solid content of the actinic ray-sensitive or radiation-sensitive resin composition, preferably 30.0 to 99.9 mass%, more preferably 40.0 to 99.9 mass%, and still more preferably 60.0 to 90.0 mass%.

Such resins (A) may be used alone or in combination of two or more thereof. When two or more thereof are used, the total content thereof is preferably within such a preferred content range.

### Compound (B) that generates acid upon irradiation with actinic ray or radiation

The composition of the present invention may further include a compound (B) that is a compound that generates an acid upon irradiation with an actinic ray or a radiation and is different from the onium salt (N1).

The compound (B) may have the form of a low-molecular-weight compound or may have the form of being incorporated into a portion of a polymer. Alternatively, the form of a low-molecular-weight compound and the form of being incorporated into a portion of a polymer may be used in combination.

When the compound (B) has the form of a low-molecular-weight compound, the compound (B) has a molecular weight of preferably 5000 or less, more preferably 4000 or less, and still more preferably 3000 or less. The lower limit is not particularly limited, but is preferably 100 or more.

When the compound (B) has the form of being incorporated into a portion of a polymer, it may be incorporated into a portion of the resin (A) or may be incorporated into a resin different from the resin (A).

The compound (B) preferably has the form of a low-molecular-weight compound.

The compound (B) may be, for example, a compound represented by "M⁺ X⁻" (onium salt) and is preferably a compound that generates an organic acid upon exposure.

Examples of the organic acid include sulfonic acids (such as aliphatic sulfonic acids, aromatic sulfonic acids, and camphorsulfonic acid), carboxylic acids (such as aliphatic carboxylic acids, aromatic carboxylic acids, and aralkyl carboxylic acids), carbonylsulfonylimidic acid, bis(alkylsulfonyl)imidic acids, and tris(alkylsulfonyl)methide acids.

In the compound represented by "M⁺ X⁻", M⁺ represents a cation, and preferably represents an organic cation.

Specific examples and preferred ranges of M⁺ are the same as the above-described specific examples and preferred ranges of M⁺ in the formula (N1).

In the compound represented by "M⁺ X⁻", X⁻ represents an anion, and preferably represents an organic anion.

The organic anion is not particularly limited, but may be a mono-, di-, or higher valent organic anion.

The organic anion is preferably an anion that has a very low capability of causing a nucleophilic reaction, and more preferably a non-nucleophilic anion.

Examples of the non-nucleophilic anion include sulfonate anions (such as aliphatic sulfonate anions, aromatic sulfonate anions, and a camphorsulfonate anion), carboxylate anions (such as aliphatic carboxylate anions, aromatic carboxylate anions, and aralkyl carboxylate anions), a sulfonylimide anion, bis(alkylsulfonyl)imide anions, and tris(alkylsulfonyl)methide anions.

In such an aliphatic sulfonate anion or aliphatic carboxylate anion, the aliphatic moiety may be a linear or branched alkyl group or may be a cycloalkyl group, and is preferably a linear or branched alkyl group having 1 to 30 carbon atoms, or a cycloalkyl group having 3 to 30 carbon atoms.

The alkyl group may be, for example, a fluoroalkyl group (that may have a substituent other than a fluorine atom, or may be a perfluoroalkyl group).

In such an aromatic sulfonate anion or aromatic carboxylate anion, the aryl group is preferably an aryl group having 6 to 14 carbon atoms, and may be, for example, a phenyl group, a tolyl group, or a naphthyl group.

The above-described alkyl group, cycloalkyl group, and aryl group may have a substituent. The substituent is not particularly limited; examples include a nitro group, halogen atoms such as a fluorine atom and a chlorine atom, a carboxyl group, a hydroxyl group, an amino group, a cyano group, alkoxy groups (preferably having 1 to 15 carbon atoms), alkyl groups (preferably having 1 to 10 carbon atoms), cycloalkyl groups (preferably having 3 to 15 carbon atoms), aryl groups (preferably having 6 to 14 carbon atoms), alkoxycarbonyl groups (preferably having 2 to 7 carbon atoms), acyl groups (preferably having 2 to 12 carbon atoms), alkoxycarbonyloxy groups (preferably having 2 to 7 carbon atoms), alkylthio groups (preferably having 1 to 15 carbon atoms), alkylsulfonyl groups (preferably having 1 to 15 carbon atoms), alkyliminosulfonyl groups (preferably having 1 to 15 carbon atoms), and aryloxysulfonyl groups (preferably having 6 to 20 carbon atoms).

In such an aralkyl carboxylate anion, the aralkyl group is preferably an aralkyl group having 7 to 14 carbon atoms.

Examples of the aralkyl group having 7 to 14 carbon atoms include a benzyl group, a phenethyl group, a naphthylmethyl group, a naphthylethyl group, and a naphthylbutyl group.

The sulfonylimide anion may be, for example, a saccharin anion.

In such a bis(alkylsulfonyl)imide anion or a tris(alkylsulfonyl)methide anion, the alkyl groups are preferably an alkyl group having 1 to 5 carbon atoms. In the alkyl group, a substituent may be a halogen atom, an alkyl group substituted with a halogen atom, an alkoxy group, an alkylthio group, an alkyloxysulfonyl group, an aryloxysulfonyl group, or a cycloalkylaryloxysulfonyl group, and is preferably a fluorine atom or an alkyl group substituted with a fluorine atom.

In the bis(alkylsulfonyl)imide anion, the alkyl groups may be bonded together to form a ring structure. This results in an increase in the acid strength.

Other examples of the non-nucleophilic anion include phosphorus fluoride (for example, PF₆⁻), boron fluoride (for example, BF₄⁻), and antimony fluoride (for example, SbF₆⁻).

The non-nucleophilic anion is also preferably an anion represented by the following formula (AN1).

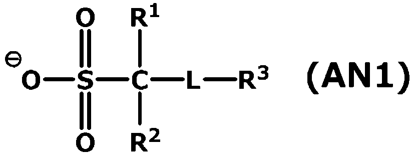

In the formula (AN1), R¹ and R² each independently represent a hydrogen atom or a substituent.

The substituent is not particularly limited, but is preferably a group that is not electron-withdrawing groups. Examples of the group that is not electron-withdrawing groups include hydrocarbon groups, a hydroxy group, oxyhydrocarbon groups, oxycarbonylhydrocarbon groups, an amino group, hydrocarbon-substituted amino groups, and hydrocarbon-substituted amide groups.

Such groups that are not electron-withdrawing groups are each independently preferably -R', -OH, -OR', -OCOR', -NH₂, -NR'₂, -NHR', or -NHCOR'. R' are monovalent hydrocarbon groups.

Examples of the monovalent hydrocarbon groups represented by R' above include monovalent linear or branched hydrocarbon groups such as alkyl groups such as a methyl group, an ethyl group, a propyl group, and a butyl group; alkenyl groups such as an ethenyl group, a propenyl group, and a butenyl group; and alkynyl groups such as an ethynyl group, a propynyl group, and a butynyl group; monovalent alicyclic hydrocarbon groups such as cycloalkyl groups such as a cyclopropyl group, a cyclobutyl group, a cyclopentyl group, a cyclohexyl group, a norbornyl group, and an adamantyl group; and cycloalkenyl groups such as a cyclopropenyl group, a cyclobutenyl group, a cyclopentenyl group, and a norbornenyl group; and monovalent aromatic hydrocarbon groups such as aryl groups such as a phenyl group, a tolyl group, a xylyl group, a mesityl group, a naphthyl group, a methylnaphthyl group, an anthryl group, and methylanthryl group; and aralkyl groups such as a benzyl group, a phenethyl group, a phenylpropyl group, a naphthylmethyl group, and an anthrylmethyl group.

In particular, R¹ and R² are each independently preferably a hydrocarbon group (preferably a cycloalkyl group) or a hydrogen atom.

L represents a divalent linking group.

When a plurality of L's are present, L's may be the same or different.

The divalent linking group may be, for example, -O-CO-O-, -COO-, -CONH-, -CO-, - O-, -S-, -SO-, -SO₂-, an alkylene group (preferably having 1 to 6 carbon atoms), a cycloalkylene group (preferably having 3 to 15 carbon atoms), an alkenylene group (preferably having 2 to 6 carbon atoms), or a divalent linking group that is a combination of a plurality of the foregoing. In particular, the divalent linking group is preferably -O-CO-O-, -COO-, -CONH-, -CO-, -O-, - SO₂-, -O-CO-O-alkylene group-, -COO-alkylene group-, or -CONH-alkylene group-, and more preferably -O-CO-O-, -O-CO-O-alkylene group-, -COO-, -CONH-, -SO₂-, or -COO-alkylene group-.

L is preferably, for example, a group represented by the following formula (AN1-1). *^{a}-(CR^{2a}₂)_{X}-Q-(CR^{2b}₂)_{Y}-*^{b} (AN1-1)

In the formula (AN1-1), *^{a} represents a bonding site to R³ in the formula (AN1). *^{b} represents a bonding site to -C(R¹)(R²)- in the formula (AN1).

X and Y each independently represent an integer of 0 to 10, and is preferably an integer of 0 to 3.

R^{2a} and R^{2b} each independently represent a hydrogen atom or a substituent.

When a plurality of R^{2a}'s and a plurality of R^{2b}'s are present, the plurality of R^{2a}'s and the plurality of R^{2b}'s present may be individually the same or different.

Note that, when Y is 1 or more, in the formula (AN1), R_{2b}'s in CR^{2b}₂ directly bonded to -C(R¹)(R²)- are not fluorine atoms.

Q represents *^{A}-O-CO-O-*^{B}, *^{A}-CO-*^{B}, *^{A}-CO-O-*^{B}, *^{A}-O-CO-*^{B}, *^{A}-O-*^{B}, *^{A}-S-*^{B}, or *^{A}-SO₂-*^{B.}

Note that, when X + Y in the formula (AN1-1) is 1 or more, and R₂ₐ's and R_{2b}'s in the formula (AN1-1) are all hydrogen atoms, Q represents *^{A}-O-CO-O-*^{B}, *^{A}-CO-*^{B}, *^{A}-O-CO-*^{B}, *^{A}-O-*^{B}, *^{A}-S-*^{B}, or *^{A}-SO₂-*^{B}.

*^{A} represent a bonding site on the R³ side in the formula (AN1) and *^{B} represent a bonding site on the -SO₃⁻ side in the formula (AN1).

In the formula (AN1), R³ represents an organic group.

The organic group is not particularly limited as long as it has 1 or more carbon atoms, and may be a linear group (for example, a linear alkyl group) or a branched group (for example, a branched alkyl group such as a t-butyl group), or may be a cyclic group. The organic group may have or may not have a substituent. The organic group may have or may not have a heteroatom (such as an oxygen atom, a sulfur atom, and/or a nitrogen atom).

In particular, R³ is preferably an organic group having a ring structure. The ring structure may be monocyclic or polycyclic, and may have a substituent. In the organic group including a ring structure, the ring is preferably directly bonded to L in the formula (AN1).

The organic group having a ring structure, for example, may have or may not have a heteroatom (such as an oxygen atom, a sulfur atom, and/or a nitrogen atom). The heteroatom may substitute one or more carbon atoms forming the ring structure.

The organic group having a ring structure is preferably, for example, a hydrocarbon group having a ring structure, a lactone ring group, or a sultone ring group. In particular, the organic group having a ring structure is preferably a hydrocarbon group having a ring structure.

The hydrocarbon group having a ring structure is preferably a monocyclic or polycyclic cycloalkyl group. Such groups may have a substituent.

The cycloalkyl group may be monocyclic (such as a cyclohexyl group) or polycyclic (such as an adamantyl group), and preferably has 5 to 12 carbon atoms.

The lactone group and the sultone group are, for example, preferably a group provided by removing, in any one of the above-described structures represented by the formulas (LC1-1) to (LC1-21) and the above-described structures represented by the formulas (SL1-1) to (SL1-3), a single hydrogen atom from a ring-member atom constituting the lactone structure or the sultone structure.

The non-nucleophilic anion may be a benzenesulfonate anion, and is preferably a benzenesulfonate anion substituted with a branched alkyl group or a cycloalkyl group.

The non-nucleophilic anion is also preferably an anion represented by the following formula (AN2).

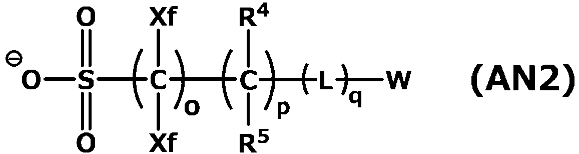

In the formula (AN2), o represents an integer of 1 to 3. p represents an integer of 0 to 10. q represents an integer of 0 to 10.

Xf's represent a hydrogen atom, a fluorine atom, an alkyl group substituted with at least one fluorine atom, or an organic group not having fluorine atoms. The alkyl group preferably has 1 to 10 carbon atoms, and more preferably 1 to 4 carbon atoms. The alkyl group substituted with at least one fluorine atom is preferably a perfluoroalkyl group.

Xf's are preferably a fluorine atom or a perfluoroalkyl group having 1 to 4 carbon atoms, and more preferably a fluorine atom or CF₃; still more preferably, both of Xf's are fluorine atoms.

R⁴ and R⁵ each independently represent a hydrogen atom, a fluorine atom, an alkyl group, or an alkyl group substituted with at least one fluorine atom. When a plurality of R⁴'s and a plurality of R⁵'s are present, R⁴'s and R⁵'s may be individually the same or different.

For R⁴ and R⁵, the alkyl group preferably has 1 to 4 carbon atoms. The alkyl group may have a substituent. R⁴ and R⁵ are preferably a hydrogen atom.

L represents a divalent linking group. L has the same definition as L in the formula (AN1).

W represents an organic group including a ring structure. In particular, preferred is a cyclic organic group.

The cyclic organic group may be, for example, an alicyclic group, an aryl group, or a heterocyclic group.

The alicyclic group may be monocyclic or may be polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. In particular, preferred are alicyclic groups having a bulky structure having 7 or more carbon atoms such as a norbornyl group, a tricyclodecanyl group, a tetracyclodecanyl group, a tetracyclododecanyl group, and an adamantyl group.

The aryl group may be monocyclic or polycyclic. Examples of the aryl group include a phenyl group, a naphthyl group, a phenanthryl group, and an anthryl group.

The heterocyclic group may be monocyclic or polycyclic. In particular, in the case of a polycyclic heterocyclic group, diffusion of acid can be further suppressed. The heterocyclic group may have aromaticity or may not have aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle not having aromaticity include a tetrahydropyran ring, a lactone ring, a sultone ring, and a decahydroisoquinoline ring. In the heterocyclic group, the heterocycle is preferably a furan ring, a thiophene ring, a pyridine ring, or a decahydroisoquinoline ring.

The cyclic organic group may have a substituent. The substituent may be, for example, an alkyl group (that may be linear or branched and preferably has 1 to 12 carbon atoms), a cycloalkyl group (that may have a monocycle, a polycycle, or a spiro ring, and preferably has 3 to 20 carbon atoms), an aryl group (preferably having 6 to 14 carbon atoms), a hydroxy group, an alkoxy group, an ester group, an amide group, a urethane group, a ureido group, a thioether group, a sulfonamide group, or a sulfonic acid ester group. Note that a carbon constituting the cyclic organic group (carbon contributing to formation of the ring) may be a carbonyl carbon.

The anion represented by the formula (AN2) is preferably SO₃⁻-CF₂-CH₂-OCO-(L)_{q'}-W, SO₃⁻-CF₂-CHF-CH₂-OCO-(L)_{q'}-W, SO₃⁻-CF₂-COO-(L)_{q'}-W, SO₃⁻-CF₂-CF₂-CH₂-CH₂-(L)_{q}-W, or SO₃⁻-CF₂-CH(CF₃)-OCO-(L)_{q'}-W. Here, L, q, and W are the same as those in the formula (AN2). q' represents an integer of 0 to 10.

The non-nucleophilic anion is also preferably an aromatic sulfonate anion represented by the following formula (AN3).

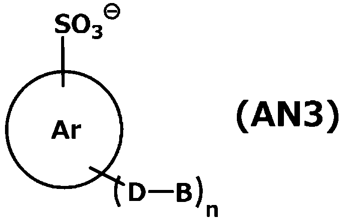

In the formula (AN3), Ar represents an aryl group (such as a phenyl group), and may further have a substituent other than the sulfonate anion and the -(D-B) group. Examples of the substituent that Ar may further have include a fluorine atom and a hydroxy group.

n represents an integer of 0 or more. n is preferably 1 to 4, more preferably 2 to 3, and still more preferably 3.

D represents a single bond or a divalent linking group. The divalent linking group may be an ether group, a thioether group, a carbonyl group, a sulfoxide group, a sulfo group, a sulfonic acid ester group, an ester group, or a group that is a combination of two or more of the foregoing.

B represents a hydrocarbon group.

B is preferably an aliphatic hydrocarbon group, and more preferably an isopropyl group, a cyclohexyl group, or an aryl group that may further have a substituent (such as a tricyclohexylphenyl group).

The non-nucleophilic anion is also preferably a disulfonamide anion.

The disulfonamide anion is, for example, an anion represented by N⁻(SO₂-R^{q})₂.

R^{q}'s represent an alkyl group that may have a substituent, and are preferably a fluoroalkyl group, and more preferably a perfluoroalkyl group. Two R^{q}'s may be bonded together to form a ring. The group formed by bonding together two R^{q}'s is preferably an alkylene group that may have a substituent, more preferably a fluoroalkylene group, and still more preferably a perfluoroalkylene group. The alkylene group preferably has 2 to 4 carbon atoms.

The compound (B) is also preferably at least one selected from the group consisting of compounds (I) to (II).

### Compound (I)

The compound (I) is a compound having one or more structural moieties X described below and one or more structural moieties Y described below, and is a compound that generates, upon irradiation with an actinic ray or a radiation, an acid including a first acidic moiety described below derived from the structural moiety X described below and a second acidic moiety described below derived from the structural moiety Y described below.

Structural moiety X: a structural moiety that is constituted by an anionic moiety A₁⁻ and a cationic moiety M₁⁺ and that forms, upon irradiation with an actinic ray or a radiation, the first acidic moiety represented by HA₁

Structural moiety Y: a structural moiety that is constituted by an anionic moiety A₂⁻ and a cationic moiety M₂⁺ and that forms, upon irradiation with an actinic ray or a radiation, the second acidic moiety represented by HA₂

The compound (I) satisfies the following condition I.

Condition I: A compound PI in which the cationic moiety M₁⁺ in the structural moiety X and the cationic moiety M₂⁺ in the structural moiety Y in the compound (I) are replaced by H⁺ has an acid dissociation constant a1 derived from an acidic moiety represented by HA₁ in which the cationic moiety M₁⁺ in the structural moiety X is replaced by H⁺, and an acid dissociation constant a2 derived from an acidic moiety represented by HA₂ in which the cationic moiety M₂⁺ in the structural moiety Y is replaced by H⁺, and the acid dissociation constant a2 is larger than the acid dissociation constant a1.

Hereinafter, the condition I will be more specifically described.

When the compound (I) is, for example, a compound that generates an acid having one first acidic moiety derived from the structural moiety X and one second acidic moiety derived from the structural moiety Y, the compound PI corresponds to a "compound having HA₁ and HA₂".

The acid dissociation constant a1 and the acid dissociation constant a2 of the compound PI will be more specifically described as follows: in determination of the acid dissociation constants of the compound PI, the pKa at the time when the compound PI turns into a "compound having A₁⁻ and HA₂" is the acid dissociation constant a1, and the pKa at the time when the "compound having A₁⁻ and HA₂" turns into a "compound having A₁⁻ and A₂⁻" is the acid dissociation constant a2.

When the compound (I) is, for example, a compound that generates an acid having two first acidic moieties derived from the structural moieties X and one second acidic moiety derived from the structural moiety Y, the compound PI corresponds to a "compound having two HA₁ and one HA₂".

In determination of the acid dissociation constants of the compound PI, the acid dissociation constant at the time when the compound PI turns into a "compound having one A₁⁻, one HA₁, and one HA₂" and the acid dissociation constant at the time when the "compound having one A₁⁻, one HA₁, and one HA₂" turns into a "compound having two A₁⁻ and one HA₂" correspond to the above-described acid dissociation constant a1. The acid dissociation constant at the time when the "compound having two A₁⁻ and one HA₂" turns into a "compound having two A₁⁻ and A₂⁻" corresponds to the acid dissociation constant a2. In other words, when the compound PI has a plurality of acid dissociation constants derived from the acidic moieties represented by HA₁ in which the cationic moiety M₁⁺ in the structural moiety X is replaced by H⁺, the value of the acid dissociation constant a2 is larger than the largest value of the plurality of the acid dissociation constants a1. Note that, in a case where the acid dissociation constant at the time when the compound PI turns into the "compound having one A₁⁻, one HA₁, and one HA₂" is defined as aa, and the acid dissociation constant at the time when the "compound having one A₁⁻, one HA₁, and one HA₂" turns into the "compound having two A₁⁻ and one HA₂" is defined as ab, the relationship between aa and ab satisfies aa < ab.

The acid dissociation constant a1 and the acid dissociation constant a2 can be determined by the above-described method of measuring an acid dissociation constant.

The compound PI corresponds to an acid generated upon irradiation of the compound (I) with an actinic ray or a radiation.

When the compound (I) has two or more structural moieties X, the structural moieties X may be the same or different. The two or more A₁⁻ and the two or more M₁⁺ may be individually the same or different.

In the compound (I), A₁⁻ and A₂⁻, and M₁⁺ and M₂⁺ may be individually the same or different, but A₁⁻ and A₂⁻ are preferably different from each other.

In the compound PI, the difference (absolute value) between the acid dissociation constant a1 (when a plurality of acid dissociation constants a1 are present, the maximum value thereof) and the acid dissociation constant a2 is preferably 0.1 or more, more preferably 0.5 or more, and still more preferably 1.0 or more. Note that the upper limit value of the difference (absolute value) between the acid dissociation constant a1 (when a plurality of acid dissociation constants a1 are present, the maximum value thereof) and the acid dissociation constant a2 is not particularly limited, but is, for example, 16 or less.

In the compound PI, the acid dissociation constant a2 is preferably 20 or less, and more preferably 15 or less. Note that the lower limit value of the acid dissociation constant a2 is preferably -4.0 or more.

In the compound PI, the acid dissociation constant a1 is preferably 2.0 or less, and more preferably 0 or less. Note that the lower limit value of the acid dissociation constant a1 is preferably -20.0 or more.

The anionic moiety A₁⁻ and the anionic moiety A₂⁻ are structural moieties including a negatively charged atom or atomic group and may be, for example, structural moieties selected from the group consisting of formulas (AA-1) to (AA-3) and formulas (BB-1) to (BB-6) below.

The anionic moiety A₁⁻ is preferably an anionic moiety that can form an acidic moiety having a small acid dissociation constant, in particular, more preferably any one of the formulas (AA-1) to (AA-3), and still more preferably any one of the formulas (AA-1) and (AA-3).

The anionic moiety A₂⁻ is preferably an anionic moiety that can form an acidic moiety having a larger acid dissociation constant than the anionic moiety A₁⁻, more preferably any one of the formulas (BB-1) to (BB-6), and still more preferably any one of the formulas (BB-1) and (BB-4).

Note that, in the formulas (AA-1) to (AA-3) and the formulas (BB-1) to (BB-6) below, * represent a bonding site.

In the formula (AA-2), R^{A} represent a monovalent organic group. The monovalent organic groups represented by R^{A} are not particularly limited, but may be, for example, a cyano group, a trifluoromethyl group, or a methanesulfonyl group.

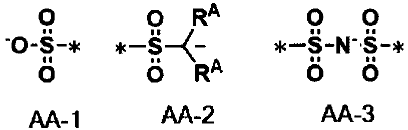

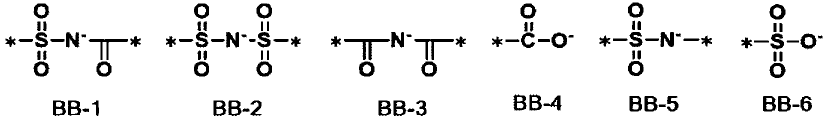

The cationic moiety M₁⁺ and the cationic moiety M₂⁺ are structural moieties including a positively charged atom or atomic group and may be, for example, singly charged organic cations. Note that such an organic cation may be, for example, the above-described organic cation represented by M⁺.

### Compound (II)

A compound (II) is a compound having two or more structural moieties X above and one or more structural moieties Z below, and is a compound that generates, upon irradiation with an actinic ray or a radiation, an acid including two or more first acidic moieties derived from the structural moieties X and the structural moiety Z.
Structural moiety Z: a nonionic moiety that can neutralize acid

In the compound (II), the definition of the structural moiety X and the definitions of A₁⁻ and M₁⁺ are the same as the above-described definition of the structural moiety X and definitions of A₁⁻ and M₁⁺ in the compound (I), and preferred examples are also the same.

For a compound PII in which the cationic moiety M₁⁺ in the structural moiety X is replaced by H⁺ in the compound (II), the preferred range of the acid dissociation constant a1 derived from the acidic moiety represented by HA₁ in which the cationic moiety M₁⁺ in the structural moiety X is replaced by H⁺ is the same as in the acid dissociation constant a1 in the compound PI.

Note that, when the compound (II) is, for example, a compound that generates an acid having two first acidic moieties derived from the structural moiety X and the structural moiety Z, the compound PII corresponds to a "compound having two HA₁". In determination of the acid dissociation constants of this compound PII, the acid dissociation constant at the time when the compound PII turns into a "compound having one A ⁻ and one HA₁" and the acid dissociation constant at the time when the "compound having one A₁⁻ and one HA₁" turns into a "compound having two A₁⁻" correspond to the acid dissociation constant a1.

The acid dissociation constant a1 can be determined by the above-described method of measuring an acid dissociation constant.

The compound PII corresponds to an acid generated upon irradiation of the compound (II) with an actinic ray or a radiation.

Note that the two or more structural moieties X may be the same or different. The two or more A₁⁻ and the two or more M₁⁺ may be individually the same or different.

The nonionic moiety that can neutralize acid in the structural moiety Z is not particularly limited, and is preferably, for example, a moiety including a group that can electrostatically interact with a proton or a functional group having an electron.

Examples of the group that can electrostatically interact with a proton or the functional group having an electron include a functional group having a macrocyclic structure such as cyclic polyether, and a functional group having a nitrogen atom having an unshared electron pair that does not contribute to π-conjugation. Examples of the nitrogen atom having an unshared electron pair that does not contribute to π-conjugation include nitrogen atoms having partial structures represented by the following formulas.

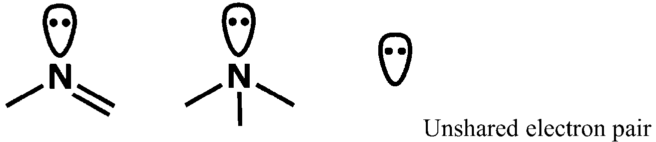

The partial structure of the group that can electrostatically interact with a proton or the functional group having an electron may be, for example, a crown ether structure, an azacrown ether structure, a primary to tertiary amine structure, a pyridine structure, an imidazole structure, or a pyrazine structure; in particular, preferred are primary to tertiary amine structures.

Examples of the non-cationic moieties that the compound (I) and the compound (II) can have are as follows.

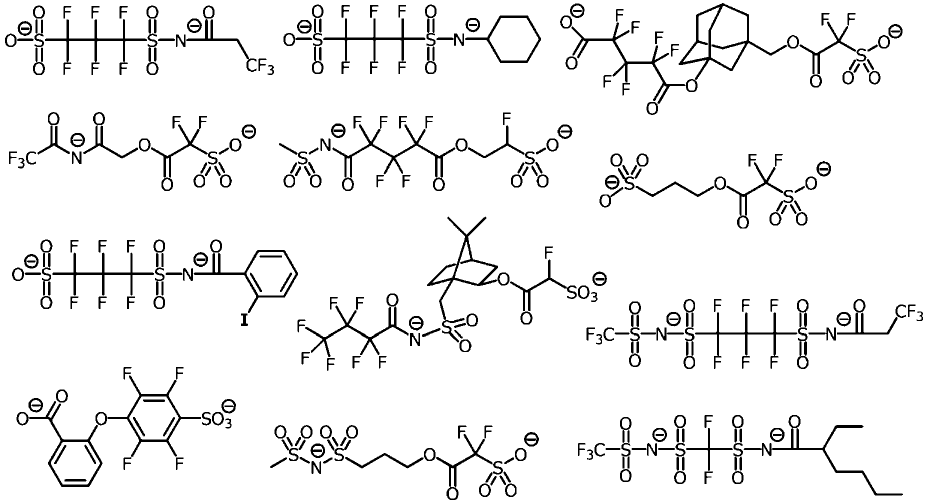

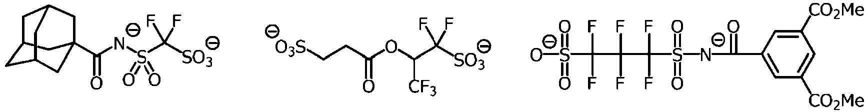

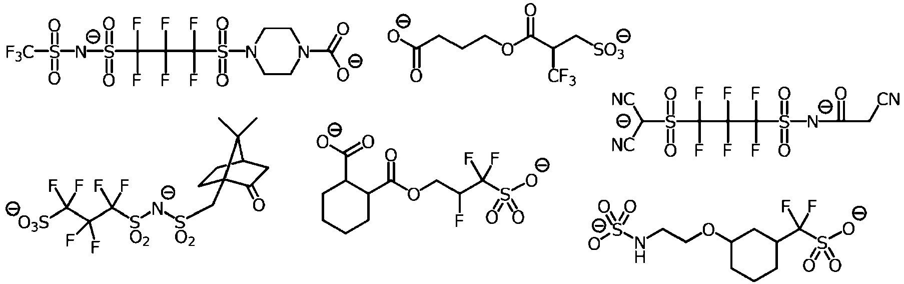

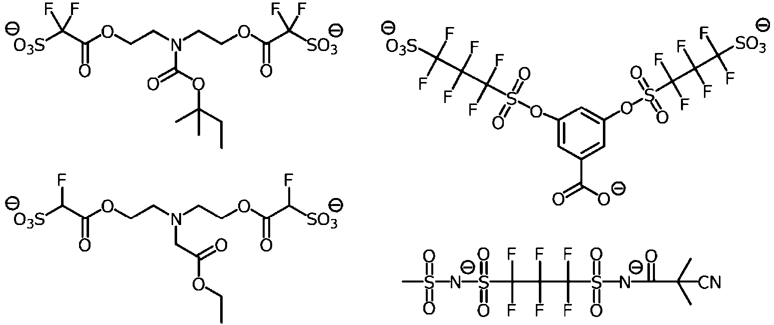

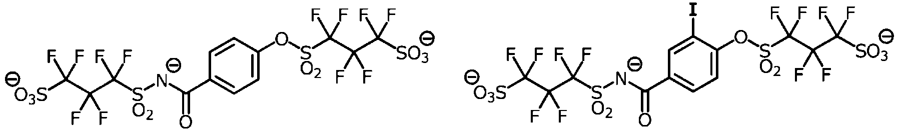

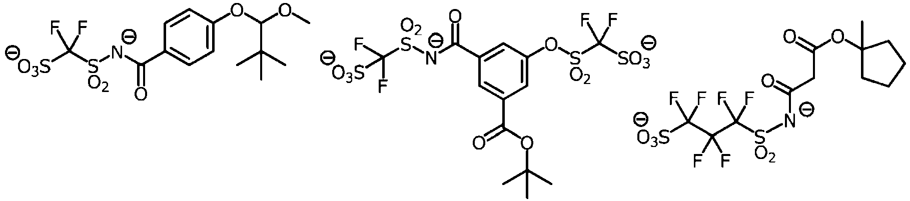

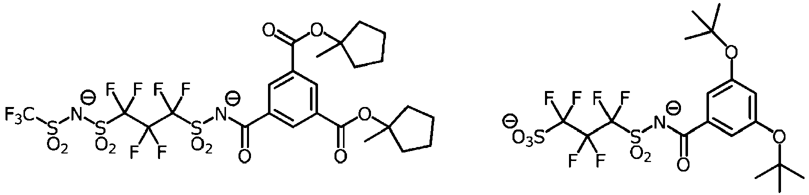

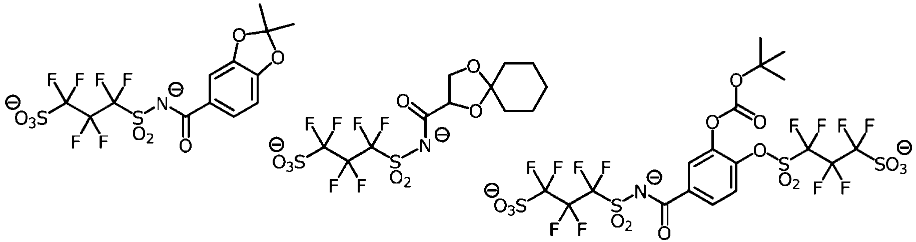

Specific examples of the compound (B) include, for example, the compounds described in [0320] to [0321] of WO2022/172715A. The above description is incorporated herein.

When the composition of the present invention includes the compound (B), the content of the compound (B) is not particularly limited, but is, relative to the total solid content of the composition of the present invention, preferably 0.5 mass% or more, and more preferably 1.0 mass% or more. When the composition of the present invention includes the compound (B), the content of the compound (B) is, relative to the total solid content of the composition of the present invention, preferably 60.0 mass% or less, more preferably 50.0 mass% or less, and still more preferably 40.0 mass% or less.

Such compounds (B) may be used alone or in combination of two or more thereof. When two or more thereof are used, the total content thereof is preferably within such a preferred content range.

### Acid diffusion control agent

The composition of the present invention may include an acid diffusion control agent.

The acid diffusion control agent serves as a quencher that traps the acid generated from the photoacid generator or the like upon exposure and that suppresses the reaction of the acid-decomposable resin, in the unexposed region, caused by an excess of generated acid.

The type of the acid diffusion control agent is not particularly limited, and examples thereof include a basic compound (DA), a low-molecular-weight compound (DB) having a nitrogen atom and having a group that leaves by the action of an acid, and a compound (DC) whose acid diffusion control ability is reduced or lost upon irradiation with an actinic ray or a radiation.

Examples of the compound (DC) include an onium salt compound (DD) that becomes a weak acid relative to the photoacid generator, and a basic compound (DE) whose basicity is reduced or lost upon irradiation with an actinic ray or a radiation.

Specific examples of the basic compound (DA) include, for example, those described in Paragraphs [0132] to [0136] of WO2020/066824A; specific examples of the basic compound (DE) whose basicity is reduced or lost upon irradiation with an actinic ray or a radiation include those described in Paragraphs [0137] to [0155] of WO2020/066824A, and those described in Paragraph [0164] of WO2020/066824A; and, specific examples of the low-molecular-weight compound (DB) having a nitrogen atom and having a group that leaves by the action of an acid include those described in Paragraphs [0156] to [0163] of WO2020/066824A.

Specific examples of the onium salt compound (DD) that becomes a weak acid relative to the photoacid generator include, for example, those described in Paragraphs [0305] to [0314] of WO2020/158337A.

In addition to those described above, for example, the publicly known compounds disclosed in Paragraphs [0627] to [0664] in US2016/0070167A, Paragraphs [0095] to [0187] in US2015/0004544A, Paragraphs [0403] to [0423] in US2016/0237190A, and Paragraphs [0259] to [0328] in US2016/0274458A can be suitably used as acid diffusion control agents.

When the composition of the present invention includes an acid diffusion control agent, the content of the acid diffusion control agent (when there are a plurality of acid diffusion control agents, the total content thereof) is, relative to the total solid content of the composition of the present invention, preferably 0.1 to 30.0 mass%, more preferably 0.1 to 15.0 mass%, and still more preferably 1.0 to 15.0 mass%.

The acid diffusion control agents may be used alone or in combination of two or more thereof. When two or more thereof are used, the total content thereof is preferably within such a preferred content range.

### Hydrophobic resin

The composition of the present invention may further include a hydrophobic resin different from the resin (A) (also referred to as "hydrophobic resin (E)").

The hydrophobic resin (E) is preferably designed so as to be localized in the surface of a resist film; however, unlike surfactants, the hydrophobic resin does not necessarily need to have intramolecularly a hydrophilic group, and does not necessarily contribute to homogeneous mixing of a polar substance and a nonpolar substance.

Advantages due to addition of the hydrophobic resin (E) may be control of static and dynamic contact angles (for water) at the surface of the resist film, and suppression of outgassing.

From the viewpoint of localization in the surface layer of the film, the hydrophobic resin (E) preferably has any one or more, more preferably two or more, of a fluorine atom, a silicon atom, and a CH₃ partial structure included in the side chain portion of the resin. The hydrophobic resin preferably has a hydrocarbon group having 5 or more carbon atoms. The resin may have such a group in the main chain or, as a substituent, in a side chain.

Examples of the hydrophobic resin (E) include the compounds described in Paragraphs [0275] to [0279] in WO2020/004306A.

When the composition of the present invention includes a hydrophobic resin (E), the content of the hydrophobic resin (E) relative to the total solid content of the composition of the present invention is preferably 0.01 to 20.0 mass%, and more preferably 0.1 to 15.0 mass%.

Such hydrophobic resins (E) may be used alone or in combination of two or more thereof. When two or more thereof are used, the total content thereof is preferably within such a preferred content range.

### Surfactant

The composition of the present invention may include a surfactant. In the case of including a surfactant, a pattern having higher adhesiveness and a less number of development defects can be formed.

The surfactant is preferably a fluorine-based and/or silicone-based surfactant.

Examples of the fluorine-based and/or silicone-based surfactant include the surfactants disclosed in Paragraphs [0218] and [0219] of WO2018/193954A.

Such surfactants may be used alone or in combination of two or more thereof.

When the composition of the present invention includes a surfactant, the surfactant content relative to the total solid content of the composition of the present invention is preferably 0.0001 to 2.0 mass%, more preferably 0.0005 to 1.0 mass%, and still more preferably 0.1 to 1.0 mass%.

Such surfactants may be used alone or in combination of two or more thereof. When two or more thereof are used, the total content thereof is preferably within such a preferred content range.

### Solvent

The composition of the present invention preferably includes a solvent.

The solvent preferably includes at least one of (M1) a propylene glycol monoalkyl ether carboxylate or (M2) at least one selected from the group consisting of a propylene glycol monoalkyl ether, a lactate, an acetate, an alkoxypropionate, a chain ketone, a cyclic ketone, a lactone, and an alkylene carbonate. Note that the solvent may further include a component other than the components (M1) and (M2).

A combination of the above-described solvent and the above-described resin is preferred from the viewpoint of improving the coatability of the composition of the present invention and reducing the number of pattern development defects. The above-described solvent is well-balanced in terms of solubility of the above-described resin, boiling point, and viscosity, to thereby suppress, for example, unevenness of the film thickness of the resist film and generation of deposit during spin-coating.

Details of the component (M1) and the component (M2) are described in Paragraphs [0218] to [0226] in WO2020/004306A, and these contents are incorporated herein.

When the solvent further includes a component other than the components (M1) and (M2), the content of the component other than the components (M1) and (M2) relative to the total amount of the solvent is preferably 5 to 30 mass%.

The content of the solvent in the composition of the present invention is set such that the solid-content concentration is preferably 0.5 to 30 mass%, and more preferably 1 to 20 mass%. This further improves the coatability of the composition of the present invention. Other additives

The composition of the present invention may further include a dissolution-inhibiting compound, a dye, a plasticizer, a photosensitizer, a light absorbent, and/or a compound that promotes solubility in a developer (for example, a phenol compound having a molecular weight of 1000 or less, or an alicyclic or aliphatic compound including a carboxyl group).

The "dissolution-inhibiting compound" is a compound that is decomposed by the action of an acid to cause a decrease in the degree of solubility in organic-based developers, and has a molecular weight of 3000 or less.

The composition of the present invention is also suitably used as a photosensitive composition for EUV exposure.

### Actinic ray-sensitive or radiation-sensitive film and pattern forming method

The present invention also relates to an actinic ray-sensitive or radiation-sensitive film formed from the composition of the present invention. The actinic ray-sensitive or radiation-sensitive film of the present invention is preferably a resist film.

The procedures of the pattern forming method using the composition of the present invention are not particularly limited, but preferably have the following steps:
Step 1: a step of using the composition of the present invention to form a resist film on a substrate;
Step 2: a step of exposing the resist film; and
Step 3: a step of developing the exposed resist film using a developer.

Hereinafter, procedures of the steps will be individually described in detail.

### Step 1: Resist film formation step

The step 1 is a step of using the composition of the present invention to form a resist film on a substrate.

Examples of the method of using the composition of the present invention to form a resist film on a substrate include a method of applying the composition of the present invention onto a substrate.

Note that the composition of the present invention is preferably filtered through a filter before application as needed. The filter preferably has a pore size of 0.1 µm or less, more preferably 0.05 µm or less, and still more preferably 0.03 µm or less. The filter is preferably formed of polytetrafluoroethylene, polyethylene, or nylon.

The composition of the present invention can be applied onto a substrate (for example, formed of silicon or silicon dioxide-covered silicon) used in the production of integrated circuit elements by an appropriate application method using a spinner, a coater, or the like. The application process is preferably spin-coating using a spinner. The spin-coating using a spinner is preferably performed at a rotation rate of 1000 to 3000 rpm (rotations per minute).

After application of the composition of the present invention, the substrate may be dried to form a resist film. Note that, as needed, as underlayers of the resist film, various underlying films (an inorganic film, an organic film, or an antireflection film) may be formed.

The drying process may be, for example, a process of performing heating to achieve drying. The heating can be performed using means included in an ordinary exposure device and/or an ordinary development device, or may alternatively be performed using a hot plate, for example. The heating temperature is preferably 80 to 150°C, more preferably 80 to 140°C, and still more preferably 80 to 130°C. The heating time is preferably 30 to 1000 seconds, more preferably 60 to 800 seconds, and still more preferably 60 to 600 seconds.

The film thickness of the resist film is not particularly limited, but is, from the viewpoint of enabling formation of more precise fine patterns, preferably 10 to 120 nm. In particular, in the case of employing EUV exposure, the film thickness of the resist film is more preferably 10 to 65 nm, and still more preferably 15 to 50 nm. In the case of employing ArF liquid immersion exposure, the film thickness of the resist film is more preferably 10 to 120 nm, and still more preferably 15 to 90 nm.

Note that, for an overlying layer of the resist film, a topcoat composition may be used to form a topcoat.

The topcoat composition preferably does not mix with the resist film, and can be uniformly applied for an overlying layer of the resist film. The topcoat is not particularly limited; a publicly known topcoat can be formed by a publicly known process; for example, on the basis of descriptions of Paragraphs [0072] to [0082] in JP2014-059543A, a topcoat can be formed.

For example, a topcoat including a basic compound and described in JP2013-61648A is preferably formed on the resist film. Specific examples of the basic compound that can be included in the topcoat include basic compounds that may be included in the composition of the present invention.

The topcoat also preferably includes a compound including at least one group or bond selected from the group consisting of an ether bond, a thioether bond, a hydroxy group, a thiol group, a carbonyl bond, and an ester bond.

### Step 2: Exposure step

The step 2 is a step of exposing the resist film.

The exposure process may be a process of irradiating the formed resist film, through a predetermined mask, with an actinic ray or a radiation.

Examples of the actinic ray or the radiation include infrared light, visible light, ultraviolet light, far-ultraviolet light, extreme ultraviolet light, X-rays, and electron beams; preferred is 250 nm or less; more preferred is 220 nm or less; particularly preferred is far-ultraviolet light having wavelengths of 1 to 200 nm; and specific examples thereof include the KrF excimer laser (248 nm), the ArF excimer laser (193 nm), an F₂ excimer laser (157 nm), EUV (13.5 nm), X-rays, and electron beams.

After the exposure, before development, baking (heating) is preferably performed. The baking accelerates the reaction in the exposed regions, to provide higher sensitivity and a better pattern profile.

The heating temperature is preferably 80 to 150°C, more preferably 80 to 140°C, and still more preferably 80 to 130°C.

The heating time is preferably 10 to 1000 seconds, more preferably 10 to 180 seconds, and still more preferably 30 to 120 seconds.

The heating can be performed using means included in an ordinary exposure device and/or an ordinary development device, and may alternatively be performed using a hot plate, for example.

This step is also referred to as post-exposure baking.

### Step 3: Development step

The step 3 is a step of using a developer to develop the exposed resist film, to form a pattern.

The developer may be an alkali developer or may be a developer containing an organic solvent (hereafter, also referred to as organic-based developer).

Examples of the development process include a process of immersing, for a predetermined time, the substrate in a tank filled with the developer (dipping process), a process of puddling, with the developer, the surface of the substrate using surface tension and leaving the developer at rest for a predetermined time to achieve development (puddling process), a process of spraying the developer to the surface of the substrate (spraying process), and a process of scanning, at a constant rate, over the substrate rotated at a constant rate, a developer ejection nozzle to continuously eject the developer (dynamic dispensing process).

After the step of performing development, a step of performing exchange with another solvent to stop the development may be performed.

The development time is not particularly limited as long as the resin in the unexposed regions is sufficiently dissolved in the time, and is preferably 10 to 300 seconds, and more preferably 20 to 120 seconds.

The temperature of the developer is preferably 0 to 50°C, and more preferably 15 to 35°C.

The alkali developer employed is preferably an alkali aqueous solution including an alkali. The type of the alkali aqueous solution is not particularly limited, but may be, for example, an alkali aqueous solution including a quaternary ammonium salt represented by tetramethylammonium hydroxide, an inorganic alkali, a primary amine, a secondary amine, a tertiary amine, an alcoholamine, a cyclic amine, or the like. In particular, the alkali developer is preferably an aqueous solution of a quaternary ammonium salt represented by tetramethylammonium hydroxide (TMAH). To the alkali developer, an appropriate amount of an alcohol, a surfactant, or the like may be added. The alkali developer ordinarily preferably has an alkali concentration of 0.1 to 20 mass%. The alkali developer ordinarily preferably has a pH of 10.0 to 15.0.

The organic-based developer is preferably a developer containing at least one organic solvent selected from the group consisting of ketone-based solvents, ester-based solvents, alcohol-based solvents, amide-based solvents, ether-based solvents, and hydrocarbon-based solvents.

A plurality of such solvents may be mixed together, or such a solvent may be mixed with a solvent other than those described above or water. The developer as a whole has a moisture content of preferably less than 50 mass%, more preferably less than 20 mass%, still more preferably less than 10 mass%, and particularly preferably contains substantially no moisture.

In the organic-based developer, the content of the organic solvent relative to the total amount of the developer is preferably 50 mass% or more and 100 mass% or less, more preferably 80 mass% or more and 100 mass% or less, still more preferably 90 mass% or more and 100 mass% or less, and particularly preferably 95 mass% or more and 100 mass% or less.

### Other step

The pattern forming method preferably includes a step of, after the step 3, using a rinse liquid to perform rinsing.

After the development step using an alkali developer, in the rinsing step, the rinse liquid employed may be, for example, pure water. Note that, to the pure water, an appropriate amount of surfactant may be added.

To the rinse liquid, an appropriate amount of surfactant may be added.

After the development step using an organic-based developer, in the rinsing step, the rinse liquid employed is not particularly limited as long as it does not dissolve the pattern, and may be a solution including an ordinary organic solvent. The rinse liquid employed is preferably a rinse liquid containing at least one organic solvent selected from the group consisting of hydrocarbon-based solvents, ketone-based solvents, ester-based solvents, alcohol-based solvents, amide-based solvents, and ether-based solvents.

The process of performing the rinsing step is not particularly limited; examples include a process of continuously ejecting, onto the substrate rotated at a constant rate, the rinse liquid (spin-coating process), a process of immersing, in a tank filled with the rinse liquid, the substrate for a predetermined time (dipping process), and a process of spraying, to the surface of the substrate, the rinse liquid (spraying process).

The pattern forming method may include a heating step (Post Bake) performed after the rinsing step. In this step, baking removes the developer and the rinse liquid remaining between and within the patterns. In addition, this step also provides an effect of annealing the resist pattern to address the rough surface of the pattern. The heating step after the rinsing step is performed ordinarily at 40 to 250°C (preferably 90 to 200°C) for ordinarily 10 seconds to 3 minutes (preferably 30 seconds to 120 seconds).

The formed pattern may be used as a mask for subjecting the substrate to etching treatment. Specifically, the pattern formed in the step 3 may be used as a mask for processing the substrate (or the underlayer film and the substrate), to form a pattern in the substrate.

The process of processing the substrate (or the underlayer film and the substrate) is not particularly limited, but is preferably a process of using the pattern formed in the step 3 as a mask for subjecting the substrate (or the underlayer film and the substrate) to dry etching, to thereby form a pattern in the substrate. The dry etching is preferably oxygen plasma etching.

Various materials used in the composition and the pattern forming method of the present invention (for example, a solvent, a developer, a rinse liquid, an antireflection film-forming composition, and a topcoat-forming composition) preferably do not include impurities such as metals. The content of impurities included in such materials is preferably 1 mass ppm (parts per million) or less, more preferably 10 mass ppb (parts per billion) or less, still more preferably 100 mass ppt or less, particularly preferably 10 mass ppt or less, and most preferably 1 mass ppt or less. The lower limit is not particularly limited, but is preferably 0 mass ppt or more. Examples of the metallic impurities include Na, K, Ca, Fe, Cu, Mg, Al, Li, Cr, Ni, Sn, Ag, As, Au, Ba, Cd, Co, Pb, Ti, V, W, and Zn.

The process of removing, from the various materials, impurities such as metals may be, for example, filtration using a filter. The details of filtration using a filter are described in Paragraph [0321] in WO2020/004306A.

Examples of the process of reducing the amount of impurities such as metals included in the various materials include a process of selecting, as raw materials constituting the various materials, raw materials having lower metal content, a process of subjecting raw materials constituting the various materials to filtration using a filter, and a process of performing distillation under conditions under which contamination is minimized by, for example, lining the interior of the apparatuses with TEFLON (registered trademark).

Instead of the filtration using a filter, an adsorption material may be used to remove impurities; alternatively, the filtration using a filter may be used in combination with an adsorption material. Such adsorption materials can be publicly known adsorption materials, and examples include inorganic-based adsorption materials such as silica gel and zeolite, and organic-based adsorption materials such as active carbon. In order to reduce the amount of impurities such as metals included in the various materials, ingress of metallic impurities in the production steps needs to be prevented. Whether or not metallic impurities are sufficiently removed from the production apparatuses can be determined by measuring the content of metallic components included in the washing liquid having been used for washing the production apparatuses. The content of metallic components included in the washing liquid having been used is preferably 100 mass ppt (parts per trillion) or less, more preferably 10 mass ppt or less, and still more preferably 1 mass ppt or less. The lower limit is not particularly limited, but is preferably 0 mass ppt or more.

To organic-based treatment liquids such as the rinse liquid, in order to prevent electrostatic buildup and the subsequent electrostatic discharge causing failure of the chemical solution pipe and various parts (such as a filter, an O-ring, and a tube), a conductive compound may be added. The conductive compound is not particularly limited, but may be, for example, methanol. The amount of addition is not particularly limited, but is, from the viewpoint of maintaining preferred development performance or rinsing performance, preferably 10 mass% or less, and more preferably 5 mass% or less. The lower limit is not particularly limited, but is preferably 0.01 mass% or more.

Examples of the chemical solution pipe include various pipes formed of SUS (stainless steel), or coated with polyethylene, polypropylene, or a fluororesin (such as polytetrafluoroethylene or a perfluoroalkoxy resin) treated so as to be antistatic. Similarly for the filter and the O-ring, polyethylene, polypropylene, or a fluororesin (such as polytetrafluoroethylene or a perfluoroalkoxy resin) treated so as to be antistatic can be used. Method for producing electronic device

The present invention also relates to a method for producing an electronic device, the method including the above-described pattern forming method, and an electronic device produced by the production method.

In preferred embodiments, an electronic device according to the present invention is mounted on electric and electronic apparatuses (home appliances, OA (Office Automation), media-related apparatuses, optical apparatuses, communication apparatuses, and the like). Onium salt represented by formula (N2-1)

The present invention also relates to the above-described onium salt represented by the formula (N2-1).

### Onium salt represented by formula (N5-1)

The present invention also relates to the above-described onium salt represented by the formula (N5-1).

### EXAMPLES

Hereinafter, the present invention will be described further in detail with reference to Examples. In the following Examples, materials, usage amounts, ratios, details of treatments, and orders of treatments can be appropriately changed without departing from the spirit and scope of the present invention. Thus, the scope of the present invention should not be construed as being limited to the following Examples.

Various components used in the resist compositions of Examples and Comparative Examples will be described below.

### Onium salt (N1)

The onium salt (N1) employed were PAG1 to PAG33. PAG1 to PAG33 are photoacid generators.

In Comparative Examples, Z-1 and Z-2 were used as photoacid generators that are not the onium salt (N1). For convenience, Table 2 below will describe Z-1 and Z-2 also in the column of Onium salt (N1).

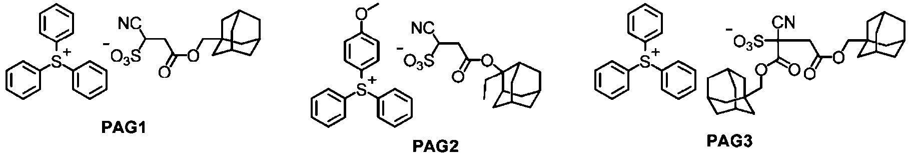

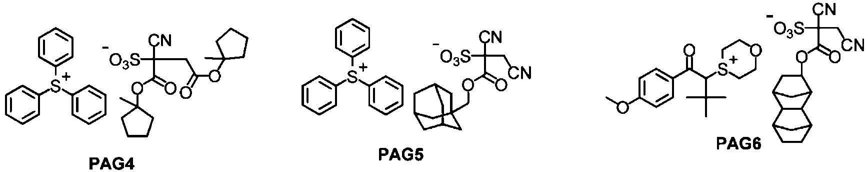

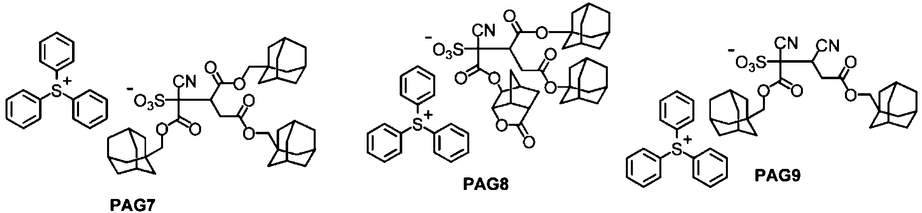

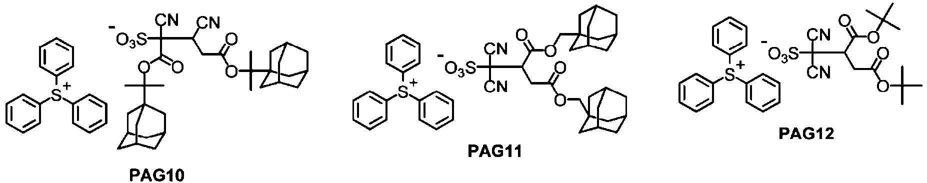

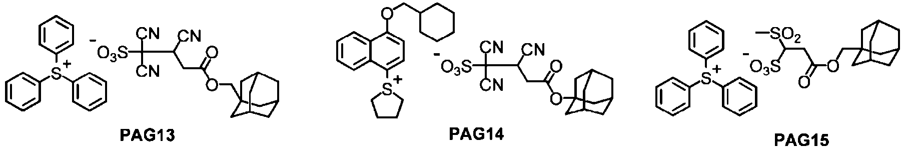

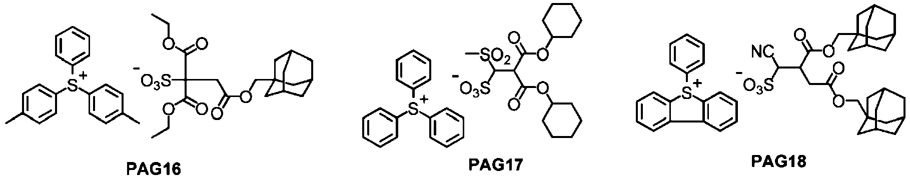

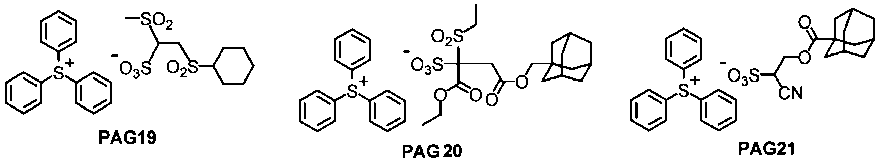

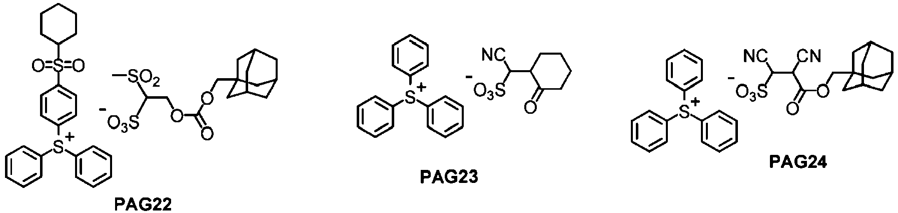

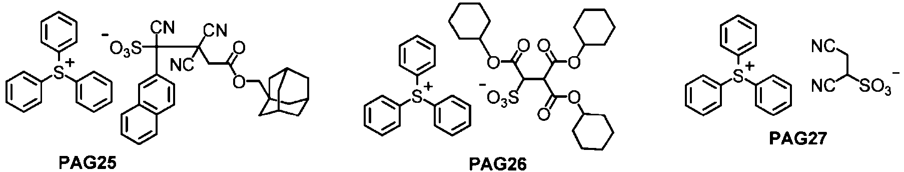

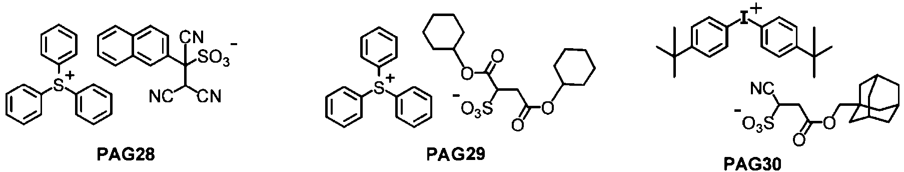

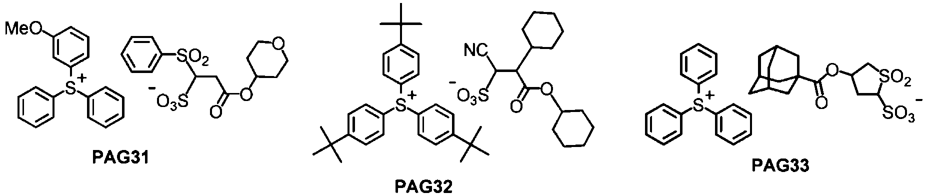

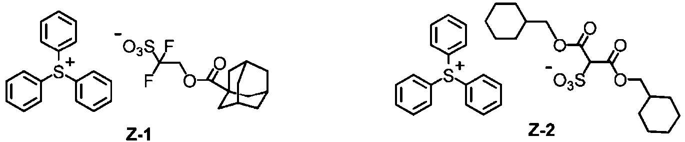

Synthesis examples of the onium salt (N1) will be described below.

### Synthesis Example 1: synthesis of PAG1

### Synthesis of intermediate 1-1

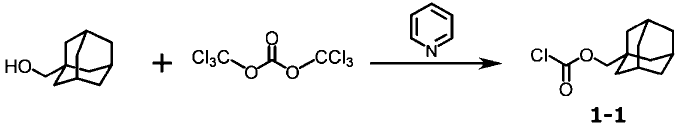

To a mixed solution of 35.7 g (0.12 mol) of triphosgene and 544 ml of toluene cooled to -10°C or less under a nitrogen atmosphere under stirring, 30.5 g (0.39 mol) of pyridine was carefully added dropwise such that the internal temperature was kept at 0°C or less. In this state, stirring was performed at 0°C or less for 1 hour; subsequently, 40 g (0.24 mol) of adamantanemethanol was gradually added, and stirring was performed at room temperature (23°C) for 5 hours. The reaction solution was filtered and the filtrate was concentrated under a reduced pressure to obtain 31.5 g of a crude product of an intermediate 1-1 (yield: 57%). This crude product, which included toluene, was directly used for the next reaction.
¹H NMR (CDCl₃): 3.91, 2.01, 1.79-1.60, 1.55 ppm

### Synthesis of intermediate 1-2

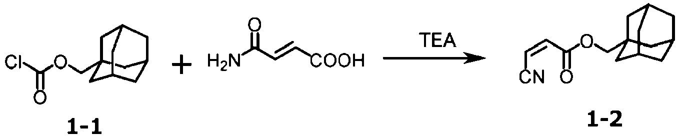

To a mixed solution of 6.9 g (0.060 mol) of maleic monoamide and 64 g of methylene chloride cooled to 0°C or less in a nitrogen atmosphere under stirring, 12.4 g (0.122 mol) of triethylamine (TEA) was added dropwise; subsequently, 43.8 g (purity: 68.5%, 0.131 mol) of the intermediate 1-1 was added dropwise; and stirring was performed at room temperature overnight. To the reaction solution, 69 g of water was added, and the organic layer was extracted. The organic layer was further washed with 69 g of water three times, and subsequently concentrated under a reduced pressure. The obtained residue was purified by silica gel column chromatography (hexane/ethyl acetate = 4/1 (volumetric ratio)) to thereby obtain 13.6 g of an intermediate 1-2 (yield: 89%).
¹H NMR (CDCl₃): 6.58, 5.95, 3.88, 2.00, 1.78-1.51 ppm

### Synthesis of intermediate 1-3

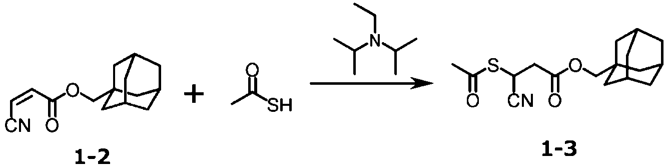

To a mixed solution of 13.4 g (0.054 mol) of the intermediate 1-2 and 214 ml of tetrahydrofuran (THF) cooled to -20°C or less under a nitrogen atmosphere under stirring, a mixed solution of 7.3 g (0.096 mol) of thioacetic acid, 16.2 g (0.125 mol) of diisopropylethylamine, and 53 ml of THF was added dropwise to the solution; and stirring was performed at room temperature overnight. The reaction solution was cooled to 0°C; 178 g of methylene chloride and 668 g of water were added, and the organic layer was extracted. The organic layer was washed with 668 g of a 10 mass% aqueous ammonium chloride solution and 668 g of water, and subsequently concentrated under a reduced pressure. The obtained residue was purified by silica gel column chromatography (hexane/ethyl acetate = 4/1 (volumetric ratio)) to thereby obtain 14.4 g of an intermediate 1-3 (yield: 82%).
¹H NMR (CDCl₃): 4.65, 3.77, 2.96, 2.42, 2.00, 1.78-1.50 ppm

### Synthesis of intermediate 1-4

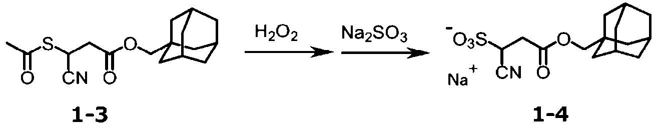

To a mixed solution of 14.0 g (0.044 mol) of the intermediate 1-3 and 280 ml of acetic acid under stirring at 40°C under a nitrogen atmosphere, 46.5 g (0.41 mol) of a 30 mass% hydrogen peroxide solution was carefully added dropwise. After stirring at 40°C overnight, the mixture was cooled to -5°C or less, and a mixed solution of 51.7 g (0.41 mol) of sodium sulfite and 310 g of water was added. This was stirred at 0°C or less for 1 hour, and then concentrated under a reduced pressure to distill off the solvent to obtain a crude product of an intermediate 1-4. This crude product was not further purified and directly used for the next reaction.

### Synthesis of PAG1

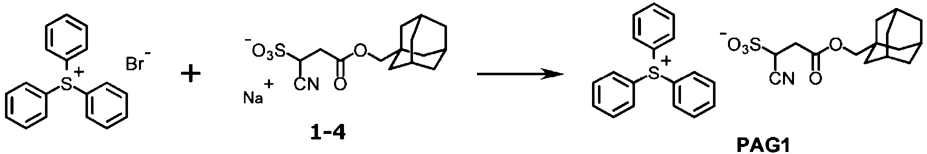

To a mixed solvent of 14.0 g (0.041 mol) of triphenylsulfonium bromide, 142 ml of methylene chloride, and 700 ml of water, 93.0 g of the crude product of the intermediate 1-4 (purity: 15%, 0.041 mol) was added, and stirring was performed at room temperature for 1 hour. After the reaction, the organic layer was extracted, washed three times with 700 ml of water, and then concentrated under a reduced pressure. To the residue, 200 ml of cyclopentyl methyl ether was added; stirring was performed at room temperature; filtration was performed and the resultant crystals were vacuum-dried to thereby obtain 15 g of PAG1 (yield: 63%).
¹H NMR (CDCl₃): 7.78-7.67, 4.16, 3.72, 3.31, 3.09, 1.97, 1.74-1.61, 1.53 ppm

### Synthesis Example 2: synthesis of PAG15

### Synthesis of intermediate 15-1

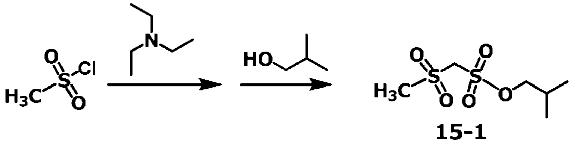

To a mixed solution of 26.4 g (0.26 mol) of triethylamine and 70 ml of acetonitrile under stirring at -40°C under a nitrogen atmosphere, 20 g (0.17 mol) of methanesulfonyl chloride was slowly added dropwise. This was stirred for 1 hour; subsequently, 6.5 g (0.087 mol) of 2-butanol was slowly added dropwise, and stirring was further performed for 2 hours. Filtration was performed to remove precipitated triethylamine hydrochloride; subsequently, to the filtrate, 150 ml of ethyl acetate was added; the resultant solution was added to 100 ml of 1 mol/1 aqueous hydrochloric acid ice-cooled to 10°C or less. A liquid separation operation was performed and the aqueous layer was removed; subsequently, the organic layer was washed three times with 100 ml of water and concentrated under a reduced pressure to distill off the solvent. The obtained residue was purified by silica gel column chromatography (hexane/ethyl acetate = 2/1 (volumetric ratio)) to thereby obtain 11.7 g of an intermediate 15-1 (yield: 58%). ¹H NMR (CDCl₃): 4.56, 4.22, 3.25, 2.11, 1.01 ppm

### Synthesis of intermediate 15-2

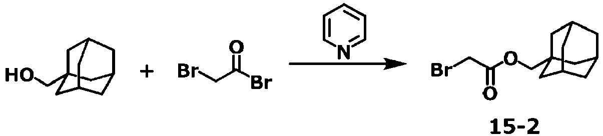

To a mixed solution of 10 g (0.060 mol) of 1-adamantanemethanol, 7.1 g (0.090 mol) of pyridine, and 100 ml of methylene chloride under stirring under ice-cooling at 5°C or less, 13.4 g (0.066 mol) of bromoacetic acid bromide was slowly added dropwise. This was stirred for 1 hour, and subsequently 80 ml of water was slowly added dropwise. A liquid separation operation was performed and the aqueous layer was removed; subsequently, the organic layer was washed once with 100 ml of aqueous HBr diluted to 0.5 mol/l and three times with 80 ml of water, and subsequently concentrated under a reduced pressure to distill off the solvent to obtain 16 g of a crude product of an intermediate 15-2. This crude product was not further purified and directly used for the next reaction.

### Synthesis of intermediate 15-3

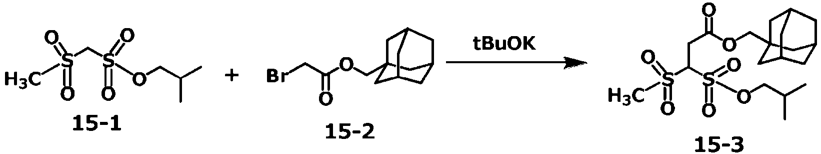

To a mixed solution of 5.0 g (0.022 mol) of the intermediate 15-1, 50 ml of tetrahydrofuran (THF), and 20 ml of N,N-dimethylformamide (DMF), 2.6 g (0.023 mol) of tert-butoxy potassium was slowly added at 0°C under a nitrogen atmosphere, and this was stirred for 10 minutes. Subsequently, 6.9 g (0.024 mol) of the intermediate 15-2 was slowly added, and stirring was further performed at room temperature (23°C) for 5 hours. The reaction solution was added to 70 ml of 1 mol/l aqueous hydrochloric acid under ice-cooling at 10°C or less. Ethyl acetate (150 ml) was added to perform a liquid separation operation, and the aqueous layer was removed; subsequently, the organic layer was washed three times with 70 ml of water, and concentrated under a reduced pressure to distill off the solvent. The obtained residue was purified by silica gel column chromatography (hexane/ethyl acetate = 4/1 (volumetric ratio)) to thereby obtain 3.5 g of an intermediate 15-3 (yield: 37%).
¹H NMR (CDCl₃): 5.06, 4.21, 3.79, 3.34-3.18, 3.27, 2.06, 2.00, 1.78-1.61, 1.54, 1.00 ppm

### Synthesis of intermediate 15-4

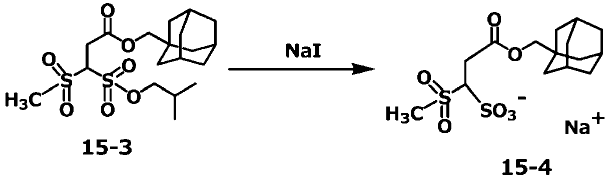

To a mixed solution of 3.0 g (6.9 mmol) of the intermediate 15-3 and 50 ml of acetonitrile, 1.1 g (7.6 mmol) of sodium iodide was added, and stirring was performed at 50°C for 3 hours. The precipitated crystals were collected by filtration to obtain 2.4 g of an intermediate 15-4 (yield: 87%).
¹H NMR (DMSO-d6): 4.27, 3.65, 3.13, 2.98-2.80, 1.94, 1.73-1.56, 1.50 ppm

### Synthesis of PAG15

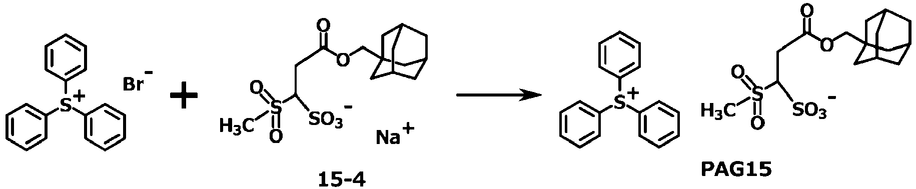

To a mixed solvent of 2.1 g (5.2 mmol) of the intermediate 15-4, 35 ml of methylene chloride, and 35 ml of water, 1.8 g (5.4 mmol) of triphenylsulfonium bromide was added, and stirring was performed at room temperature (23°C) for 1 hour. After the reaction, the organic layer was extracted, washed once with 35 ml of 0.1 mol/l aqueous hydrochloric acid and four times with 35 ml of water, and then concentrated under a reduced pressure. To the residue, 30 ml of tert-butyl methyl ether was added; stirring was performed at room temperature; filtration was performed and the resultant solid was vacuum-dried to obtain 3 g of PAG15 (yield: 88%). ¹H NMR (CDCl₃): 7.82-7.66, 4.76, 3.68, 3.31, 3.35-3.12, 1.95, 1.74-1.58, 1.52 ppm

### Other onium salts (N1) were also synthesized in the same manner as described above. Resin (A)

The resin (A) (resin that is subjected to action of an acid to undergo an increase in polarity) employed were B-1 to B-6.

The structural formulas and contents (mol%) of repeating units included in B-1 to B-6, and the weight-average molecular weights (Mw) and dispersities (Mw/Mn) of B-1 to B-6 will be described below.

The content of each repeating unit is the content ratio (molar ratio) of the repeating unit relative to all the repeating units included in the resin.

The weight-average molecular weight (Mw) and dispersity (Mw/Mn) of the resin were measured by GPC (carrier: tetrahydrofuran (THF)) (polystyrene-equivalent amounts). The contents of the repeating units were measured by ¹³C-NMR (nuclear magnetic resonance).

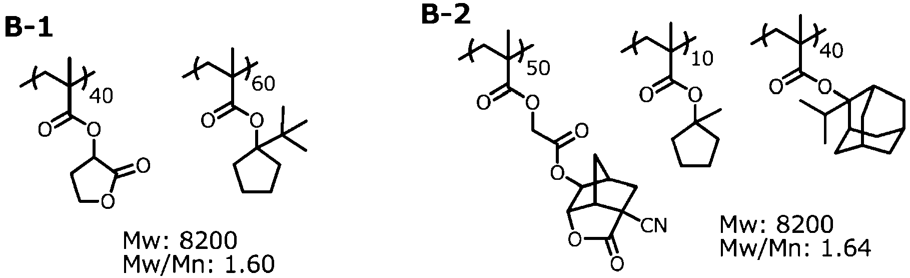

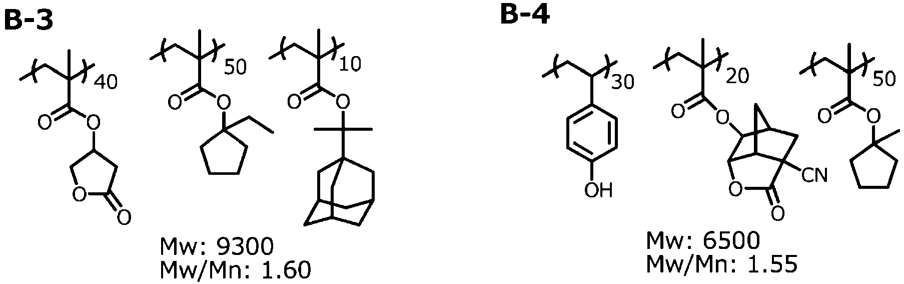

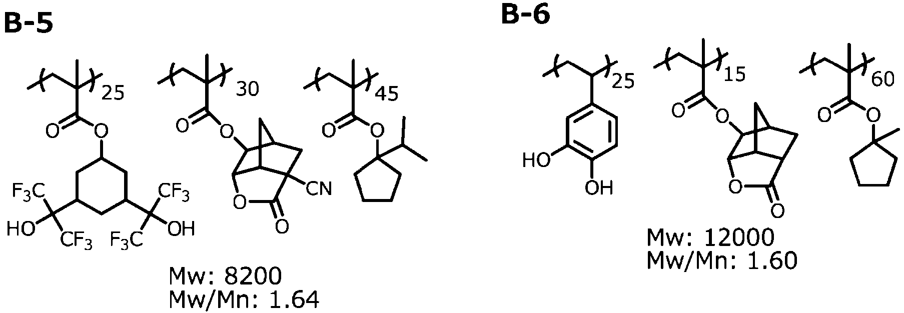

### Acid diffusion control agent

The acid diffusion control agents employed were C-1 to C-6.

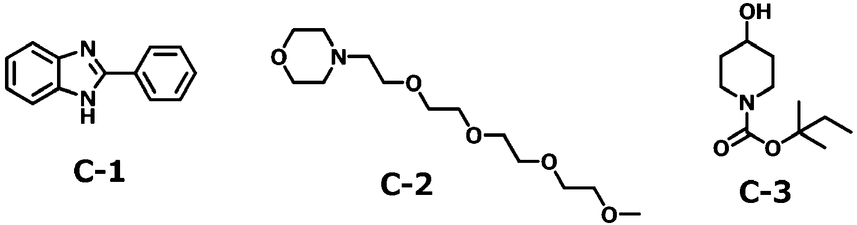

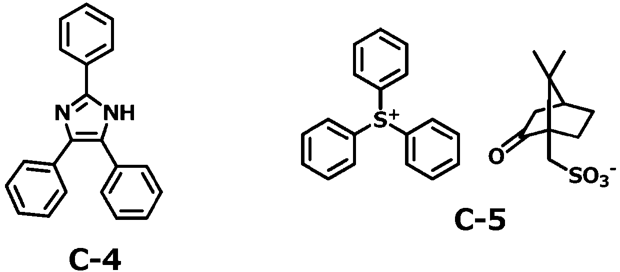

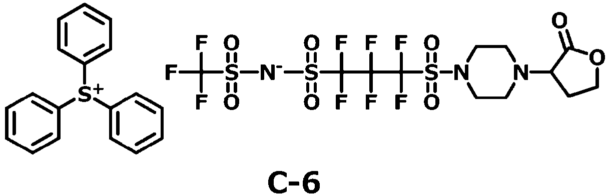

### Hydrophobic resin

The hydrophobic resins employed were D-1 and D-2.

The structural formulas and contents (mol%) of repeating units included in D-1 and D-2, and the weight-average molecular weights (Mw) and the dispersities (Mw/Mn) of D-1 and D-2 will be described below.

The content of each repeating unit is the content ratio (molar ratio) of the repeating unit relative to all the repeating units included in the resin.

The weight-average molecular weight (Mw) and dispersity (Mw/Mn) of the resin were measured by GPC (carrier: tetrahydrofuran (THF)) (polystyrene-equivalent amounts). The contents of the repeating units were measured by ¹³C-NMR.

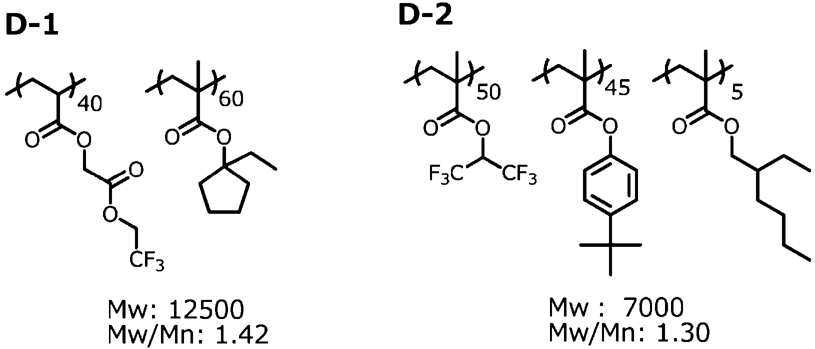

### Surfactant

The surfactant employed was E-1.
E-1: PolyFox PF-6320 (manufactured by OMNOVA Solutions Inc.; fluorine-based) Solvent

The solvents employed are as follows.
S1: propylene glycol monomethyl ether acetate (PGMEA)
S2: propylene glycol monomethyl ether (PGME)
S3: γ-butyrolactone
S4: ethyl lactate
S5: cyclohexanone
S6: 2-heptanone

### ArF exposure

### Preparation of resist compositions

The components described in Table 1 and Table 2 were dissolved in the solvents described in Table 1 and Table 2 to prepare solutions having a solid-content concentration of 4.0 mass%, and the solutions were filtered through a polyethylene filter having a pore size of 0.02 µm to prepare resist compositions.

Note that the solid content means all the components other than the solvent. The obtained resist compositions were used in Examples and Comparative Examples.

In Table 1 and Table 2, the columns "mass%" indicate the content (mass%) of each component relative to the total solid content in the resist composition. Table 1 and Table 2 also describe the amounts of the solvents used (mass ratios).

For such a component, when two or more thereof were used, the types and the contents thereof are described so as to be separated by "/". For example, in the resist composition A-7, "PAG1/PAG13" indicates that two onium salts (N1), PAG1 and PAG13, were used, and "7.4/8.1" indicates that the PAG1 content is 7.4 mass% and the PAG13 content is 8.1 mass%.

**Table 1**

| Resist composition | Resin (A) | | Onium salt (N1) | | Acid diffusion control agent | | Hydrophobic resin | | Surfactant | | Solvent | |
|---|---|---|---|---|---|---|---|---|---|---|---|---|
| | Type | mass% | Type | mass% | Type | mass% | Type | mass% | Type | mass% | Type | Mass ratio |
| A-1 | B-1 | 81.3 | PAG1 | 14.7 | C-1 | 2.3 | D-1 | 1.5 | E-1 | 0.1 | S1/S2 | 70/30 |
| A-2 | B-1 | 82.7 | PAG1 | 14.7 | C-3 | 2.6 | - | - | - | - | S1/S2 | 80/20 |
| A-3 | B-3 | 75.0 | PAG1 | 14.7 | C-6 | 10.3 | - | - | - | - | S1/S3 | 80/20 |
| A-4 | B-3 | 81.2 | PAGI | 14.7 | C-3 | 2.6 | D-2 | 1.5 | - | - | S1/S2 | 90/10 |
| A-5 | B-5 | 81.9 | PAG1 | 14.7 | C-2 | 3.3 | - | - | - | - | S1/S2/S4 | 70/25/5 |
| A-6 | B-6 | 81.7 | PAG1 | 14.7 | C-4 | 3.6 | - | - | - | - | S1/S2 | 60/40 |
| A-7 | B-2 | 82.2 | PAG1/PAG13 | 7.4/8.1 | C-1 | 2.3 | - | - | - | - | S1/S2 | 70/30 |
| A-8 | B-1 | 81.8 | PAG2 | 15.8 | C-1 | 2.3 | - | - | - | - | S1/S2/S3 | 70/25/5 |
| A-9 | B-2 | 72.4 | PAG2 | 15.8 | C-6 | 10.3 | D-2 | 1.5 | - | - | S1/S2 | 70/30 |
| A-10 | B-4 | 76.9 | PAG3 | 19.6 | C-4 | 3.6 | - | - | - | - | S1/S2 | 50/50 |
| A-11 | B-3 | 80.4 | PAG4 | 16.2 | C-2 | 3.3 | - | - | - | - | S1/S3 | 75/25 |
| A-12 | B-5 | 80.2 | PAG4 | 16.2 | C-4 | 3.6 | - | - | - | - | S1/S2/S6 | 70/25/5 |
| A-13 | B-3 | 80.5 | PAG5 | 15.4 | C-3 | 2.6 | D-1 | 1.5 | - | - | S1/S2 | 75/25 |
| A-14 | B-6 | 78.7 | PAG5 | 15.4 | C-5 | 5.9 | - | - | - | - | S1/S2 | 60/40 |
| A-15 | B-2 | 78.4 | PAG6 | 16.8 | C-2 | 3.3 | D-1 | 1.5 | - | - | S1/S2/S5 | 70/25/5 |
| A-16 | B-3 | 75.7 | PAG6 | 16.8 | C-5 | 5.9 | D-2 | 1.5 | - | - | S1/S2 | 70/30 |
| A-17 | B-5 | 79.6 | PAG6 | 16.8 | C-4 | 3.6 | - | - | - | - | S1/S2 | 90/10 |
| A-18 | B-4 | 73.0 | PAG7 | 24.7 | C-1 | 2.3 | - | - | - | - | S1/S3 | 80/20 |
| A-19 | B-1 | 72.2 | PAG8 | 23.7 | C-3 | 2.6 | D-2 | 1.5 | - | - | S1/S2/S3 | 80/10/10 |
| A-20 | B-6 | 72.7 | PAG8 | 23.7 | C-4 | 3.6 | - | - | - | - | S1/S2 | 70/30 |
| A-21 | B-2 | 76.1 | PAG9 | 20.5 | C-2 | 3.3 | - | - | - | - | S1/S2/S4 | 35/60/5 |
| A-22 | B-5 | 72.0 | PAG9 | 20.5 | C-5 | 5.9 | D-2 | 1.5 | - | - | S1/S2 | 40/60 |
| A-23 | B-1 | 74.2 | PAG10 | 21.9 | C-1 | 2.3 | D-1 | 1.5 | - | - | S1/S2 | 95/5 |
| A-24 | B-4 | 74.5 | PAG10 | 21.9 | C-4 | 3.6 | - | - | - | - | S1/S2 | 80/20 |
| A-25 | B-3 | 72.0 | PAG11 | 20.5 | C-5 | 5.9 | D-2 | 1.5 | - | - | S1/S4 | 95/5 |
| A-26 | B-6 | 74.6 | PAG11 | 20.5 | C-2 | 3.3 | D-1 | 1.5 | - | - | S1/S2 | 70/30 |
| A-27 | B-4 | 80.5 | PAG12 | 15.9 | C-4 | 3.6 | - | - | - | - | S1/S2 | 70/30 |
| A-28 | B-2 | 79.8 | PAG13 | 16.3 | C-1 | 2.3 | D-1 | 1.5 | - | - | S1/S4 | 90/10 |
| A-29 | B-5 | 77.7 | PAG13 | 16.3 | C-5 | 5.9 | - | - | - | - | S1/S2 | 50/50 |
| A-30 | B-1 | 78.3 | PAG14 | 17.6 | C-3 | 2.6 | D-2 | 1.5 | - | - | S1/S3 | 85/15 |

**Table 2**

| Resist composition | Resin (A) | | Onium salt (N1) | | Acid diffusion control agent | | Hydrophobic resin | | Surfactant | | Solvent | |
|---|---|---|---|---|---|---|---|---|---|---|---|---|
| | Type | mass% | Type | mass% | Type | mass% | Type | mass% | Type | mass% | Type | Mass ratio |
| A-31 | B-1 | 80.0 | PAGl5 | 16.1 | C-1 | 2.3 | D-1 | 1.5 | E-1 | 0.1 | S1/S2 | 70/30 |
| A-32 | B-3 | 79.8 | PAGl5 | 16.1 | C-3 | 2.6 | D-2 | 1.5 | - | - | S1/S2/S3/S4 | 65/20/10/5 |
| A-33 | B-5 | 72.1 | PAGl5 | 16.1 | C-6 | 10.3 | D-2 | 1.5 | - | - | S1/S2/S5 | 75/20/5 |
| A-34 | B-6 | 80.4 | PAGl5 | 16.1 | C-4 | 3.6 | - | - | - | - | S1/S2 | 70/30 |
| A-35 | B-1 | 79.0 | PAG16 | 18.4 | C-3 | 2.6 | - | - | - | - | S1/S2 | 40/60 |
| A-36 | B-4 | 79.2 | PAG16 | 18.4 | C-1 | 2.3 | - | - | - | - | S1/S3/S4 | 85/10/5 |
| A-37 | B-5 | 72.1 | PAG17 | 17.6 | C-6 | 10.3 | - | - | - | - | S1/S2 | 80/20 |
| A-38 | B-1/B-5 | 38.1/34.0 | PAG17 | 17.6 | C-6 | 10.3 | - | - | - | - | S1/S2 | 80/20 |
| A-39 | B-2 | 76.1 | PAG18 | 19.9 | C-3 | 2.6 | D-1 | 1.5 | - | - | S1/S2 | 70/30 |
| A-40 | B-5 | 76.1 | PAG18 | 19.9 | C-3 | 2.6 | D-2 | 1.5 | - | - | S1/S2 | 60/40 |
| A-41 | B-3 | 81.8 | PAG19 | 14.9 | C-2 | 3.3 | - | - | - | - | S1/S2 | 50/50 |
| A-42 | B-6 | 74.8 | PAG19 | 14.9 | C-6 | 10.3 | - | - | - | - | S1/S2/S4 | 80/15/5 |
| A-43 | B-5 | 78.2 | PAG20 | 18.2 | C-4 | 3.6 | - | - | - | - | S1/S2 | 50/50 |
| A-44 | B-1 | 83.0 | PAG21 | 14.4 | C-3 | 2.6 | - | - | - | - | S1/S2 | 60/40 |
| A-45 | B-5 | 82.1 | PAG21 | 14.4 | C-4 | 3.6 | - | - | - | - | S1/S2/S3 | 75/15/10 |
| A-46 | B-1 | 76.5 | PAG22 | 20.1 | C-2 | 3.3 | - | - | - | - | S1/S2 | 70/30 |
| A-47 | B-4 | 82.1 | PAG23 | 12.0 | C-5 | 5.9 | - | - | - | - | S1/S2/S5 | 70/25/5 |
| A-48 | B-1 | 82.0 | PAG24 | 15.4 | C-3 | 2.6 | - | - | - | - | S1/S2 | 80/20 |
| A-49 | B-1 | 77.9 | PAG25 | 19.5 | C-3 | 2.6 | - | - | - | - | S1/S2 | 80/20 |
| A-50 | B-1 | 78.6 | PAG26 | 18.8 | C-3 | 2.6 | - | - | - | - | S1/S2 | 70/30 |
| A-51 | B-1 | 86.9 | PAG27 | 10.6 | C-3 | 2.6 | - | - | - | - | S1/S2 | 80/20 |
| A-52 | B-1 | 83.1 | PAG28 | 14.3 | C-3 | 2.6 | - | - | - | - | S1/S2 | 70/30 |
| A-53 | B-1 | 81.8 | PAG29 | 15.6 | C-3 | 2.6 | - | - | - | - | S1/S2 | 80/20 |
| A-54 | B-4 | 71.7 | PAG30 | 18.0 | C-6 | 10.3 | - | - | - | - | S1/S3 | 85/15 |
| A-55 | B-5 | 79.7 | PAG31 | 16.8 | C-4 | 3.6 | - | - | - | - | S1/S2 | 40/60 |
| A-56 | B-3 | 78.1 | PAG32 | 19.4 | C-3 | 2.6 | - | - | - | - | S1/S2/S4 | 80/15/5 |
| A-57 | B-1 | 72.2 | PAG33 | 16.0 | C-6 | 10.3 | D-1 | 1.5 | - | - | S1/S2 | 70/30 |
| A-58 | B-1 | 73.6 | PAGl5/z-2 | 8.1/8.0 | C-6 | 10.3 | - | - | - | - | S1/S2 | 90/10 |
| RA-1 | B-5 | 82.0 | Z-1 | 14.7 | C-2 | 3.3 | - | - | - | - | S1/S2 | 80/20 |
| RA-2 | B-5 | 80.7 | Z-2 | 16.0 | C-2 | 3.3 | - | - | - | - | S1/S5 | 70/30 |

### Pattern forming method (1): ArF exposure, alkali development (positive)

A resist composition immediately after production in Table 3 was applied onto a 6-inch Si wafer having been subjected to hexamethyldisilazane (HMDS) treatment in advance, using a spin coater Mark8 manufactured by Tokyo Electron Ltd., and dried on a hot plate at 100°C for 60 seconds to obtain a resist film having a film thickness of 90 nm. Here, 1 inch is 0.0254 m.

The wafer on which the resist film was formed was subjected to pattern exposure through an exposure mask using an ArF excimer laser scanner (manufactured by ASML, PAS5500/1500, wavelength: 193 nm, NA: 0.50). Subsequently, the resist film was baked at a temperature of 115°C for 60 seconds, then developed with a 2.38 mass% aqueous tetramethylammonium hydroxide solution (TMAHaq) for 30 seconds, rinsed with pure water, and then spin-dried. This provided a resist pattern of a 1:1 line-and-space pattern having a line width of 50 nm.

### Performance evaluation

### LWR (line width roughness) performance

When a 50 nm (1:1) line-and-space pattern resolved at the optimal exposure dose for resolving a line pattern having a line width of 50 nm on average was observed from above the pattern using a critical dimension scanning electron microscope (SEM (Hitachi, Ltd., S-9380II)), the line widths were observed at 50 points, and the standard deviation (σ) thereof was determined. The measurement variation of the line widths was evaluated on the basis of 3σ, and the value of 3σ was defined as LWR (nm). The smaller the value of LWR, the better the LWR performance. The LWR is preferably 4.9 nm or less, more preferably 4.4 nm or less, and particularly preferably 3.9 nm or less.

### Post-development residue

A section of the 1:1 line-and-space pattern having a line width of 50 nm was observed using a scanning electron microscope (SEM, manufactured by Hitachi, Ltd., S-9380II); cases where residue was not visually observed at all were evaluated as A, cases where a small but acceptable amount of residue was observed were evaluated as B, and cases where a large amount of residue was observed were evaluated as C.

The results will be described in Table 3.

**Table 3**

| | Resist composition | LWR (nm) | Post-development residue | Remarks |
|---|---|---|---|---|
| AP-1 | A-1 | 3.5 | A | Example |
| AP-2 | A-2 | 3.6 | A | Example |
| AP-3 | A-3 | 3.6 | A | Example |
| AP-4 | A-4 | 3.5 | A | Example |
| AP-5 | A-5 | 3.7 | A | Example |
| AP-6 | A-7 | 3.6 | A | Example |
| AP-7 | A-8 | 3.7 | A | Example |
| AP-8 | A-9 | 3.6 | A | Example |
| AP-9 | A-11 | 3.6 | A | Example |
| AP-10 | A-12 | 3.7 | A | Example |
| AP-11 | A-13 | 3.9 | A | Example |
| AP-12 | A-15 | 3.9 | A | Example |
| AP-13 | A-16 | 3.8 | A | Example |
| AP-14 | A-17 | 4.0 | A | Example |
| AP-15 | A-19 | 3.6 | A | Example |
| AP-16 | A-21 | 3.7 | A | Example |
| AP-17 | A-22 | 3.6 | A | Example |
| AP-18 | A-23 | 3.6 | A | Example |
| AP-19 | A-25 | 3.5 | A | Example |
| AP-20 | A-28 | 3.5 | A | Example |
| AP-21 | A-29 | 3.7 | A | Example |
| AP-22 | A-30 | 3.6 | A | Example |
| AP-23 | A-31 | 3.6 | A | Example |
| AP-24 | A-32 | 3.7 | A | Example |
| AP-25 | A-33 | 3.6 | A | Example |
| AP-26 | A-35 | 3.6 | A | Example |
| AP-27 | A-37 | 3.7 | A | Example |
| AP-28 | A-38 | 3.5 | A | Example |
| AP-29 | A-39 | 3.5 | A | Example |
| AP-30 | A-40 | 3.6 | A | Example |
| AP-31 | A-41 | 3.6 | A | Example |
| AP-32 | A-43 | 3.7 | A | Example |
| AP-33 | A-44 | 3.8 | A | Example |
| AP-34 | A-45 | 4.0 | A | Example |
| AP-35 | A-46 | 3.6 | A | Example |
| AP-36 | A-48 | 3.6 | A | Example |
| AP-37 | A-49 | 3.6 | A | Example |
| AP-38 | A-50 | 3.8 | A | Example |
| AP-39 | A-51 | 4.6 | B | Example |
| AP-40 | A-52 | 4.6 | B | Example |
| AP-41 | A-53 | 4.7 | B | Example |
| AP-42 | A-55 | 3.6 | A | Example |
| AP-43 | A-56 | 3.6 | A | Example |
| AP-44 | A-57 | 3.5 | A | Example |
| AP-45 | A-58 | 4.2 | A | Example |
| RAP-1 | RA-1 | 5.2 | C | Comparative Example |
| RAP-2 | RA-2 | 5.1 | C | Comparative Example |

### Pattern forming method (2): ArF exposure, organic-solvent development (negative)

A resist composition immediately after production in Table 4 was applied onto a 6-inch Si wafer having been subjected to hexamethyldisilazane (HMDS) treatment in advance, using a spin coater Mark8 manufactured by Tokyo Electron Ltd., and dried on a hot plate at 100°C for 60 seconds to obtain a resist film having a film thickness of 90 nm. Here, 1 inch is 0.0254 m.

The wafer on which the resist film was formed was subjected to pattern exposure through an exposure mask using an ArF excimer laser scanner (manufactured by ASML, PAS5500/1500, wavelength: 193 nm, NA: 0.50). Subsequently, the resist film was baked at a temperature of 115°C for 60 seconds, then developed with n-butyl acetate for 30 seconds, and spin-dried. This provided a resist pattern of a 1:1 line-and-space pattern having a line width of 50 nm.

The LWR performance and the post-development residue were evaluated by the same methods as in the above-described performance evaluation of the pattern forming method (1).

The results will be described in Table 4.

**Table 4**

| | Resist composition | LWR (nm) | Post-development residue | Remarks |
|---|---|---|---|---|
| AN-1 | A-1 | 3.7 | A | Example |
| AN-2 | A-2 | 3.8 | A | Example |
| AN-3 | A-3 | 3.8 | A | Example |
| AN-4 | A-4 | 3.7 | A | Example |
| AN-5 | A-7 | 3.8 | A | Example |
| AN-6 | A-8 | 3.9 | A | Example |
| AN-7 | A-11 | 3.8 | A | Example |
| AN-8 | A-13 | 4.1 | A | Example |
| AN-9 | A-15 | 4.1 | A | Example |
| AN-10 | A-17 | 4.2 | A | Example |
| AN-11 | A-22 | 3.8 | A | Example |
| AN-12 | A-23 | 3.8 | A | Example |
| AN-13 | A-29 | 3.9 | A | Example |
| AN-14 | A-30 | 3.8 | A | Example |
| AN-15 | A-31 | 3.8 | A | Example |
| AN-16 | A-35 | 3.8 | A | Example |
| AN-17 | A-37 | 3.9 | A | Example |
| AN-18 | A-38 | 3.7 | A | Example |
| AN-19 | A-39 | 3.7 | A | Example |
| AN-20 | A-40 | 3.8 | A | Example |
| AN-21 | A-41 | 3.8 | A | Example |
| AN-22 | A-43 | 3.9 | A | Example |
| AN-23 | A-45 | 4.2 | A | Example |
| AN-24 | A-46 | 3.8 | A | Example |
| AN-26 | A-48 | 3.8 | A | Example |
| AN-25 | A-49 | 3.8 | A | Example |
| AN-27 | A-50 | 4.0 | A | Example |
| AN-28 | A-51 | 4.8 | B | Example |
| AN-29 | A-52 | 4.8 | B | Example |
| AN-30 | A-53 | 4.9 | B | Example |
| AN-31 | A-55 | 3.8 | A | Example |
| AN-32 | A-56 | 3.8 | A | Example |
| AN-33 | A-57 | 3.7 | A | Example |
| AN-34 | A-58 | 4.4 | A | Example |
| RAN-1 | RA-1 | 5.4 | C | Comparative Example |
| RAN-2 | RA-2 | 5.3 | C | Comparative Example |

### EUV exposure

### Preparation of resist compositions

The components described in Table 1 and Table 2 were mixed so as to provide a solid-content concentration of 2.0 mass%. Subsequently, such an obtained mixed solution was filtered first through a polyethylene filter having a pore size of 50 nm, then through a nylon filter having a pore size of 10 nm, and finally through a polyethylene filter having a pore size of 5 nm, in this order; in this way, resist compositions were prepared.

Note that the solid content means all the components other than the solvent. The obtained resist compositions were used in Examples and Comparative Examples.

In Table 1 and Table 2, the columns "mass%" indicate the content (mass%) of each component relative to the total solid content in the resist composition. Table 1 and Table 2 also describe the amounts of solvents used (mass ratios).

### Pattern forming method (3): EUV-exposure, alkali development (positive)

An underlayer film-forming composition AL412 (manufactured by Brewer Science, Inc.) was applied onto a silicon wafer, and baked at 205°C for 60 seconds to form an underlayer film having a film thickness of 20 nm. Onto the underlayer film, a resist composition described in Table 5 was applied and baked at 100°C for 60 seconds to form a resist film having a film thickness of 30 nm.

An EUV exposure apparatus (manufactured by Exitech Ltd., Micro Exposure Tool, NA: 0.3, Quadrupole, outer sigma: 0.68, inner sigma: 0.36) was used to subject the obtained silicon wafer having the resist film to pattern irradiation. Note that the reticle employed was a mask having a line size of 25 nm and line:space =1:1.

The exposed resist film was baked at 90°C for 60 seconds, subsequently developed with an aqueous tetramethylammonium hydroxide solution (2.38 mass%) for 30 seconds, and subsequently rinsed with pure water for 30 seconds. Subsequently, this was spin-dried to obtain a positive pattern.

### Performance evaluation

### LWR performance

When a 25 nm (1:1) line-and-space pattern resolved at the optimal exposure dose for resolving a line pattern having a line width of 25 nm on average was observed from above the pattern using a critical dimension scanning electron microscope (SEM (Hitachi, Ltd., S-9380II)), the line widths were observed at 50 points, and the standard deviation (σ) thereof was determined. The measurement variation of the line widths was evaluated on the basis of 3σ, and the value of 3σ was defined as LWR (nm). The smaller the value of LWR, the better the LWR performance. The LWR is preferably 4.9 nm or less, more preferably 4.2 nm or less, and particularly preferably 3.7 nm or less.

### Post-development residue

A section of the 1:1 line-and-space pattern having a line width of 25 nm was observed using a scanning electron microscope (SEM, manufactured by Hitachi, Ltd., S-9380II); cases where residue was not visually observed at all were evaluated as A, cases where a small but acceptable amount of residue was observed were evaluated as B, and cases where a large amount of residue was observed were evaluated as C.

The results will be described in Table 5.

**Table 5**

| | Resist composition | LWR (nm) | Post-development residue | Remarks |
|---|---|---|---|---|
| EP-1 | A-1 | 3.3 | A | Example |
| EP-2 | A-2 | 3.4 | A | Example |
| EP-3 | A-5 | 3.5 | A | Example |
| EP-4 | A-7 | 3.4 | A | Example |
| EP-5 | A-8 | 3.5 | A | Example |
| EP-6 | A-10 | 3.5 | A | Example |
| EP-7 | A-12 | 3.5 | A | Example |
| EP-8 | A-13 | 3.7 | A | Example |
| EP-9 | A-17 | 3.8 | A | Example |
| EP-10 | A-18 | 3.4 | A | Example |
| EP-11 | A-22 | 3.4 | A | Example |
| EP-12 | A-24 | 3.5 | A | Example |
| EP-13 | A-27 | 3.4 | A | Example |
| EP-14 | A-29 | 3.5 | A | Example |
| EP-15 | A-31 | 3.5 | A | Example |
| EP-16 | A-33 | 3.4 | A | Example |
| EP-17 | A-36 | 3.4 | A | Example |
| EP-18 | A-37 | 3.5 | A | Example |
| EP-19 | A-38 | 3.3 | A | Example |
| EP-20 | A-40 | 3.4 | A | Example |
| EP-21 | A-41 | 3.4 | A | Example |
| EP-22 | A-43 | 3.5 | A | Example |
| EP-23 | A-45 | 3.8 | A | Example |
| EP-24 | A-47 | 4.0 | A | Example |
| EP-25 | A-48 | 3.4 | A | Example |
| EP-26 | A-49 | 3.4 | A | Example |
| EP-27 | A-50 | 3.6 | A | Example |
| EP-28 | A-51 | 4.4 | B | Example |
| EP-29 | A-52 | 4.4 | B | Example |
| EP-30 | A-53 | 4.5 | B | Example |
| EP-31 | A-54 | 3.4 | A | Example |
| EP-32 | A-55 | 3.5 | A | Example |
| EP-33 | A-57 | 3.3 | A | Example |
| EP-34 | A-58 | 4.0 | A | Example |
| REP-1 | RA-1 | 5.0 | C | Comparative Example |
| REP-2 | RA-2 | 5.0 | C | Comparative Example |

### Pattern forming method (4): EUV exposure, organic-solvent development (negative)

An underlayer film-forming composition AL412 (manufactured by Brewer Science, Inc.) was applied onto a silicon wafer, and baked at 205°C for 60 seconds to form an underlayer film having a film thickness of 20 nm. Onto the underlayer film, a resist composition described in Table 6 was applied and baked at 100°C for 60 seconds to form a resist film having a film thickness of 30 nm.

An EUV exposure apparatus (manufactured by Exitech Ltd., Micro Exposure Tool, NA: 0.3, Quadrupole, outer sigma: 0.68, inner sigma: 0.36) was used to subject the obtained silicon wafer having the resist film to pattern irradiation. Note that the reticle employed was a mask having a line size of 25 nm and line:space = 1:1.

The exposed resist film was baked at 90°C for 60 seconds, and subsequently developed with n-butyl acetate for 30 seconds; and this was spin-dried to obtain a negative pattern.

The LWR performance and the post-development residue were evaluated by the same methods as in the above-described performance evaluation of the pattern forming method (3).

The results will be described in Table 6.

**Table 6**

| | Resist composition | LWR (nm) | Post-development residue | Remarks |
|---|---|---|---|---|
| EN-1 | A-1 | 3.5 | A | Example |
| EN-2 | A-2 | 3.6 | A | Example |
| EN-3 | A-3 | 3.6 | A | Example |
| EN-4 | A-6 | 3.6 | A | Example |
| EN-5 | A-7 | 3.6 | A | Example |
| EN-6 | A-8 | 3.7 | A | Example |
| EN-7 | A-10 | 3.7 | A | Example |
| EN-8 | A-12 | 3.7 | A | Example |
| EN-9 | A-14 | 3.9 | A | Example |
| EN-10 | A-16 | 3.8 | A | Example |
| EN-11 | A-18 | 3.6 | A | Example |
| EN-12 | A-20 | 3.6 | A | Example |
| EN-13 | A-24 | 3.7 | A | Example |
| EN-14 | A-26 | 3.6 | A | Example |
| EN-15 | A-27 | 3.6 | A | Example |
| EN-16 | A-31 | 3.6 | A | Example |
| EN-17 | A-34 | 3.6 | A | Example |
| EN-18 | A-36 | 3.6 | A | Example |
| EN-19 | A-37 | 3.7 | A | Example |
| EN-20 | A-38 | 3.5 | A | Example |
| EN-21 | A-39 | 3.5 | A | Example |
| EN-22 | A-42 | 3.6 | A | Example |
| EN-23 | A-43 | 3.7 | A | Example |
| EN-24 | A-47 | 4.2 | A | Example |
| EN-25 | A-48 | 3.6 | A | Example |
| EN-26 | A-49 | 3.6 | A | Example |
| EN-27 | A-50 | 3.8 | A | Example |
| EN-28 | A-51 | 4.6 | B | Example |
| EN-29 | A-52 | 4.6 | B | Example |
| EN-30 | A-53 | 4.7 | B | Example |
| EN-31 | A-54 | 3.6 | A | Example |
| EN-32 | A-56 | 3.6 | A | Example |
| EN-33 | A-57 | 3.5 | A | Example |
| EN-34 | A-58 | 4.2 | A | Example |
| REN-1 | RA-1 | 5.2 | C | Comparative Example |
| REN-2 | RA-2 | 5.2 | C | Comparative Example |

The results in Tables 3 to 6 have demonstrated that the resist compositions used in Examples have high LWR performance and can suppress post-development residue.

The present invention can provide an actinic ray-sensitive or radiation-sensitive resin composition that has high LWR performance and can achieve reduction in the amount of post-development residue.

The present invention can also provide a resist film using the actinic ray-sensitive or radiation-sensitive resin composition, a pattern forming method, a method for producing an electronic device, and onium salts that can be used in the actinic ray-sensitive or radiation-sensitive resin composition.

The present invention has been described in detail and with reference to specific embodiments thereof; however, it would be apparent to those skilled in the art that various changes and modifications can be made therein without departing from the spirit and scope of the present invention.

This application is based on a Japanese patent application filed on December 27, 2022 (JP2022-210176), and the contents of which are incorporated herein by reference.

## Claims

1. An actinic ray-sensitive or radiation-sensitive resin composition comprising a resin that is subjected to an action of an acid to undergo an increase in polarity, and an onium salt represented by a formula (N1) below: 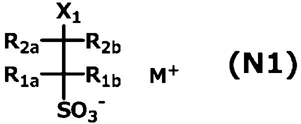
wherein, in the formula (N1), R₁ₐ represents a cyano group, a nitro group, or a substituent represented by a formula (1A) below;
R_{1b} and R_{2b} each independently represent a substituent having no fluorine atoms or a hydrogen atom;
R₂ₐ represents a cyano group, a nitro group, a substituent represented by the formula (1A) below, or a substituent represented by a formula (1B) below;
X₁ represents a hydrogen atom or an organic group;
M⁺ represents an organic cation; and
at least two of R₁ₐ, R_{1b}, R₂ₐ, R_{2b}, and X₁ may be bonded together to form a ring, 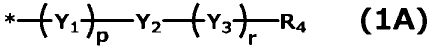
in the formula (1A), Y₁ and Y₃ each independently represent -O- or -NR₃-;
R₃ represents a hydrogen atom or an alkyl group;
Y₂ represents -C(=O)- or -SO₂-;
R₄ represents an alkyl group, a cycloalkyl group, or an aryl group;
p and r each independently represent 0 or 1, and
* represents a bonding site,
***-CH₂-R_{Y}** **(1B)**
in the formula (1B), R_{Y} represents a cyano group, a nitro group, or a substituent represented by the formula (1A); and
* represents a bonding site.

2. The actinic ray-sensitive or radiation-sensitive resin composition according to claim 1, wherein, when R₁ₐ, R_{1b}, R₂ₐ, and R_{2b} in the formula (N1) correspond to (i) below, R_{2b} represents a nitro group, a substituent represented by the formula (1A), or a substituent represented by the formula (1B),
when R₁ₐ, R_{1b}, R₂ₐ, and R_{2b} in the formula (N1) correspond to (ii) below, X₁ represents an organic group, or
when R₁ₐ, R_{1b}, and R₂ₐ in the formula (N1) correspond to (iii) below, X₁ represents an organic group,
(i) a case where both of R₁ₐ and R_{1b} are not substituents represented by the formula (1A) and only one of R₁ₐ and R_{1b} is a cyano group, R₂ₐ is a cyano group, and R_{2b} is not a cyano group,
(ii) a case where only one of R₁ₐ and R_{1b} is a cyano group, and both of R₂ₐ and R_{2b} are cyano groups, or
(iii) a case where R₁ₐ is a substituent represented by the formula (1A), and Y₂ in the formula (1A) is -C(=O)-, R_{1b} is a hydrogen atom, and R₂ₐ is a substituent represented by the formula (1A).

3. The actinic ray-sensitive or radiation-sensitive resin composition according to claim 1, wherein, in the formula (N1), R₁ₐ represents a cyano group, a nitro group, -COOR₄, -OCOOR₄, or -SO₂R₄, and R₄ has the same meaning as R₄ in the formula (1A).

4. The actinic ray-sensitive or radiation-sensitive resin composition according to claim 1, wherein, in the formula (N1), R₂ₐ represents a cyano group, a nitro group, -COOR₄, -OCOOR₄, -SO₂R₄, or a substituent represented by a formula (1C) below; and R₄ has the same meaning as R₄ in the formula (1A),
***-CH₂-R_{Y2}** **(1C)**
in the formula (1C), R_{Y2} represents a cyano group, a nitro group, -COOR₄, -OCOOR₄, or -SO₂R₄;
R₄ has the same meaning as R₄ in the formula (1A); and
* represents a bonding site.

5. The actinic ray-sensitive or radiation-sensitive resin composition according to claim 1, wherein the onium salt represented by the formula (N1) is represented by a formula (N2) below: 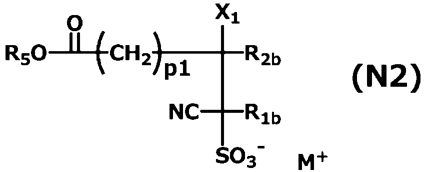
in the formula (N2), R₅ represents an alkyl group, a cycloalkyl group, or an aryl group;
p1 represents 0 or 1; and
R_{1b}, R_{2b}, X₁, and M⁺ respectively have the same meanings as R_{1b}, R_{2b}, X₁, and M⁺ in the formula (N1).

6. The actinic ray-sensitive or radiation-sensitive resin composition according to claim 1, wherein the onium salt represented by the formula (N1) is represented by a formula (N3) below: 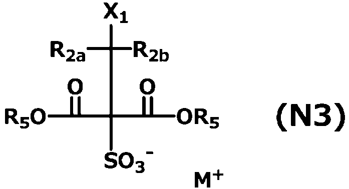
in the formula (N3), R₅ represent an alkyl group, a cycloalkyl group, or an aryl group, and the plurality of R₅ may be the same or different; and
R₂ₐ, R_{2b}, X₁, and M⁺ respectively have the same meanings as R₂ₐ, R_{2b}, X₁, and M⁺ in the formula (N1).

7. The actinic ray-sensitive or radiation-sensitive resin composition according to claim 1, wherein the onium salt represented by the formula (N1) is represented by a formula (N4) below: 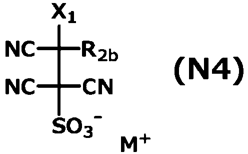 in the formula (N4), R_{2b}, X₁, and M⁺ respectively have the same meanings as R_{2b}, X₁, and M⁺ in the formula (N1).

8. The actinic ray-sensitive or radiation-sensitive resin composition according to claim 1, wherein the onium salt represented by the formula (N1) is represented by a formula (N5) below: 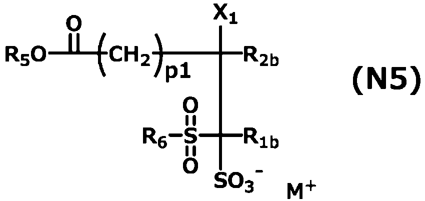
in the formula (N5), R₅ and R₆ each independently represent an alkyl group, a cycloalkyl group, or an aryl group;
p1 represents 0 or 1; and
R_{1b}, R_{2b}, X₁, and M⁺ respectively have the same meanings as R_{1b}, R_{2b}, X₁, and M⁺ in the formula (N1), and at least two of R_{1b}, R_{2b}, R₆, and X₁ may be bonded together to form a ring.

9. The actinic ray-sensitive or radiation-sensitive resin composition according to claim 1, wherein M⁺ above is a sulfonium cation or an iodonium cation.

10. A resist film formed using the actinic ray-sensitive or radiation-sensitive resin composition according to any one of claims 1 to 9.

11. A pattern forming method comprising:
a step of using the actinic ray-sensitive or radiation-sensitive resin composition according to any one of claims 1 to 9 to form a resist film on a substrate;
a step of exposing the resist film; and
a step of developing the exposed resist film using a developer.

12. A method for producing an electronic device, the method comprising the pattern forming method according to claim 11.

13. An onium salt represented by a formula (N2-1) below: 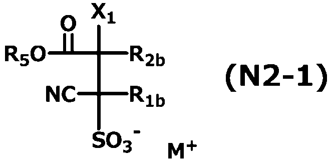
wherein, in the formula (N2-1), R₅ represents an alkyl group, a cycloalkyl group, or an aryl group;
R_{1b} and R_{2b} each independently represent a substituent having no fluorine atoms or a hydrogen atom;
X₁ represents a hydrogen atom or an organic group;
M⁺ represents an organic cation; and
at least two of R_{1b}, R_{2b}, and X₁ may be bonded together to form a ring.

14. An onium salt represented by a formula (N5-1) below: 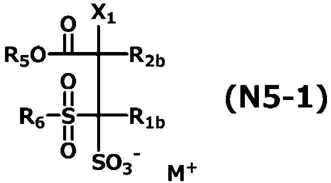
wherein, in the formula (N5-1), R₅ and R₆ each independently represent an alkyl group, a cycloalkyl group, or an aryl group;
R_{1b} and R_{2b} each independently represent a substituent having no fluorine atoms or a hydrogen atom;
X₁ represents a hydrogen atom or an organic group;
M⁺ represents an organic cation; and
at least two of R_{1b}, R_{2b}, R₆, and X₁ may be bonded together to form a ring.
